# EUROPEAN PATENT APPLICATION

(11) **EP 4 162 954 A1**
(43) Date of publication of application: **12.04.2023**
(21) Application number: 21821872.5
(22) Date of filing: 31.05.2021
(51) Int. Cl.: A61K 47/50, C07K 16/30, C07K 16/32, C07K 7/06, A61K 31/4745, A61P 35/00

(54) **CAMPTOTHECIN DRUG HAVING HIGH-STABILITY HYDROPHILIC CONNECTING UNIT AND CONJUGATE THEREOF**

(30) Priority: 08.06.2020 CN 202010510363
(71) Applicant: Baili-Bio (Chengdu) Pharmaceutical Co., Ltd., Chengdu, Sichuan 611130 (CN)
(72) Inventor: ZHU, Yi, Chengdu, Sichuan 611130 (CN); WAN, Weili, Chengdu, Sichuan 611130 (CN); ZHUO, Shi, Chengdu, Sichuan 611130 (CN); ZHANG, Yong, Chengdu, Sichuan 611130 (CN); ZHANG, Yiying, Chengdu, Sichuan 611130 (CN); YU, Tianzi, Chengdu, Sichuan 611130 (CN); LI, Gangrui, Chengdu, Sichuan 611130 (CN); YANG, Xiujuan, Chengdu, Sichuan 611130 (CN)
(74) Representative: Hobson, David James
(86) International application number: PCT/CN2021/097302
(87) International publication number: WO 2021/249228

(57) **Abstract**

A camptothecin drug having a highly stable hydrophilic connecting unit and its conjugate, or its pharmaceutically acceptable salt thereof, including methods for preparation thereof, and its applications in preventing and/or treating cancer. The conjugate can specifically bind to receptors highly expressed in tumor cells. The conjugates have excellent water solubility, stability, and homogeneity, and can be used for preventing and/or treating tumors and/or other diseases.

## Description

### TECHNICAL FIELD

The disclosure relates to a camptothecin antibody-drug conjugate having a high-stability hydrophilic connecting unit.

### BACKGROUND

Antibody-drug conjugates (ADC), as a new type of targeted drugs, generally consist of three parts: antibodies or antibody-like ligands, small-molecule drugs, and linkers that couple the ligands and drugs. Antibody-drug conjugates use the specific recognition of antibodies to antigens, transport drug molecules to the vicinity of target cells, and effectively release drug molecules to achieve the purpose of treatment. In August 2011, the U.S. Food and Drug Administration (FDA) approved the listing of Adcetris^{™}, a new ADC drug developed by Seattle Genetics for the treatment of Hodgkin's lymphoma and recurrent degenerative large cell lymphoma (ALCL), and its clinical application has been proven The safety and effectiveness of this type of drug are discussed.

Camptothecins, as small molecule compounds with anti-tumor properties, exhibit anti-tumor effects by inhibiting DNA topoisomerase I, including irinotecan, exatecan, SN38 and so on. Many camptothecin drugs have been widely used in clinical practice, and the main indications are bone cancer, prostate cancer, breast cancer, pancreatic cancer, etc. Unlike the current clinical use of irinotecan, exatecan does not need to be activated through the use of enzymes. In addition, compared with SN-38, which is the pharmacodynamic body of irinotecan, and topotecan, which is also used in clinical practice, topoisomerase I has a stronger inhibitory activity and has stronger damage against a variety of cancer cells in vitro. In particular, the expression of P-glycoprotein also shows an effect on cancer cells that are resistant to SN-38 and the like. Exatecan has not been successfully marketed as a single chemotherapeutic drug, which is speculated to be related to its higher cell activity, resulting in a narrow therapeutic window.

Antibody-drug conjugate (ADC) drugs have the advantages of increasing water solubility, improving targeting, binding specific antibodies and antigens, carrying drugs around target cells, and effectively killing tumors by releasing drugs near the target cells, reduce toxic side effects. Camptothecin drugs have considerable application prospects in ADC drugs. Currently, the antibody conjugate drug trastuzumab deruxtecan (trade name Enhertu) with Exatecan as the toxin has been approved by the U.S. FDA on December 20, 2019. As the first camptothecin ADC drug to be marketed, it has well proved the drug-making ability and application prospects of this type of drug in the ADC field.

ADC drug structure includes three key parts: antibody, linker, and toxin. Any part of the defects may affect the overall efficacy of ADC. In this field, the structural design defects of Enhertu are obvious: Camptothecin is a class of highly fat-soluble and poorly soluble drugs. The linker-toxin used by Enhertu is designed to be connected to the antibody through a Mc linker, and is connected to a tetrapeptide that can be cleaved. Fragments, matched with aminomethoxy self-eliminating spacer units, use interchain cysteine residues to achieve a drug-antibody ratio (DAR) of 8 (refer to patent CN104755494) by non-site-directed coupling technology. The design of the linker, at a high DAR value, will cause the stability of camptothecin ADC drugs to decrease, and the monomer rate will decrease, which will further reduce the efficacy and safety of ADC in vivo.

The technical problem that this application solves is to provide better anti-tumor camptothecin ADC drugs having higher safety and effectiveness and better meet clinical needs.

### SUMMARY

Based on a comprehensive understanding of ADC drugs, the inventors unexpectedly discovered a series of antibody-drug conjugates of camptothecin derivatives with highly stable hydrophilic polypeptide linking structural units. Through experiments, inventors found that, the ADC molecules of various derivatives of camptothecin carrying the peptide linker show high stability in vivo and in vitro, with a high monomer rate, and have significantly higher pharmacodynamic activity compared to the control ADCs. At the same time, the inventors created a new deprotection reagent and solvent strategy through the design and innovation of the synthetic route, which can efficiently produce the complex linker-toxin molecule.

In one aspect, the disclosure provides a ligand-drug conjugate shown in formula I or a pharmaceutically acceptable salt thereof, 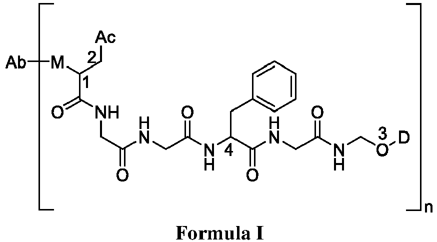 wherein:
Ab is a ligand unit, selected from an antibody, antibody fragment, and protein;
M is a connecting unit connected with Ab;
Ac is a hydrophilic structural unit;
D is a camptothecin drug;
The position-1 and position-4 chiral carbon atoms each independently has the chirality of R or S configuration;
n is selected from an integer of 1-20.

In one embodiment, the connecting unit M has a succinimide structure represented by the following formula a, or an open-ringed succinimide structure as represented by formula b1 or b2,

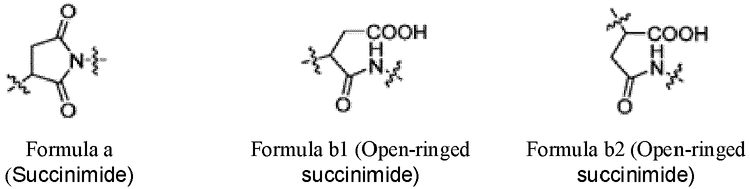

In a wherein in formula a, formula b1 and formula b2, the wavy line on the left indicates the connection to a connection site of the Ab, and the wavy line on the right indicates the connection to the position-1 tertiary carbon atom in formula I.

In one embodiment, the Ac has the structure shown in the following formula c, 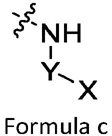
wherein X is one or more group independently selected from the group consisting of hydrophilic carboxyl group, phosphoric acid, polyphosphoric acid, phosphorous acid, sulfonic acid, sulfinic acid and polyethylene glycol (PEG);
Y is a scaffold connecting the amino group (NH) and X;
Ac is connected to the position-2 methylene carbon in the formula I through an amino functional group.

In one embodiment, Ac is non-limitingly selected from Glycine, (D/L)-Alanine, (D/L)-Leucine, (D/L)-Isoleucine, (D/L)-Valine, (D/L)-Phenylalanine, (D/L)-Proline, (D/L)-Tryptophan, (D/L)-Serine, (D/L)-Tyrosine, (D/L)-Cysteine, (D/L)-Cystine, (D/L)-Arginine, (D/L)-Histidine, (D/L)-Methionine, (D/L)-Asparagine, (D/L)-Glutamine, (D/L)-Threonine, (D/L)-Aspartic acid, (D/L)-Glutamic acid, natural or unnatural amino acid derivatives or the following structures, 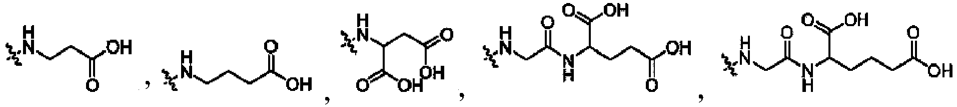 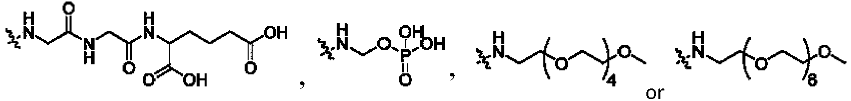 wherein the left wavy line indicating linking to the carbon atom number 2.

In one embodiment, the camptothecin drug has the structure shown in the following formula d; 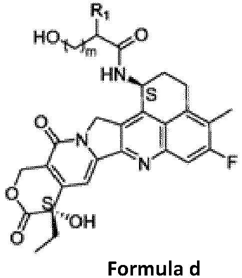
wherein R ₁ is selected from a group consisting of hydrogen atom, deuterium atom, halogen, alkyl, deuterated alkyl, haloalkyl, cycloalkyl, cycloalkylalkyl, alkoxyalkyl, heterocyclyl, aryl, substituted aryl and heteroaryl;
alternatively, R ₁ and the carbon atom to which it is connected form a C₃₋₆ cycloalkyl, cycloalkylalkyl or heterocyclic group;
the chiral carbon atom connected to R₁ has two chirality of R absolute configuration and S absolute configuration;
m is selected from 0 or 1;
the hydroxyl group connected to the carbon atom connected to R₁ is involved in linking the position-3 oxygen atom in formula I.

In one embodiment, the camptothecin drug is selected from the following compounds without limitation.

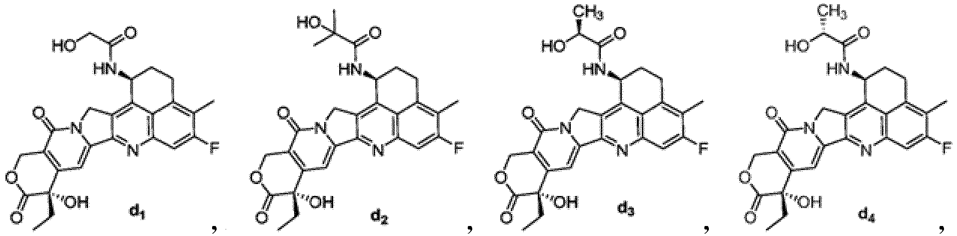

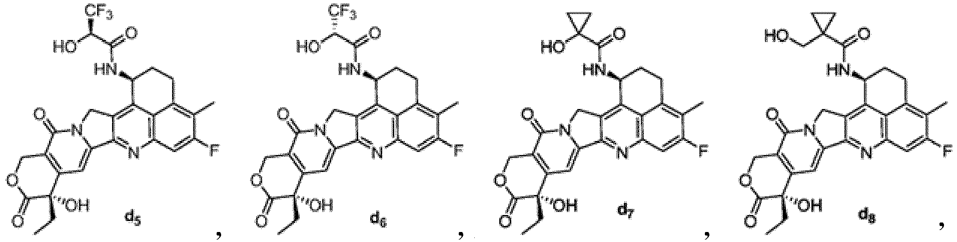

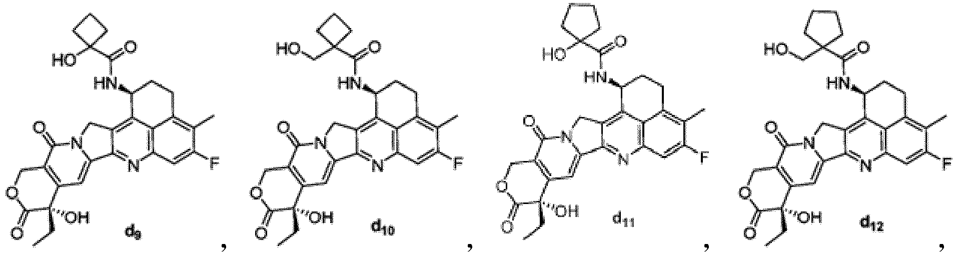

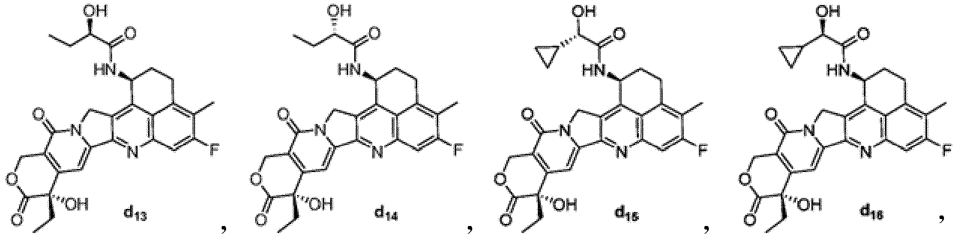

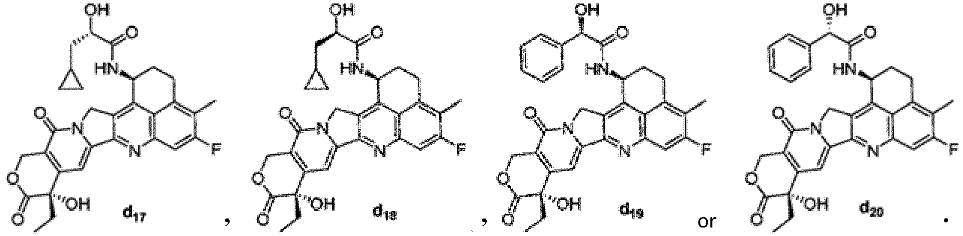

In one embodiment, the application provides a linker-drug compound or a pharmaceutically acceptable salt thereof for coupling with the ligand unit Ab to form the ligand-drug conjugate of formula I described in claim 1, having the following structure shown in formula II, 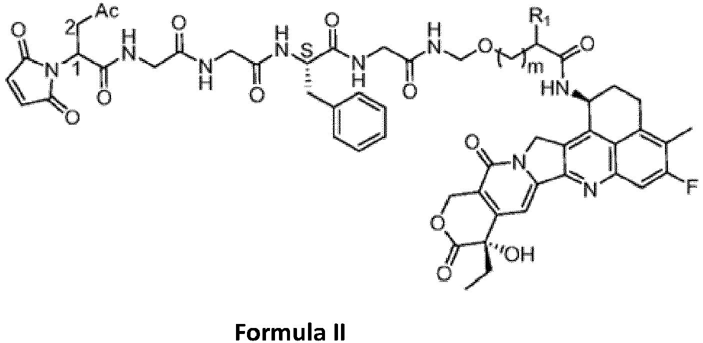
wherein R ₁ is selected from a group consisting of hydrogen atom, deuterium atom, halogen, alkyl, deuterated alkyl, haloalkyl, cycloalkyl, cycloalkylalkyl, alkoxyalkyl, heterocyclyl, aryl, substituted aryl and heteroaryl;
alternatively, R ₁ and the carbon atom to which it is connected form a C₃₋₆ cycloalkyl, cycloalkylalkyl or heterocyclic group
the chiral carbon atom at position-1 has two chirality of R absolute configuration and S absolute configuration;
Ac is a hydrophilic structural unit; and
m is selected from 0 or 1.

In one embodiment, Ac is selected from, without limitation, glycine, phosphoric acid, (D/L)-glutamic acid, or polyethylene glycol (PEG).

In one embodiment, the linker-drug compound or a pharmaceutically acceptable salt thereof is selected from the following structures, including without limitation, 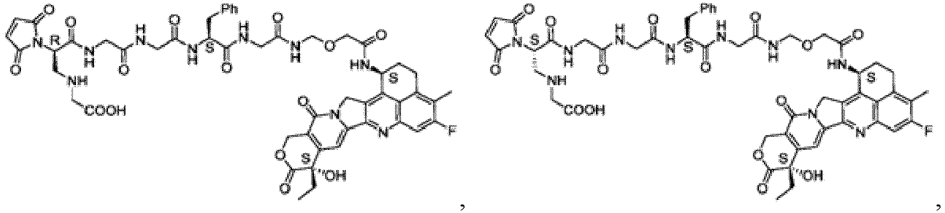 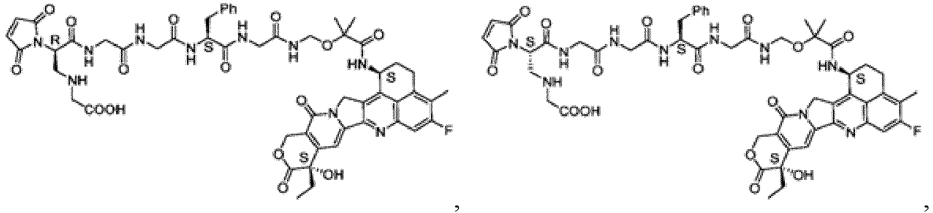 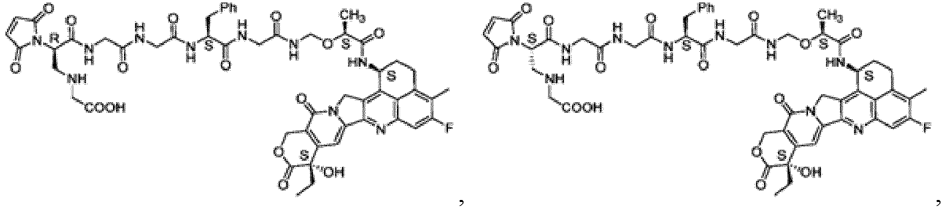 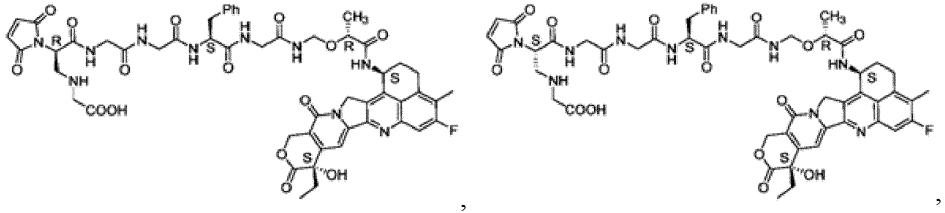 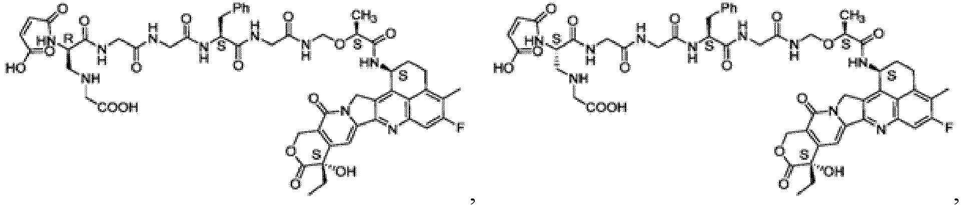 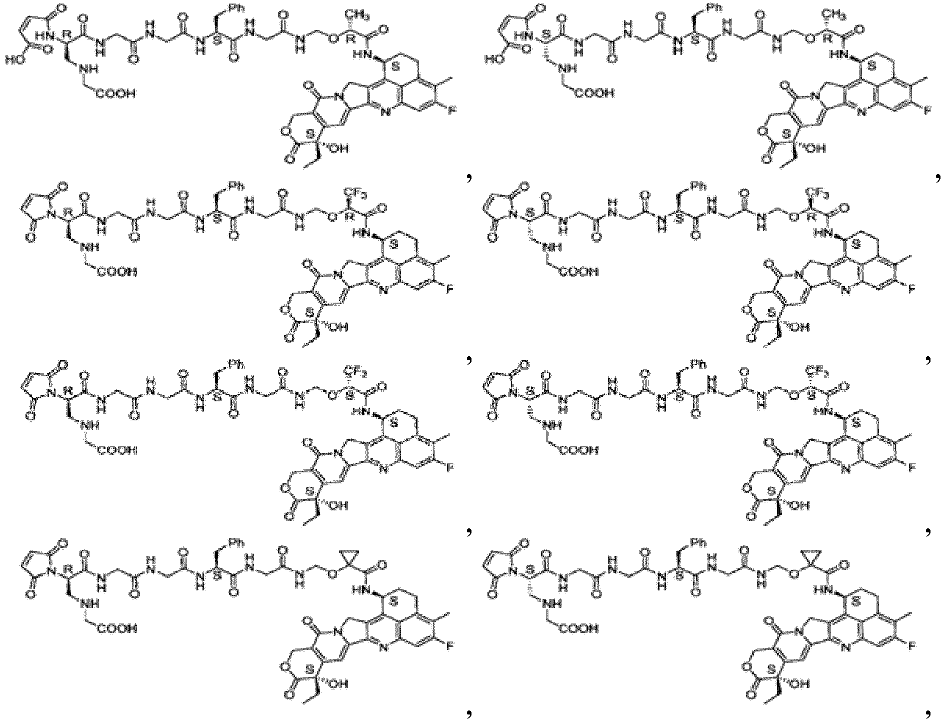 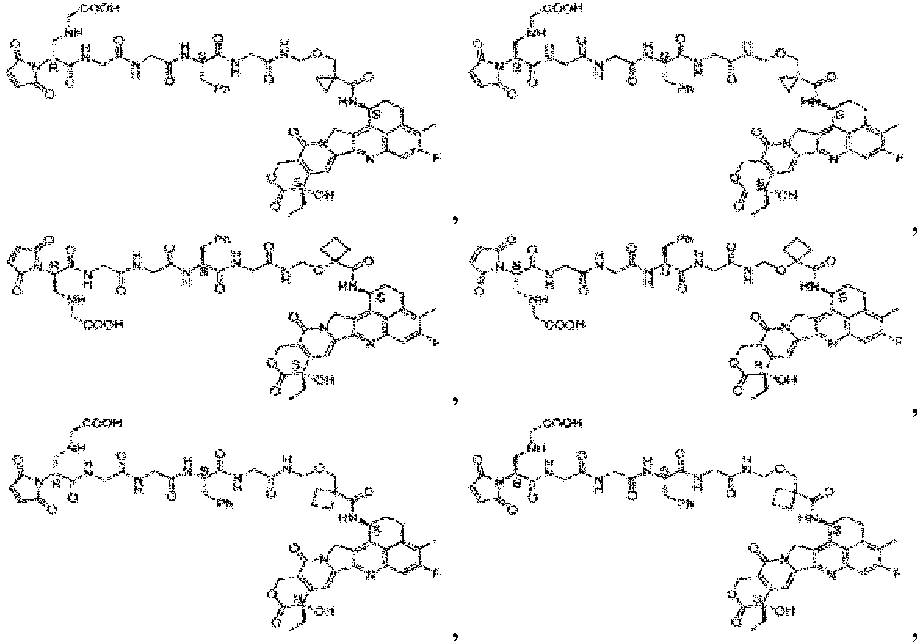 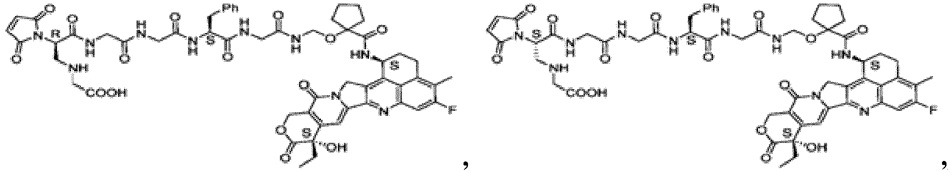 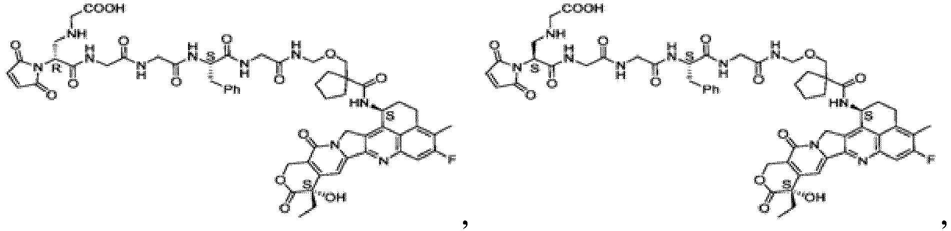 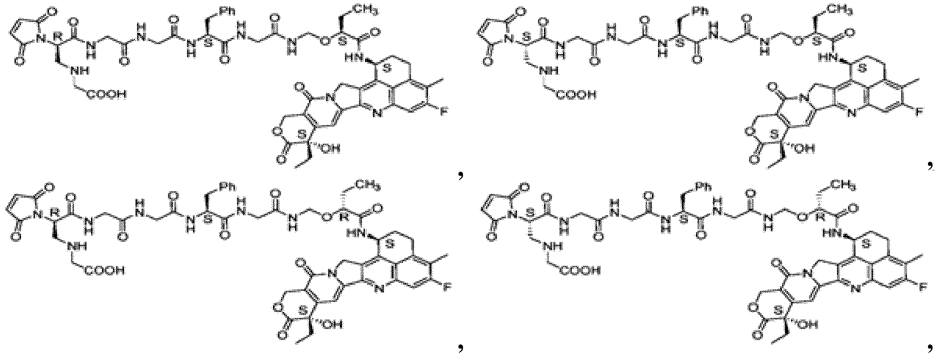 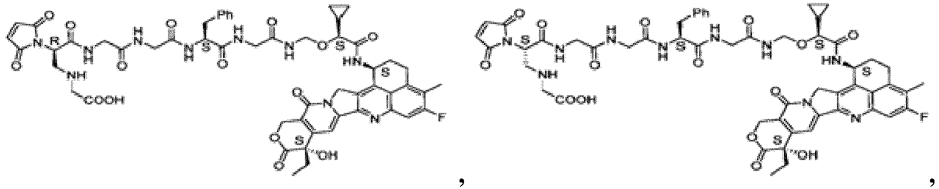 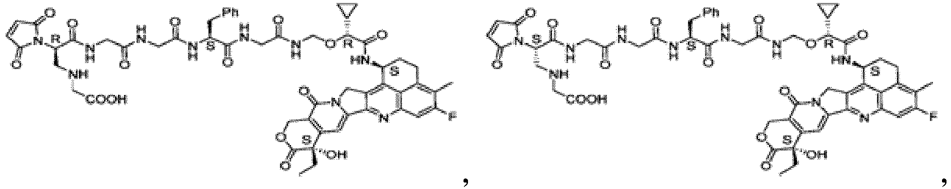 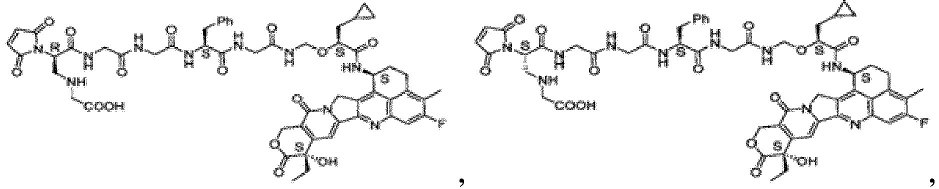 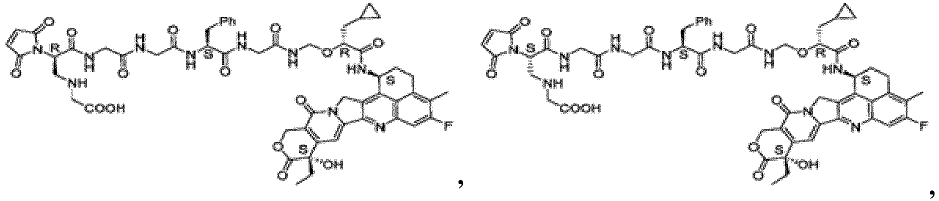 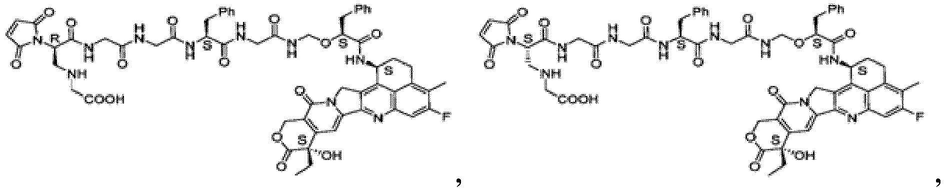 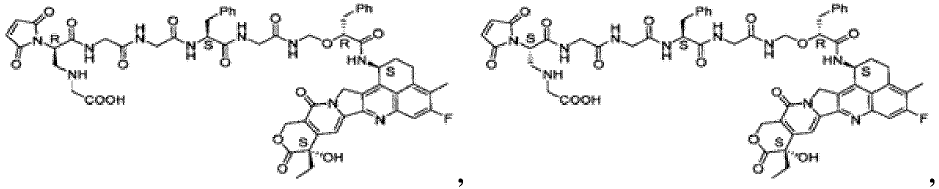 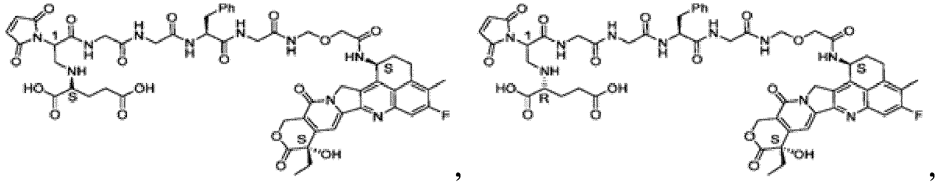 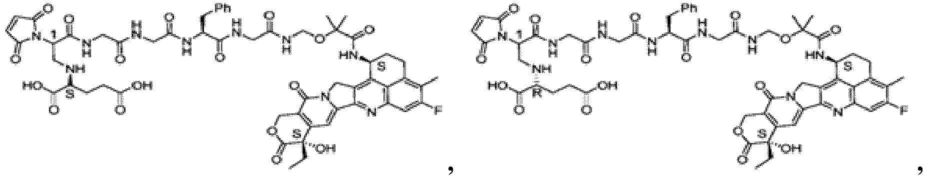 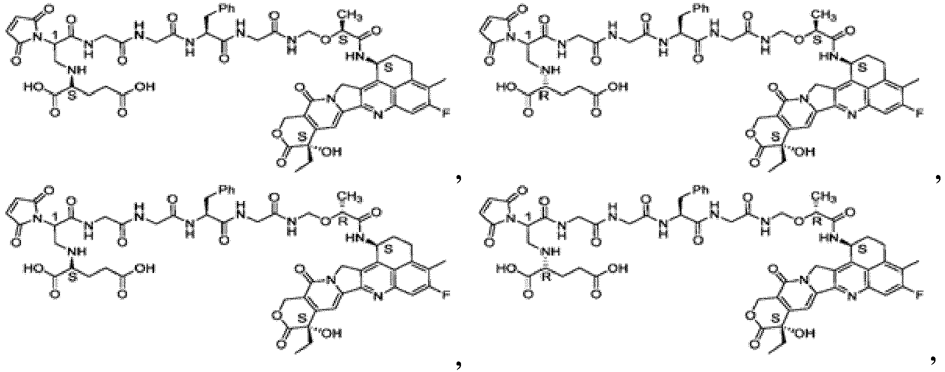 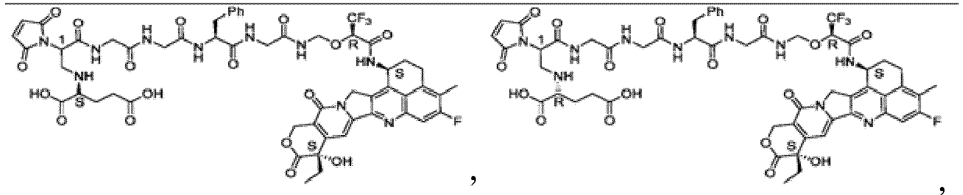 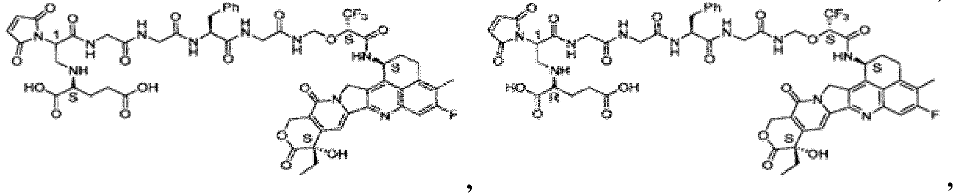 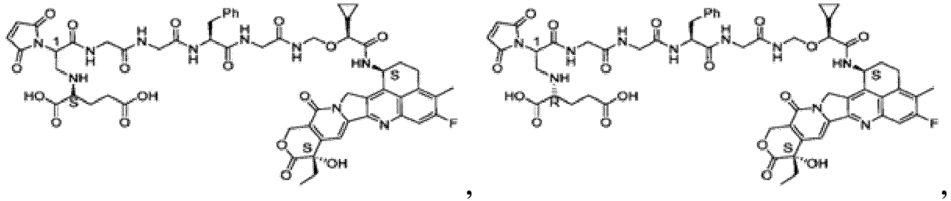 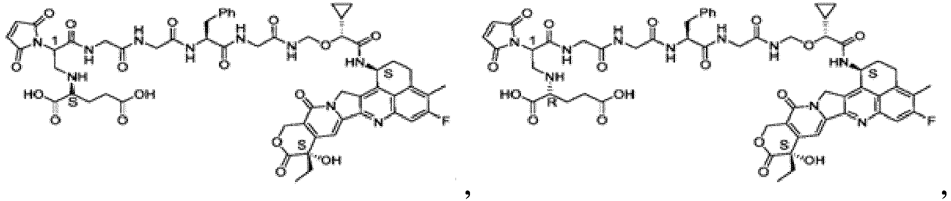 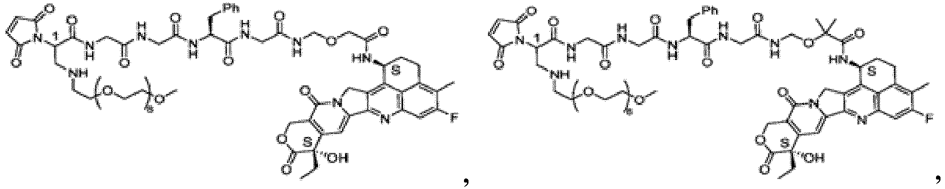 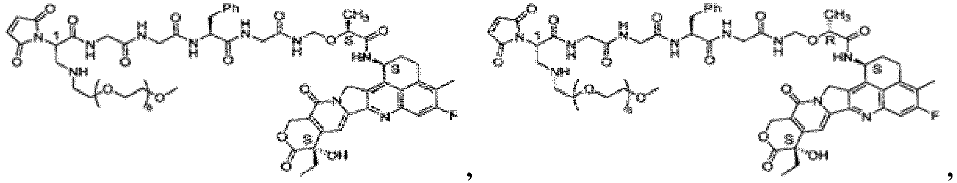 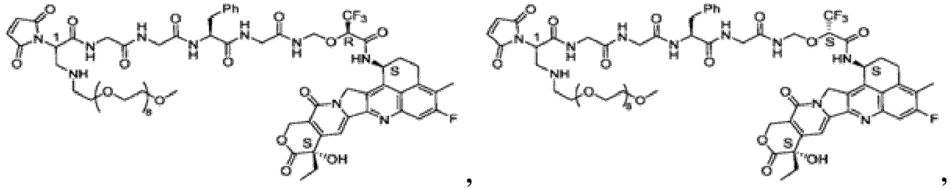 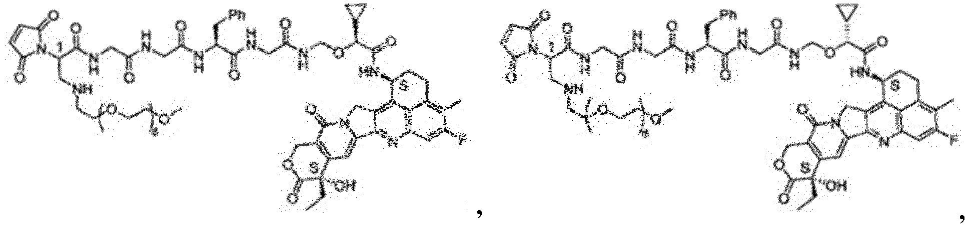 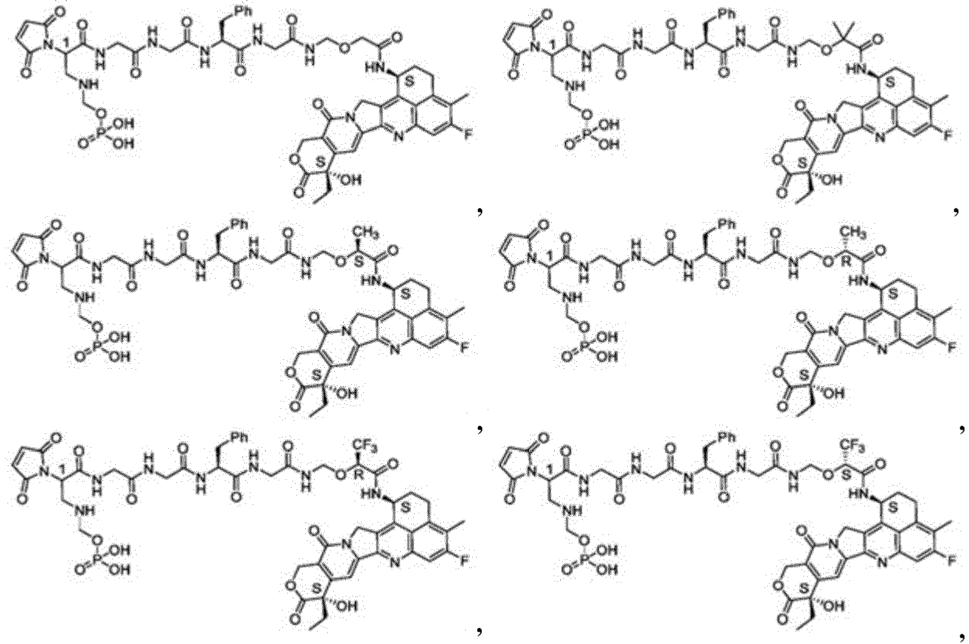 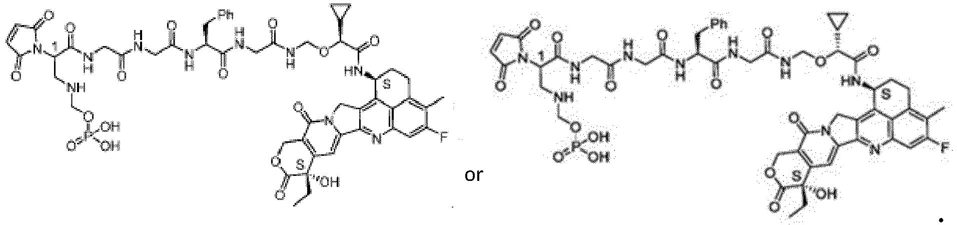 where the position-1 chiral carbon has two configurations of R absolute chirality or S absolute chirality.

In another embodiment, the ligand-drug conjugate or a pharmaceutically acceptable salt thereof having the structure shown in the following formula III, formula IV-1 or formula IV-2 is disclosed. 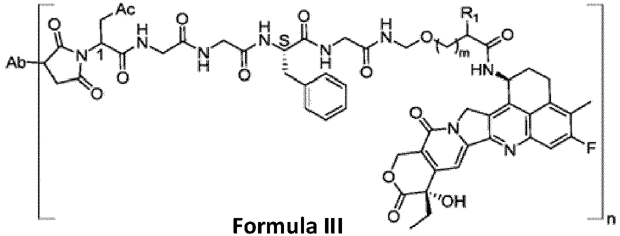 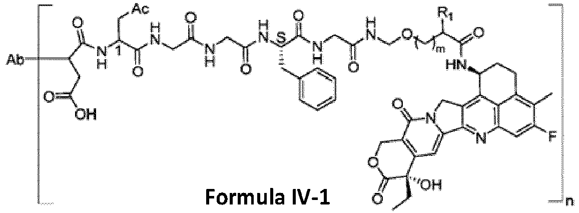 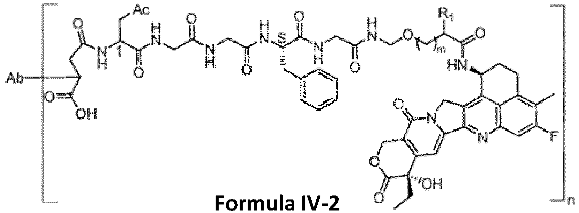
wherein Ab is the ligand unit;
Ac is a hydrophilic structural unit;
the position-1 chiral carbon has two configurations of absolute chirality of R or absolute chirality of S;
R₁, m and n are as described in formula II.

In one embodiment, the inventors disclose a ligand-drug conjugate or a pharmaceutically acceptable salt thereof, wherein the ligand unit Ab is selected from an antibody, an antibody fragment, or a protein, wherein the antibody is selected from a murine antibody, rabbit antibodies, phage display antibodies, yeast display antibodies, chimeric antibodies, humanized antibodies, fully human antibodies, antibody fragments, bispecific antibodies and multi-specific antibodies.

In one embodiment, the antibody is a monoclonal antibody, and is non-limitingly selected from the group consisting of anti-EGFRvlll antibody, anti-PD-1 antibody, anti-PD-L1 antibody, anti-DLL-3 antibody, anti-PSMA antibody, anti-CD70 antibody, anti-MUC16 antibody, anti-ENPP3 antibody, anti-TDGF1 antibody, anti-ETBR antibody, anti-MSLN antibody, anti-TIM-1 antibody, Anti-LRRC15 antibody, anti-LIV-1 antibody, anti-CanAg/AFP antibody, anti-cladin 18.2 antibody, anti-Mesothelin antibody, anti-HER2 (ErbB2) antibody, anti-EGFR antibody, anti-c-MET antibody, anti-SLlTRK6 antibody, anti-KIT/CD117 Antibody, anti-STEAP1 antibody, anti-SLAMF7/CS1 antibody, anti-NaPi2B/SLC34A2 antibody, anti-GPNMB antibody, anti-HER3 (ErbB3) antibody, anti-MUC1/CD227 antibody, anti-AXL antibody, anti-CD166 antibody, anti-B7-H3 (CD276) Antibody, anti-PTK7/CCK4 antibody, anti-PRLR antibody, anti-EFNA4 antibody, anti-5T4 antibody, anti-NOTCH3 antibody, anti-Nectin 4 antibody, anti-TROP-2 antibody, anti-CD142 antibody, anti-CA6 antibody, anti-GPR20 antibody, anti-CD174 antibody , Anti-CD71 antibody, anti-EphA2 antibody, anti-LYPD3 antibody, anti-FGFR2 antibody, anti-FGFR3 antibody, anti-FRα antibody, anti-CEACAMs antibody, anti-GCC antibody, anti-Integrin Av antibody, anti-CAIX antibody, anti-P-cadherin antibody, anti-GD3 Antibody, anti-Cadherin 6 antibody, anti-LAMP1 antibody, anti-FLT3 antibody, anti-BCMA antibody, anti-CD79b antibody, anti-CD19 antibody, anti-CD33 antibody, anti-CD56 antibody, anti-CD74 antibody, anti-CD22 antibody, anti-CD30 antibody, anti-CD37 antibody , Anti-CD47 antibody, anti-CD138 antibody, anti-CD352 antibody, anti-CD25 antibody and anti-CD123 antibody.

In one embodiment, the antibody or antigen-binding fragment comprises Trastuzumab, comprising:
A light chain sequence having an amino acid sequence of: 
MDMRVPAQLLGLLLLWLRGARCDIQMTQSPSSLSASVGDRVTITCRASQDVNTAVAWYQQKPGKAPKLLIYS
ASFLYSGVPSRFSGSRSGTDFTLTISSLQPEDFATYYCQQHYTTPPTFGQGTKVEIKRTVAAPSVFIFPPSDEQLKS
GTASVVCLLNNFYPREAKVQWKVDNALQSGNSQESVTEQDSKDSTYSLSSTLTLSKADYEKHKVYACEVTHQ
GLSSPVTKSFNRGEC* (SEQ ID NO: 1);
 and
A heavy chain sequence having an amino acid sequence of:

MDMRVPAQLLGLLLLWLRGARCEVQLVESGGGLVQPGGSLRLSCAASGFNIKDTYIHWVRQAPGKGLEWVA
RIYPTNGYTRYADSVKGRFTISADTSKNTAYLQMNSLRAEDTAVYYCSRWGGDGFYAMDYWGQGTLVTVSSA
STKGPSVFPLAPSSKSTSGGTAALGCLVKDYFPEPVTVSWNSGALTSGVHTFPAVLQSSGLYSLSSVVTVPSSSL
GTQTYICNVNHKPSNTKVDKKVEPKSCDKTHTCPPCPAPELLGGPSVFLFPPKPKDTLMISRTPEVTCVVVDVS
HEDPEVKFNWYVDGVEVHNAKTKPREEQYNSTYRVVSVLTVLHQDWLNGKEYKCKVSNKALPAPIEKTISKA
KGQPREPQVYTLPPSREEMTKNQVSLTCLVKGFYPSDIAVEWESNGQPENNYKTTPPVLDSDGSFFLYSKLTV
DKSRWQQGNVFSCSVMHEALHNHYTQKSLSLSPG (SEQ ID NO: 2).

In one embodiment, the ligand-drug conjugate or a pharmaceutically acceptable salt thereof is selected from the following succinimide structures or succinimide open-ring structures without limitation. 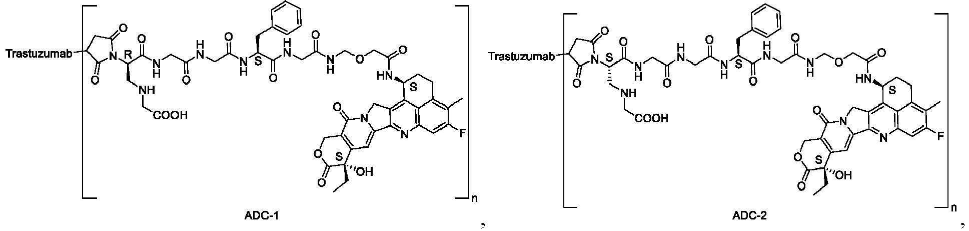 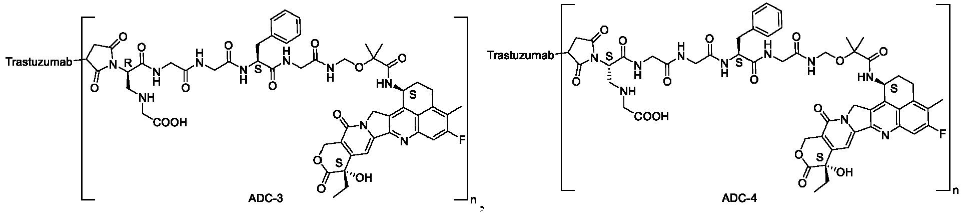 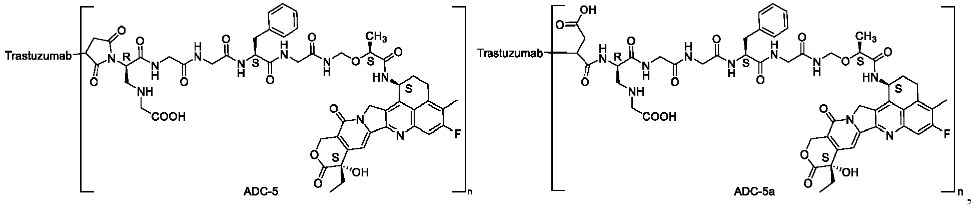 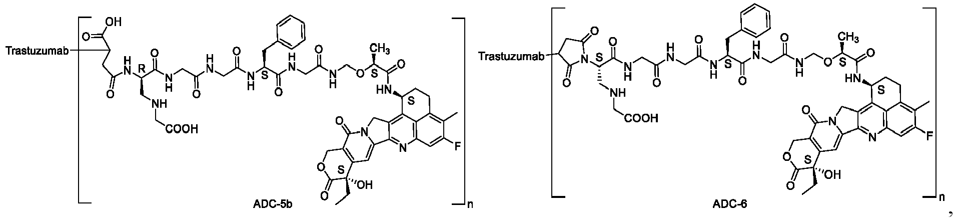 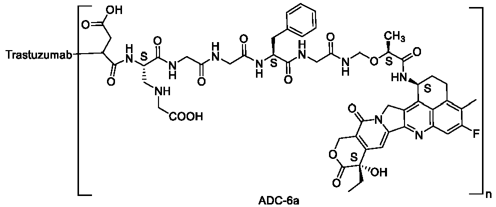 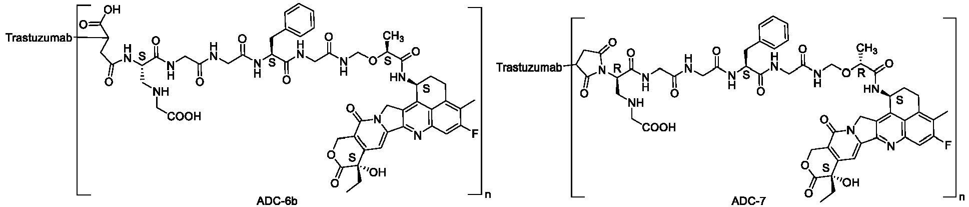 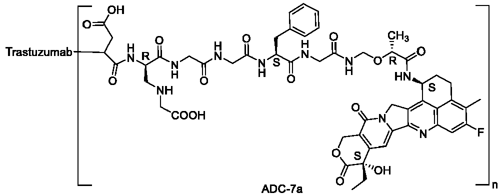 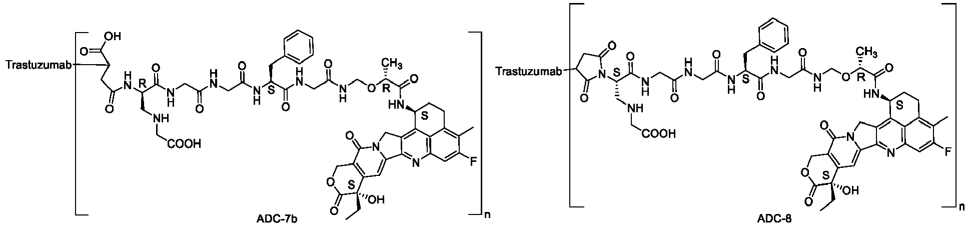 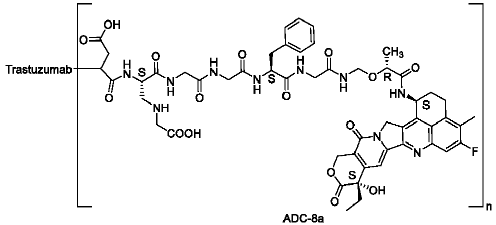 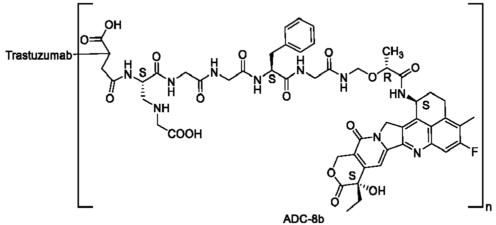 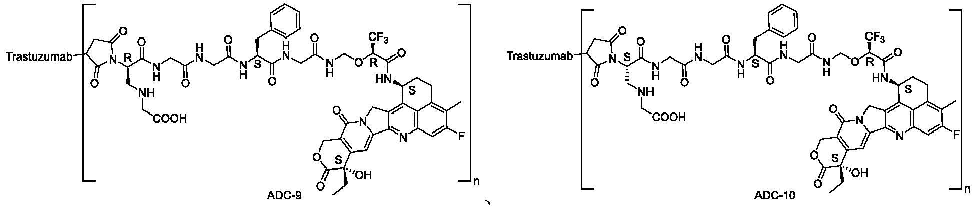 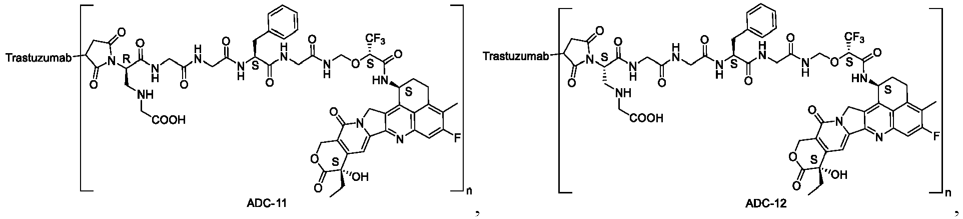 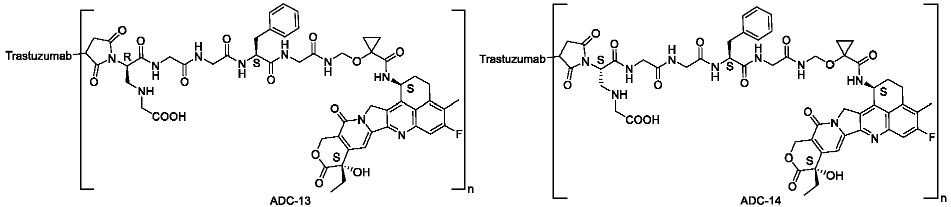 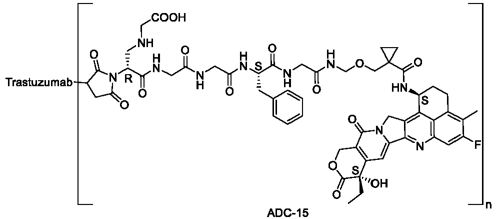 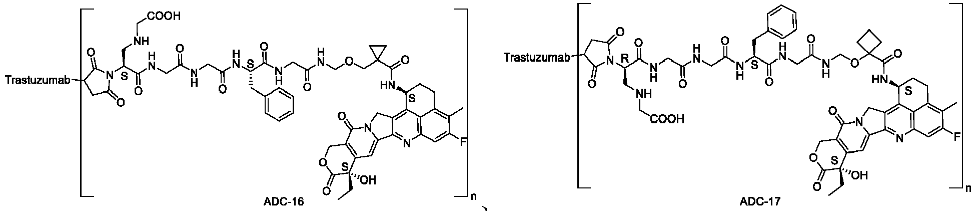 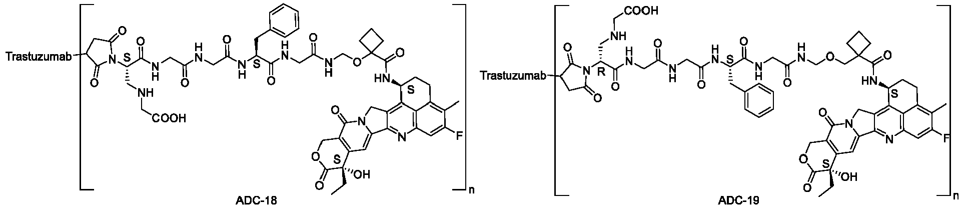 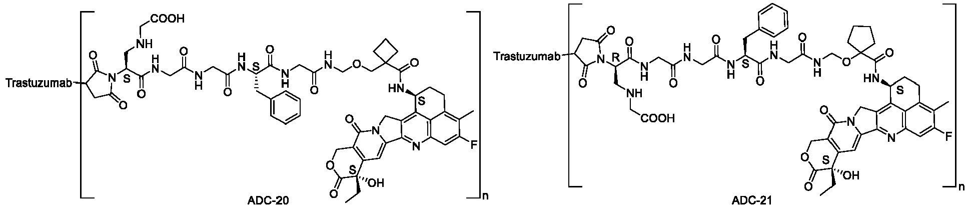 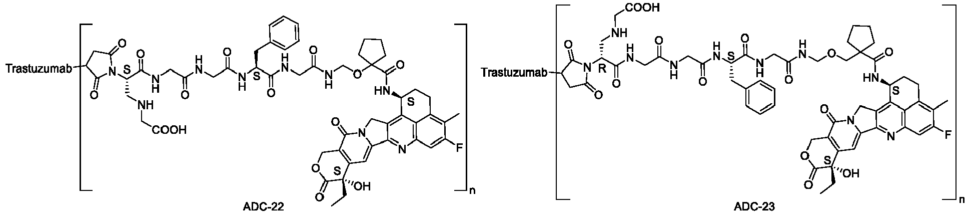 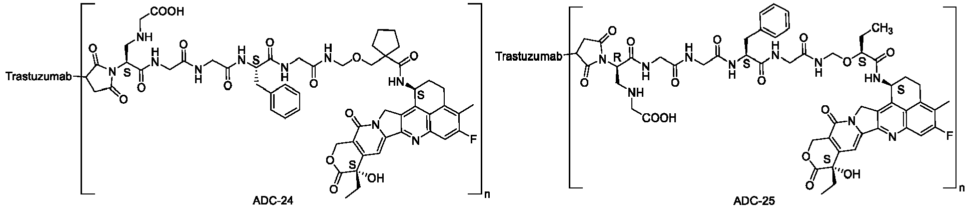 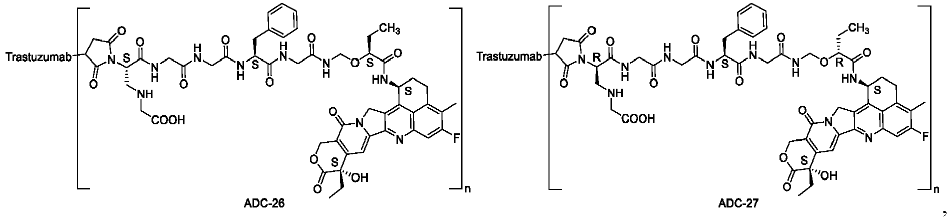 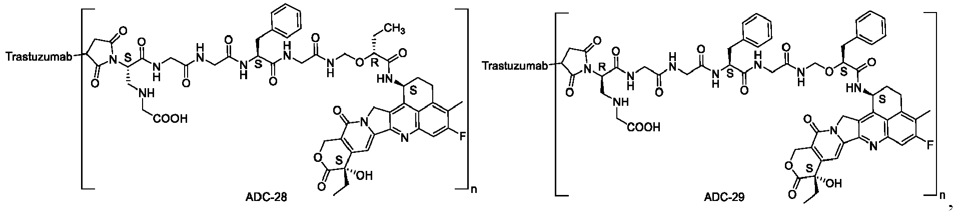 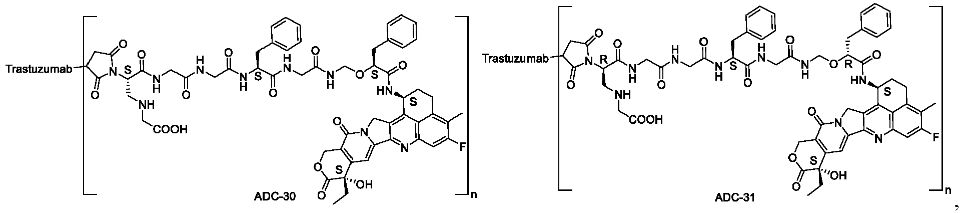 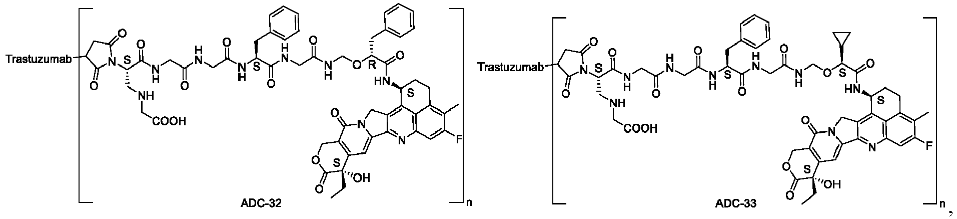 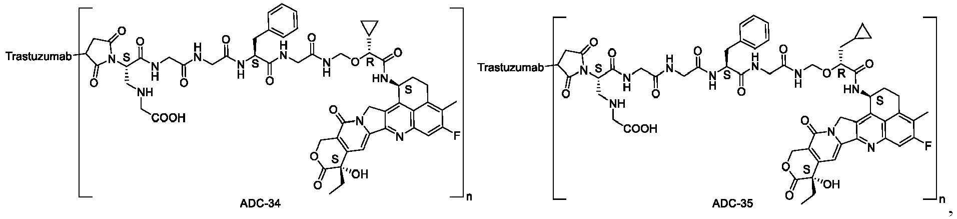 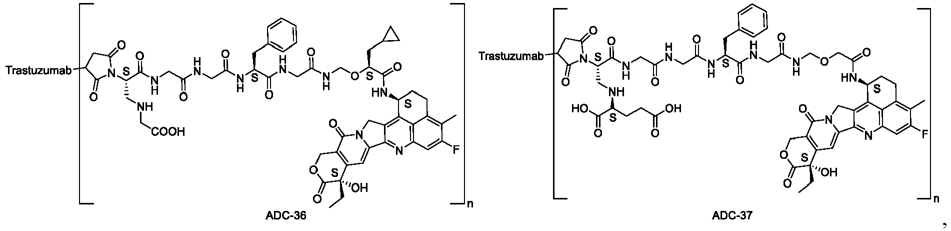 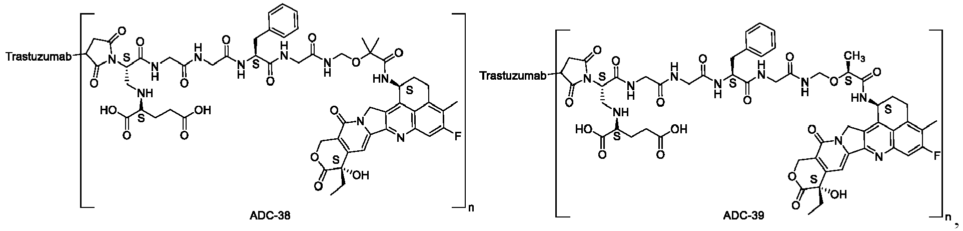 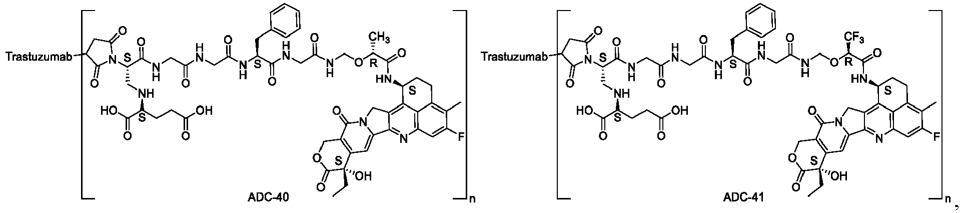 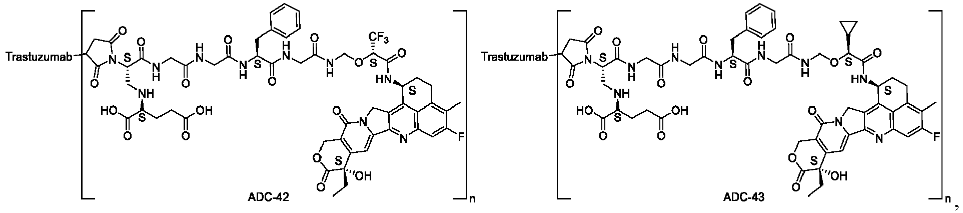 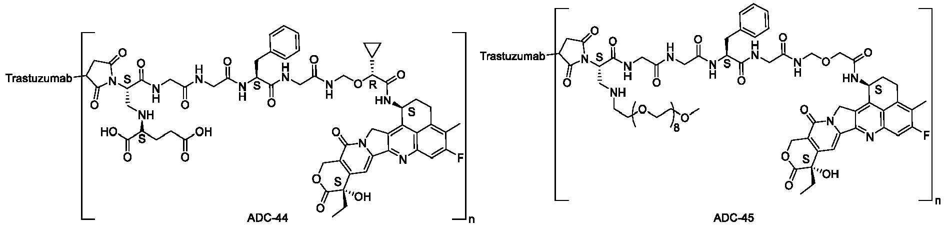 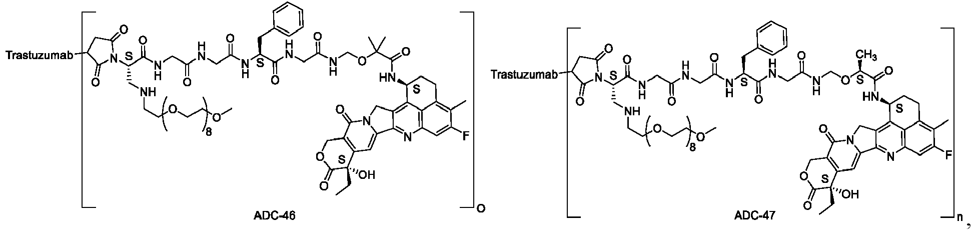 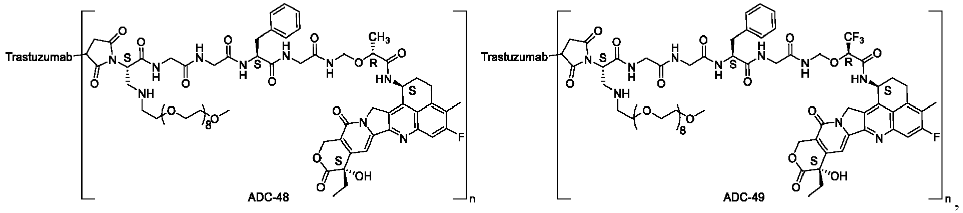 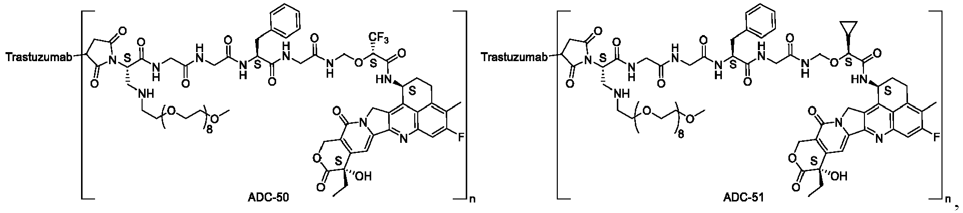 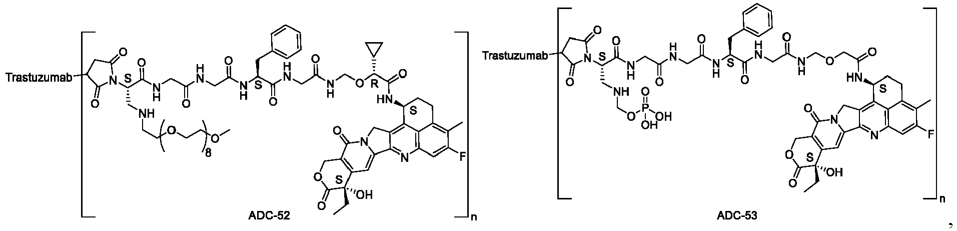 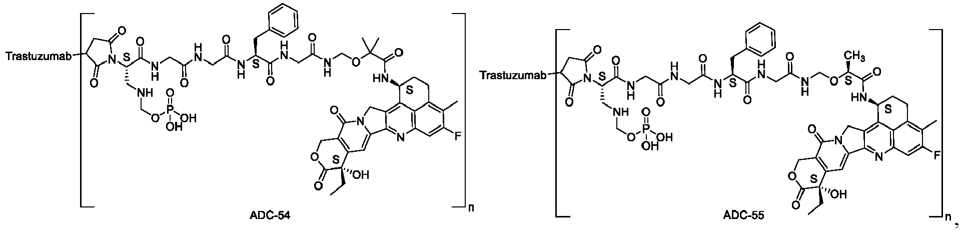 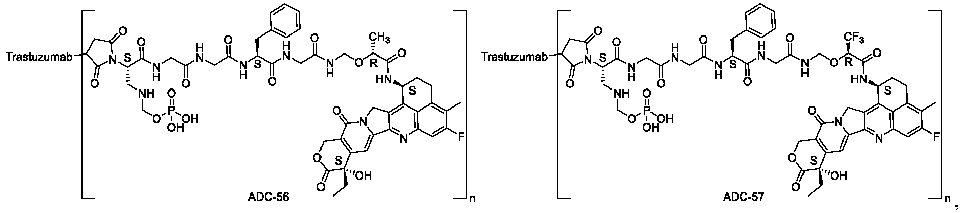 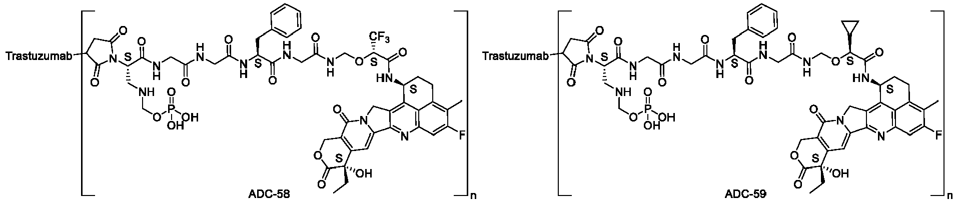 or 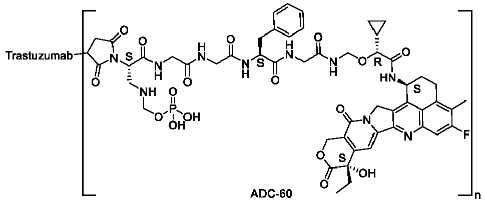 wherein n is selected from an integer of 1-10.

In one aspect, the application provides a method for preparing the disclosed linker-drug compound or a pharmaceutically acceptable salt thereof. In one embodiment, the method comprises the following steps: 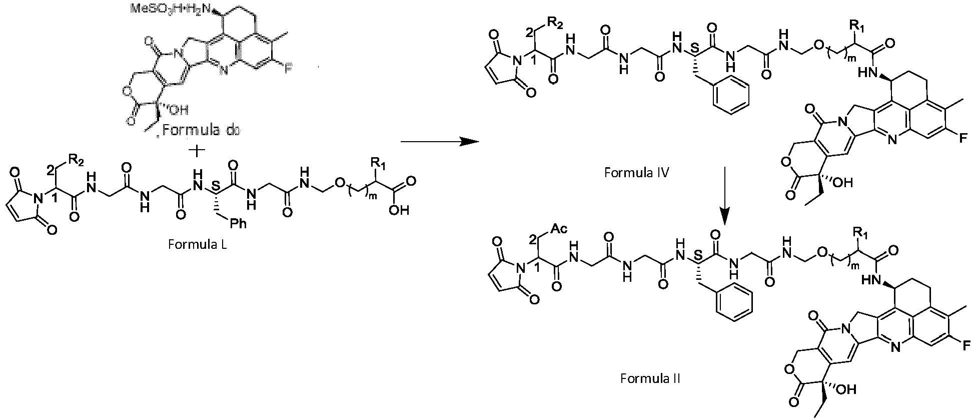
reacting a compound of formula L with Exatecan of formula d₀ or its salt in the presence of a condensing agent under an alkaline condition to provide a compound of formula IV, is then transformed into a compound of formula II;
wherein,
the position-1 carbon atom and the carbon atom connected to R ₁ each independently has the chirality of R or S configuration;
R₂ is a structure that can be converted into Ac; and
Ac, R₁, and m are as defined in formula II.

In another aspect, the present application provides a method for preparing a ligand-drug conjugate or its pharmaceutically acceptable salt thereof, said method comprising the steps of 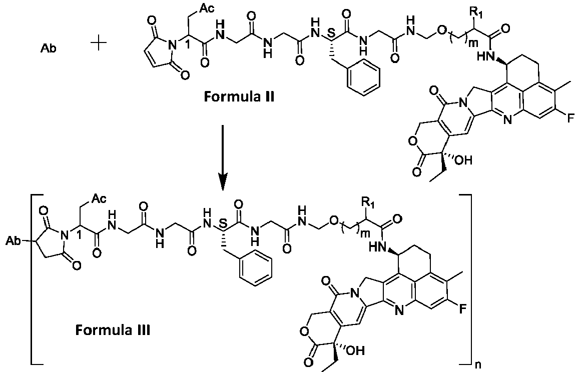
modifying the ligand Ab and then obtaining, by coupling reaction with formula II, the ligand-drug coupling compound shown in formula III;
wherein Ab is an antibody, an antibody fragment or a protein;
Ac is a hydrophilic moiety;
the chiral carbon at C1 position and the chiral carbon attached to R1 each independently has R or S configuration;
R1, m and n are as described in Formula II.

In one aspect, the application provides a pharmaceutical composition containing a therapeutically effective amount of the ligand-drug conjugate or a pharmaceutically acceptable salt or solvate thereof, and a pharmaceutically acceptable carrier, diluent, or excipient.

The pharmaceutically acceptable salt thereof includes, for example, sodium salt, potassium salt, calcium salt and magnesium salt formed with the carboxyl functional groups in the structural formulae disclosed in the specification, and acetate, trifluoroacetate, citrate, oxalate, tartrate, malate, nitrate, chloride, bromide, iodide, sulfate, bisulfate, phosphate, lactate, oleate, ascorbate, salicylate, formate, glutamate, methanesulfonate, ethanesulfonate, benzenesulfonate or p-toluenesulfonate formed with the nitrogen-containing functional groups in the structural formulae as disclosed herein.

In one embodiment, the application provides the ligand-drug conjugate or a pharmaceutically acceptable salt thereof, for use in the preparation of a medicament for the treatment of tumors, autoimmune diseases or infectious diseases, wherein an antibody of the ligand-drug conjugate specifically binds to a target cell of the tumor, the autoimmune disease or the infectious disease.

In one embodiment, the application provides ligand-drug conjugate or a pharmaceutically acceptable salt thereof, for use in the diagnosis and treatment of cancer, the cancer comprising breast cancer, ovarian cancer, cervical cancer, uterine cancer, prostate cancer, kidney cancer, urethral cancer, bladder cancer, liver cancer, gastric cancer, endometrial cancer, Salivary gland cancer, esophageal cancer, lung cancer, colon cancer, rectal cancer, colorectal cancer, bone cancer, skin cancer, thyroid cancer, pancreatic cancer, melanoma, glioma, neuroblastoma, glioma multiforme , Sarcoma, lymphoma and leukemia and other solid tumors or hematoma drugs.

### BRIEF DESCRIPTION OF THE DRAWINGS

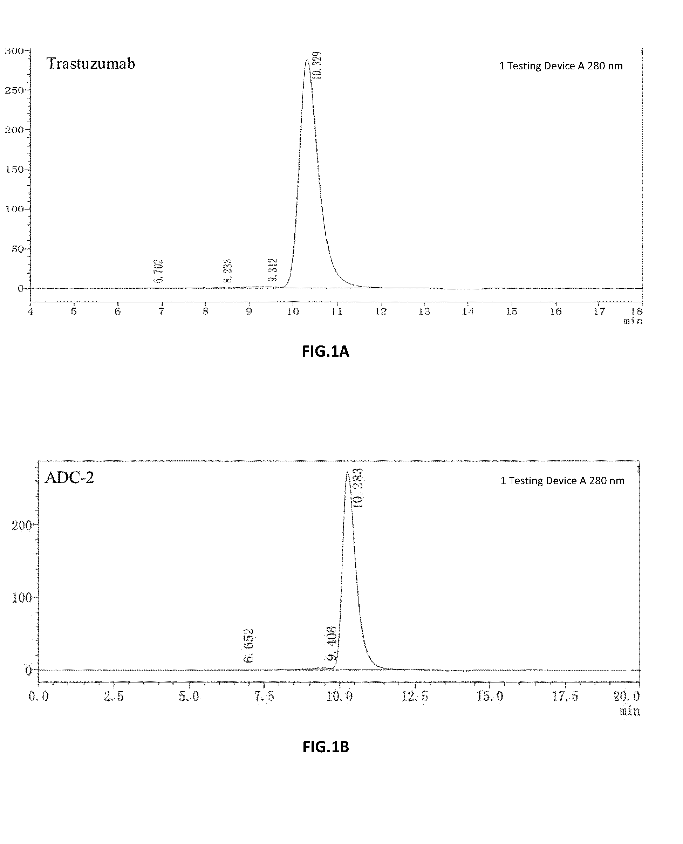

FIG. 1A shows the detection result of the monomer rate SEC-HPLC of Trastuzumab.
FIG. 1B shows the detection result of ADC-2 monomer rate SEC-HPLC.

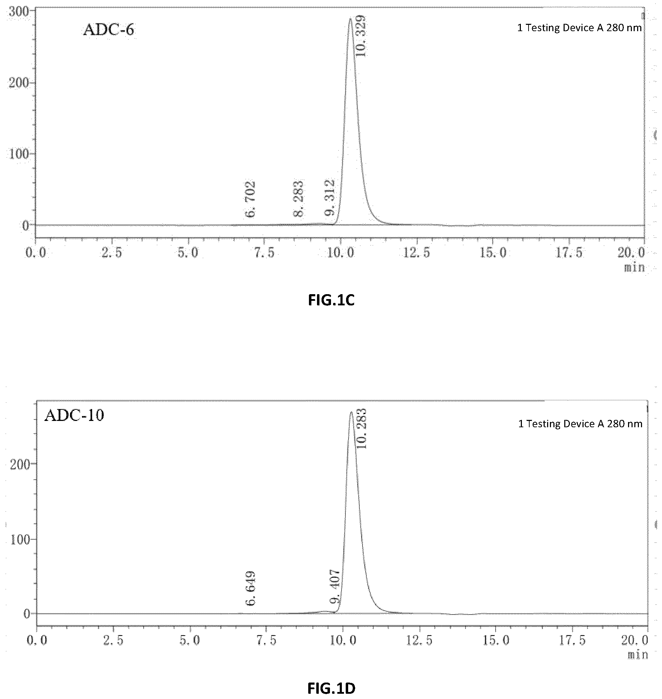

FIG. 1C shows the detection result of ADC-6 monomer rate SEC-HPLC.
FIG. 1D shows the detection result of ADC-10 monomer ratio SEC-HPLC.

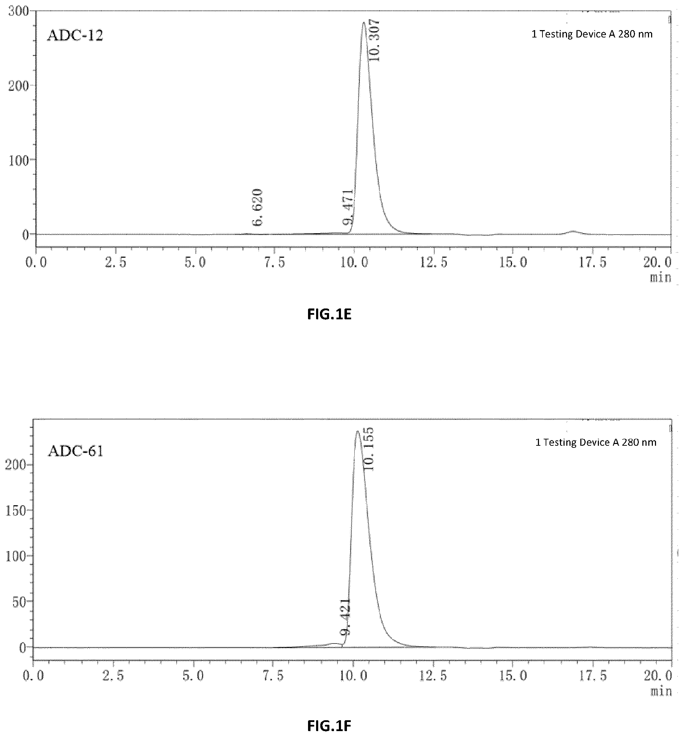

FIG. 1E shows the detection result of ADC-12 monomer ratio SEC-HPLC.
FIG. 1F shows the SEC-HPLC detection result of ADC-61 monomer ratio in control group.

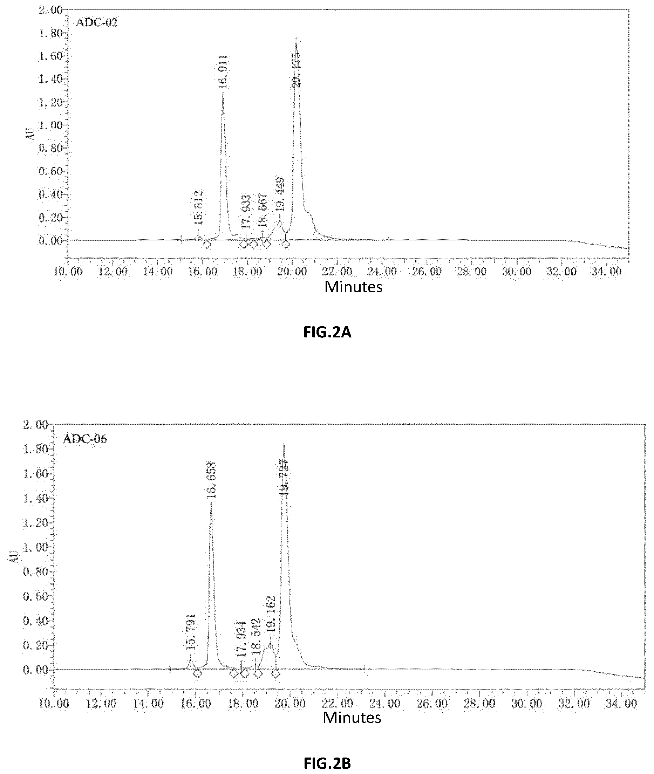

FIG. 2A shows the result of RP-HPLC detection of DAR (drug-antibody coupling ratio) value of ADC-02.
FIG. 2B shows the result of RP-HPLC detection of DAR (drug-antibody coupling ratio) value of ADC-06.

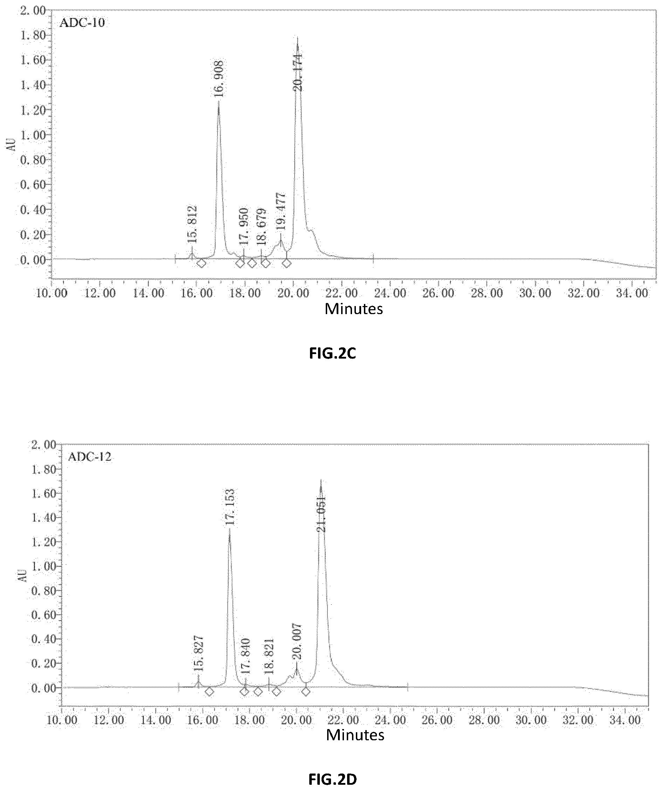

FIG. 2C shows the result of RP-HPLC detection of DAR (drug-antibody coupling ratio) value of ADC-10.
FIG. 2D shows the result of RP-HPLC detection of DAR (drug-antibody coupling ratio) value of ADC-12.

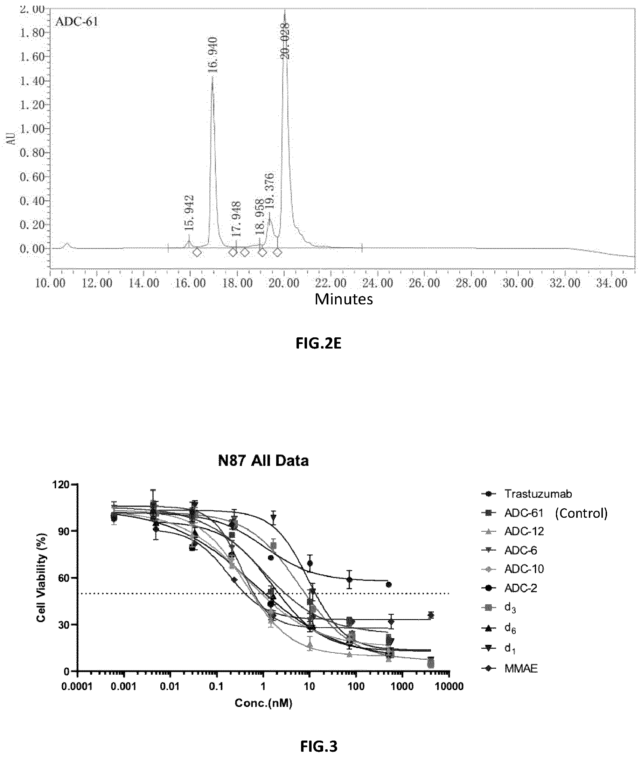

FIG. 2E shows the result of RP-HPLC detection of DAR (drug-antibody coupling ratio) value of control ADC-61.

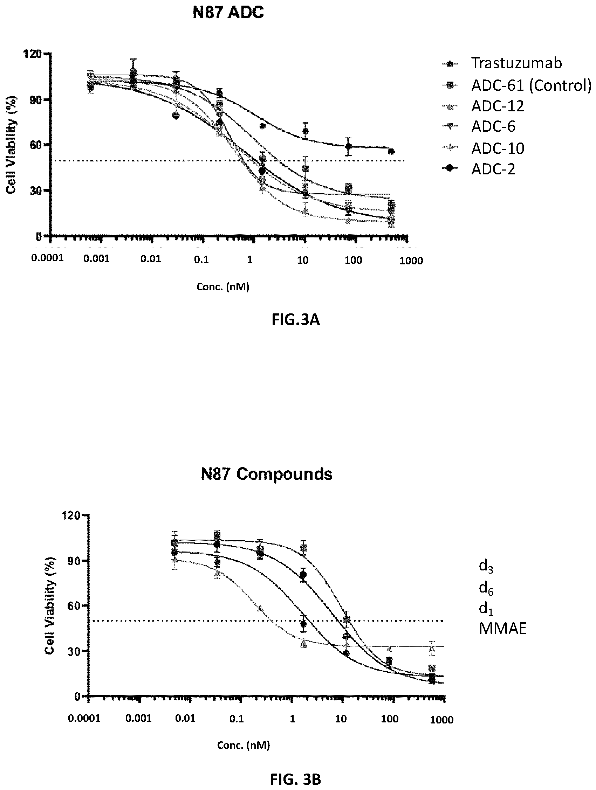

FIG. 3 shows the in vitro potency of ADC, a single drug and a naked antibody against the proliferation of N87 (human gastric cancer cells).
FIG. 3A shows the in vitro potency of ADC and naked antibody on the inhibition of N87 (human gastric cancer cell) proliferation.
FIG. 3B shows the in vitro potency of a single drug against the proliferation inhibition of N87 (human gastric cancer cells).

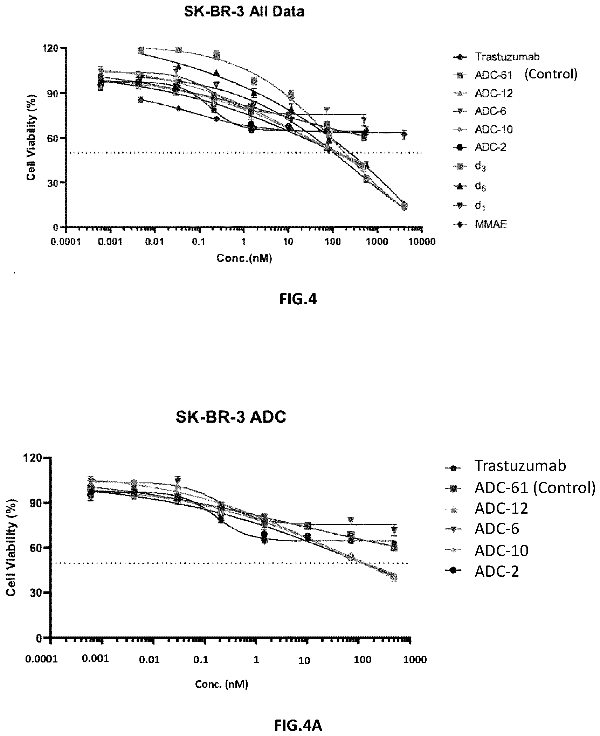

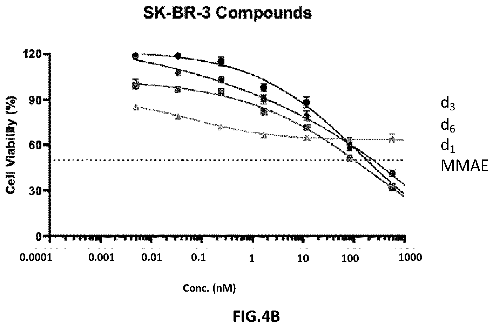

FIG. 4 shows the in vitro potency of ADC, single drug and naked antibody on SK-BR-3 (human breast adenocarcinoma cells) proliferation inhibition.
FIG. 4A shows the in vitro potency of ADC and naked antibody on SK-BR-3 (human breast adenocarcinoma cells) proliferation inhibition.
FIG. 4B shows the in vitro potency of single agents against SK-BR-3 (human breast adenocarcinoma cells) proliferation inhibition.

### DETAILED DESCRIPTION

### Abbreviations and Definitions

Unless defined otherwise, all technical and scientific terms used herein have the same meaning as commonly understood by one of ordinary skill in the art to which this disclosure belongs. Although any methods and materials similar or equivalent to those described herein can be used in the practice or testing of the disclosure, the preferred methods and materials are described herein. In describing and claiming the disclosure, the following terminology will be used in accordance with the definitions set out below.

When tradenames are used in the disclosure, applicants intend to include the formulation of the tradename product, the non-patent and active pharmaceutical portions of the tradename product.

As used herein, the following terms and phrases are intended to have the following meanings unless otherwise indicated. When a brand name is used herein, the brand name includes the product formulation, generic drug, and active pharmaceutical ingredient of the brand name product, unless the context indicates otherwise.

Unless stated to the contrary, terms used in the specification and claims have the following meanings.

The term "ligand" is a macromolecular compound capable of recognizing and binding to an antigen or receptor associated with a target cell. The role of the ligand is to present the drug to the target cell population to which the ligand binds, including but not limited to, a protein hormone, lectin, growth factor, antibody, or other molecule capable of binding to cells. In one embodiment, the ligand is represented as Ab, which may form a linkage with the linker unit through a heteroatom on the ligand, preferably an antibody or antigen-binding fragment thereof, which is selected from the group consisting of chimeric, humanized, fully human or murine antibody: preferably a monoclonal antibody.

The ligand unit is a targeting agent that specifically binds to the target moiety. The ligand is capable of specifically binding to cellular components or to other target molecules of interest. The target moiety or target is typically on the cell surface. In some aspects, the ligand unit functions to deliver the drug unit to the particular target cell population with which the ligand unit interacts. Ligands include, but are not limited to, proteins, polypeptides, and peptides, as well as non-proteins such as sugars. Suitable ligand units include, for example, antibodies, such as full-length (intact) antibodies and antigen-binding fragments thereof. In embodiments where the ligand unit is a non-antibody targeting agent, it may be a peptide or polypeptide, or a non-proteinaceous molecule. Examples of such targeting agents include interferons, lymphokines, hormones, growth factors and colony stimulating factors, vitamins, nutrient transport molecules, or any other cell binding molecule or substance. In some embodiments, the linker is covalently attached to the sulfur atom of the ligand. In some aspects, the sulfur atom is a sulfur atom of a cysteine residue, which forms an interchain disulfide bond of the antibody. In another aspect, the sulfur atom is a sulfur atom of a cysteine residue that has been introduced into a ligand unit, which forms an interchain disulfide bond of the antibody. In another aspect, the sulfur atom is a sulfur atom of a cysteine residue that has been introduced into a ligand unit (e.g., by site-directed mutagenesis or chemical reaction). In other aspects, the linker-bound sulfur atom is selected from cysteine residues that form interchain disulfide bonds of the antibody or additional cysteine residues that have been incorporated into ligand units (e.g., by site-directed mutagenesis or chemical reaction). In some embodiments, the numbering system is according to the EU index as in Kabat { [ Kabat E.A et al, (1991) ], Sequences of Immunological Interest (Sequences of proteins of Immunological Interest), fifth edition, NIH publication 91-3242 }.

As used herein, "antibody" or "antibody unit", within the scope of it, includes any part of an antibody structure. This unit may bind, reactively associate, or complex with a receptor, antigen or other receptor unit present in the targeted cell population. An antibody can be any protein or proteinaceous molecule that can bind, complex, or otherwise react with a portion of a cell population to be treated or biologically engineered. The antibody constituting the antibody-drug conjugate herein retains its antigen-binding ability in its original wild state. Thus, the antibodies herein are capable of specifically binding to an antigen. Antigens contemplated include, for example, Tumor Associated Antigens (TAA), cell surface receptor proteins and other cell surface molecules, cell survival regulators, cell proliferation regulators, molecules associated with tissue growth and differentiation (e.g., known or predicted to be functional), lymphokines, cytokines, molecules involved in the regulation of cell circulation, molecules involved in angiogenesis, and molecules associated with angiogenesis (e.g., known or predicted to be functional). The tumor associated factor may be a cluster differentiation factor (e.g., a CD protein).

Antibodies useful in antibody drug conjugates include, but are not limited to, antibodies directed against cell surface receptors and tumor associated antigens. Such tumor-associated antigens are well known in the art and can be prepared by antibody preparation methods and information well known in the art. In order to develop effective cellular level targets for cancer diagnosis and treatment, researchers have sought transmembrane or other tumor-associated polypeptides. These targets are capable of being specifically expressed on the surface of one or more cancer cells, while expressing little or no expression on the surface of one or more non-cancer cells. Typically, such tumor-associated polypeptides are more overexpressed on the surface of cancer cells relative to the surface of non-cancer cells. The confirmation of such tumor-associated factors can greatly improve the specific targeting property of antibody-based cancer treatment. For convenience, antigen-related information well known in the art is labeled as follows, including name, other names, and GenBank accession numbers. Nucleic acid and protein sequences corresponding to tumor associated antigens can be found in public databases, such as Genbank. The antibodies target the corresponding tumor associated antigens including all amino acid sequence variants and homologues, having at least 70%, 80%, 85%, 90% or 95% homology with the sequences identified in the references, or having biological properties and characteristics that are fully identical to the tumor associated antigen sequences in the cited references.

The term "inhibit" or "inhibition of" refers to a reduction in a detectable amount, or a complete prevention.

The term "cancer" refers to a physiological condition or disease characterized by unregulated cell growth. "tumor" includes cancer cells.

The term "autoimmune disease" is a disease or disorder that results from targeting an individual's own tissue or protein.

The term "drug" refers to a cytotoxic drug, denoted d, i.e., chemical molecules having a strong ability to damage normal growth of tumor cell. Cytotoxic drugs can kill tumor cells in principle at a high enough concentration, but due to lack of specificity, while killing tumor cells, they can also cause apoptosis of normal cells, resulting in serious side effects. The term includes toxins, such as small molecule toxins or enzymatically active toxins of bacterial, fungal, plant or animal origin, radioisotopes (e.g., At²¹¹, I¹³¹, I¹²⁵, Y⁹⁰, Re¹⁸⁶, Re¹⁸⁸, Sm¹⁵³, Bi²¹², P³² and Lu¹⁷⁶ radioactive isotopes), toxic drugs, chemotherapeutic drugs, antibiotics and nucleolytic enzymes, preferably toxic drugs.

The term "camptothecin drug" refers to a cytotoxic camptothecin and its derivatives, selected from, but not limited to, 10-hydroxycamptothecin, SN38 (7-ethyl-10-hydroxycamptothecin), topotecan, exatecan, irinotecan, or 9-nitro-10-hydroxycamptothecin and its derivatives or pharmaceutically acceptable salts.

The term "linker" or "linker fragment" or "linker unit" refers to a chemical moiety or bond that is linked at one end to a ligand and at the other end to a drug, and may be linked to a drug following attachment of another linker.

Linkers, including extenders, spacers and amino acid units, may be synthesized by methods known in the art, such as those described in US2005-0238649A1. The linker may be a "cleavable linker" that facilitates release of the drug in the cell. For example, acid-labile linkers (e.g., hydrazones), protease-sensitive (e.g., peptidase-sensitive) linkers, photolabile linkers, dimethyl linkers, or disulfide-containing linkers can be used (Chari et al Cancer Research 52: 127-; U.S. Pat. No.5,208,020.

According to the mechanism of drug release in cells, as used herein, a "linker" or a "linker of an antibody drug conjugate" can be divided into two categories: non-cleavable linkers and cleavable linkers. For ligand-drug conjugates containing a non-cleavable linker, the drug release mechanism is: after the conjugate is combined with antigen and endocytosed by cells, the antibody is enzymolyzed in lysosome to release active molecules consisting of small molecular drugs, linkers and antibody amino acid residues. The resulting structural change in the drug molecule does not reduce its cytotoxicity, but because the active molecule is charged (amino acid residues), it cannot penetrate into neighboring cells. Thus, such active drugs are unable to kill adjacent tumor cells that do not express the targeted antigen (antigen negative cells) (Ducry et al, 2010, Bioconjugate chem.21: 5-13).

The term "ligand-drug conjugate" refers to an antibody linked to a biologically active drug via a stable connecting unit. In one embodiment, the "ligand-drug conjugate" is an Antibody Drug Conjugate (ADC), which refers to a monoclonal antibody or antibody fragment linked to a biologically active toxic drug through a stable connecting unit.

The three letter codes and the one letter codes for amino acids used in this disclosure are as described in j.boil.chem.1968,243,3558.

The term "alkyl" refers to a saturated aliphatic hydrocarbon group, which is a straight or branched chain group containing 1 to 20 carbon atoms, preferably an alkyl group containing 1 to 12 carbon atoms, more preferably containing 1 to 10 carbons The most preferred is an alkyl group containing 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1 ,2-Dimethylpropyl, 2,2-Dimethylpropyl, 1-ethylpropyl, 2-methylbutyl, 3-methylbutyl, n-hexyl, 1-ethyl-2-Methylpropyl, 1,1,2-trimethylpropyl, 1,1-dimethylbutyl, 1,2-dimethylbutyl, 2,2-dimethylbutyl, 1,3 -Dimethylbutyl, 2-ethylbutyl, 2-methylpentyl, 3-methylpentyl, 4-methylpentyl, 2,3-dimethylbutyl, n-heptyl, 2 -Methylhexyl, 3-methylhexyl, 4-methylhexyl, 5-methylhexyl, 2,3-dimethylpentyl, 2,4-dimethylpentyl, 2,2-dimethyl Pentyl, 3,3-dimethylpentyl, 2-ethylpentyl, 3-ethylpentyl, n-octyl, 2,3-dimethylhexyl, 2,4-dimethylhexyl, 2,5-Dimethylhexyl, 2,2-dimethylhexyl, 3,3-dimethylhexyl, 4,4-dimethylhexyl, 2-ethylhexyl, 3-ethylhexyl, 4-ethyl 2-methylhexyl, 2-methyl-2-ethylpentyl, 2-methyl-3-ethylpentyl, n-nonyl, 2-methyl-2-ethylhexyl, 2-methyl-3-ethyl base hexyl, 2,2-diethylpentyl, n-decyl, 3,3-diethylhexyl, 2,2-diethylhexyl, and various branched isomers. More preferred are lower alkyl groups containing 1 to 6 carbon atoms. Non-limiting examples include methyl, ethyl, n-propyl, isopropyl, n-butyl, isobutyl, tert-butyl, and sec-butyl. Group, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethyl Butyl, 2,2-dimethylbutyl, 1,3-dimethylbutyl, 2-ethylbutyl, 2-methylpentyl, 3-methylpentyl, 4-methylpentyl Group, 2,3-dimethylbutyl, etc. Alkyl groups may be substituted or unsubstituted. When substituted, substituents may be substituted at any available attachment point. The substituents are preferably one or more of the following groups, which are independently selected from alkanes Group, alkenyl, alkynyl, alkoxy, alkylthio, alkylamino, halogen, mercapto, hydroxyl, nitro, cyano, cycloalkyl, heterocycloalkyl, aryl, heteroaryl, cycloalkane Oxy, heterocycloalkoxy, cycloalkylthio, heterocycloalkylthio, oxo.

The term "substituted alkyl" means that the hydrogen in the alkyl group is replaced with a substituent group, and unless otherwise indicated herein, the substituent group of the alkyl group may be a variety of groups selected from the group consisting of: -halogen, - OR', -NR'R ', -SR', -SiR 'R' ", -OC (O) R ', -C (O) R', -CO₂R', -CONR'R", -OC(O)NR'R", -NR"C(O)R', - NR'-C(O)NR"R‴, -NR"C(O)₂R', -NH-C(NH₂)=NH, -NR'C(NH₂)=NH, -NH-C(NH₂)=NR', -S(O)R', - S(O)₂R', -S(O)₂NR'R", -NR'S(O)₂R ", -CN and-NO₂. The number of substituents is from 0 to (2m '+ 1), where m' is the total number of carbon atoms in the group. R ', R ' and R ' each independently represent hydrogen, unsubstituted C₁₋₈Alkyl, unsubstituted aryl, heteroaryl, and optionally substituted heteroaryl,Aryl substituted by 1 to 3 halogens, unsubstituted C₁₋₈Alkyl radical, C₁₋₈Alkoxy or C₁₋₈Thioalkoxy, or unsubstituted aryl-C₁₋₄An alkyl group. When R 'and R' are attached to the same nitrogen atom, they may form a 3-, 4-, 5-, 6-or 7-membered ring together with the nitrogen atom. For example, -NR' R "includes 1-pyrrolidinyl and 4-morpholinyl.

The term "substituted alkyl" means that the hydrogen in the alkyl group is replaced by a substituent group. Unless otherwise specified in the context, the substituent of the alkyl group can be a variety of groups selected from the following group: -halogen, -OR', -NR'R", -SR', - SiR'R"R‴, -OC(O)R', -C(O)R', -CO₂R', -CONR'R", -OC(O) NR'R", -NR"C(O)R', -NR'-C(O)NR"R‴, - NR"C(O)₂R', -NH-C(NH ₂ )=NH, -NR'C(NH ₂ )=NH, -NH-C(NH ₂ )=NR', -S(O)R', -S(O)₂R', -S(O)₂NR'R ", -NR'S(O)₂R", -CN and -NO₂, the number of substituents ranges from 0 to (2m'+1), where m' is the total number of carbon atoms in the group. R', R" and R‴ each independently refers to hydrogen, unsubstituted C ₁₋₈ alkyl, unsubstituted aryl, aryl substituted with 1-3 halogens, unsubstituted C ₁₋₈ alkyl, C _{1- 8} alkoxy or C ₁₋₈ thioalkoxy, or unsubstituted aryl -C ₁₋₄ alkyl .R' and R"are attached to the same nitrogen atom, they may form together with the nitrogen atom, 3-, 4-, 5-, 6- or 7-membered ring. For example, -NR'R" includes 1-pyrrolidinyl and 4-morpholinyl.

The term "heteroalkyl" refers to an alkyl group containing one or more heteroatoms selected from N, O or S, wherein alkyl is as defined above.

The term "alkylene" refers to a saturated linear or branched aliphatic hydrocarbon group, which has two residues derived from the removal of two hydrogen atoms from the same carbon atom or two different carbon atoms of the parent alkane, which is A straight or branched chain group containing 1 to 20 carbon atoms, preferably containing 1 to 12 carbon atoms, more preferably an alkylene group containing 1 to 6 carbon atoms. Non-limiting examples of alkylene groups include, but are not limited to, methylene (-CH₂ -, 1,1-ethylene (-CH(CH₃)-), 1,2-ethylene (-CH₂CH₂)-, 1,1-propylene (-CH(CH₂CH₃ )-), 1,2-propylene (-CH₂CH(CH₃)-), 1,3-propylene (-CH₂CH₂CH₂-), 1,4-butylene (-CH₂CH₂CH₂CH₂-) and 1,5-butylene (-CH₂CH₂CH₂CH₂CH₂-), etc. The alkylene group may be substituted or unsubstituted. When substituted, the substituent may be substituted at any available point of attachment. The substituent is preferably independently optionally selected from alkyl, alkenyl, alkyne Group, alkoxy, alkylthio, alkylamino, halogen, mercapto, hydroxyl, nitro, cyano, cycloalkyl, heterocyclic, aryl, heteroaryl, cycloalkoxy, heterocycloalkoxy Substituted by one or more substituents in the group, cycloalkylthio group, heterocycloalkylthio group and oxo group.

The term "alkoxy" refers to -O- (alkyl) and-O- (cycloalkyl), wherein alkyl or cycloalkyl is as defined above. Non-limiting examples of alkoxy groups include: methoxy, ethoxy, propoxy, butoxy, cyclopropoxy, cyclobutoxy, cyclopentyloxy, cyclohexyloxy. Alkoxy may be optionally substituted or unsubstituted, and when substituted, the substituents are preferably one or more groups independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio.

The term "cycloalkyl" refers to a saturated or partially unsaturated monocyclic or polycyclic cyclic hydrocarbon substituent, the cycloalkyl ring containing from 3 to 20 carbon atoms, preferably from 3 to 12 carbon atoms, more preferably from 3 to 10 carbon atoms, and most preferably from 3 to 8 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like; polycyclic cycloalkyl groups include spiro, fused and bridged cycloalkyl groups.

The term "heterocyclyl" refers to a saturated or partially unsaturated mono-or polycyclic cyclic hydrocarbon substituent containing from 3 to 20 ring atoms wherein one or more of the ring atoms is selected from nitrogen, oxygen, or S(O)ₘ (wherein ₘ is an integer from 0 to 2), but does not include the ring moiety of -O-O-, -O-S- or -S-S-, the remaining ring atoms being carbon. Preferably 3 to 12 ring atoms, of which 1 to 4 are heteroatoms; more preferably, the cycloalkyl ring contains 3 to 10 ring atoms. Non-limiting examples of monocyclic heterocyclyl groups include pyrrolidinyl, piperidinyl, piperazinyl, morpholinyl, thiomorpholinyl, homopiperazinyl, and the like. Polycyclic heterocyclic groups include spiro, fused and bridged heterocyclic groups.

The term "cycloalkylalkyl" means an alkyl group substituted with one or more cycloalkyl groups, preferably one cycloalkyl group, wherein alkyl is as defined above, and wherein cycloalkyl is as defined above.

The term "haloalkyl" refers to an alkyl group substituted with one or more halogens, wherein alkyl is as defined above.

The term "deuterated alkyl" refers to an alkyl group substituted with one or more deuterium atoms, wherein alkyl is as defined above.

The term "hydroxy" refers to an-OH group.

The term "halogen" refers to fluorine, chlorine, bromine or iodine.

The term "amino" refers to the group-NH₂. The term "nitro" means-NO₂.

The term "amido" refers to-C(O)N (alkyl) or (cycloalkyl), wherein alkyl, cycloalkyl are as defined above.

The term "carboxylate" refers to-C(O)O (alkyl) or (cycloalkyl), wherein alkyl, cycloalkyl are as defined above.

The term "aryl" refers to a6 to 14 membered all carbon monocyclic or fused polycyclic (i.e., rings which share adjacent pairs of carbon atoms) group having a conjugated pielectron system, preferably 6 to 10 membered, such as phenyl. Aryl groups may be substituted or unsubstituted, and when substituted, the substituents are preferably one or more groups selected from, without limitation, alkyl, alkenyl, alkynyl, alkoxy, alkylthio, alkylamino, halogen, deuterium atoms, mercapto, hydroxyl, nitro, cyano, cycloalkyl, heterocycloalkyl, aryl, heteroaryl, cycloalkoxy, heterocycloalkoxy, cycloalkylthio, or heterocycloalkylthio.

The disclosure also includes various deuterated forms of formula I. Each available hydrogen atom attached to a carbon atom may be independently replaced by a deuterium atom. The person skilled in the art is able to synthesize the deuterated forms of formula i with reference to the relevant literature. Commercially available deuterated starting materials can be used in preparing the deuterated forms of formula i or they can be synthesized using conventional techniques using deuterated reagents, non-limiting examples of which include deuterated boranes, trideuterioborane tetrahydrofuran solutions, deuterated lithium aluminum hydrides, deuterated iodoethanes, and deuterated iodomethanes, among others.

The term "antibody" refers to an immunoglobulin, which is a tetrapeptide chain structure composed of two identical heavy chains and two identical light chains connected by interchain disulfide bonds. The amino acid composition and sequence of the constant region of the immunoglobulin heavy chain are different, so their antigenicity is also different. According to this, immunoglobulins can be divided into five categories, or isotypes of immunoglobulins, namely IgM, IgD, IgG, IgA, and IgE. The corresponding heavy chains are µ chain, δ chain, and γ chain, α chain and ε chain. The same type of Ig can be divided into different subclasses according to the difference in the amino acid composition of the hinge region and the number and position of heavy chain disulfide bonds. For example, IgG can be divided into IgG1, IgG2, IgG3, and IgG4. The light chain is divided into a kappa chain or a lambda chain by the difference of the constant region. Each of the five types of Ig can have a kappa chain or a lambda chain. In one embodiment, the antibodies may be specific antibodies against cell surface antigens on target cells. Non-limiting examples are the following antibodies: anti-EGFRvIII antibody, anti-DLL-3 antibody, anti-PSMA antibody, anti-CD70 antibody, and anti-MUC16 antibody , Anti-ENPP3 antibody, anti-TDGF1 antibody, anti-ETBR antibody, anti-MSLN antibody, anti-TIM-1 antibody, anti-LRRC15 antibody, anti-LIV-1 antibody, anti-CanAg/AFP antibody, anti-cladin 18.2 antibody, anti-Mesothelin antibody, anti-HER2 (ErbB2) antibody, anti-EGFR antibody, anti-c-MET antibody, anti-SLITRK6 antibody, anti-KIT/CD117 antibody, anti-STEAP1 antibody, anti-SLAMF7/CS1 antibody, anti-NaPi2B/SLC34A2 antibody, anti-GPNMB antibody, anti-HER3 (ErbB3) Antibody, anti-MUC1/CD227 antibody, anti-AXL antibody, anti-CD166 antibody, anti-B7-H3 (CD276) antibody, anti-PTK7/CCK4 antibody, anti-PRLR antibody, anti-EFNA4 antibody, anti-5T4 antibody, anti-NOTCH3 antibody, anti-Nectin 4 Antibodies, anti-TROP-2 antibodies, anti-CD142 antibodies, anti-CA6 antibodies, anti-GPR20 antibodies, anti-CD174 antibodies, anti-CD71 antibodies, anti-EphA2 antibodies, anti-LYPD3 antibodies, anti-FGFR2 antibodies, anti-FGFR3 antibodies, anti-FRα antibodies, anti-CEACAMs Antibody, anti-GCC antibody, anti-Integrin Av antibody, anti-CAIX antibody, anti-P-cadherin antibody, anti-GD3 antibody, anti-Cadherin 6 antibody, anti-LAMP1 antibody, anti-FLT3 antibody, anti-BCMA antibody, anti-CD79b antibody, anti-CD19 antibody, One or more of anti-CD33 antibody, anti-CD56 antibody, anti-CD74 antibody, anti-CD22 antibody, anti-CD30 antibody, anti-CD37 antibody, anti-CD138 antibody, anti-CD352 antibody, anti-CD25 antibody or anti-CD123 antibody; preferably trastuzumab Monoclonal antibody (Trastuzumab, trade name Herceptin), Pertuzumab (Pertuzumab, also known as 2C4, trade name Perjeta), Nimotuzumab (Nimotuzumab, trade name Taixinsheng), Enoblituzumab, Emibetuzumab, Inotuzumab, Pinatuzumab, Brentuximab, Gemtuzumab, Bivatuzumab, Lorvotuzumab, cBR96 and Glematumamab.

The term "solvate" or "solvate compound" means that the ligand-drug conjugate disclosed herein forms a pharmaceutically acceptable solvate with one or more solvent molecules, non-limiting examples of which include water, ethanol, acetonitrile, isopropanol, DMSO, ethyl acetate.

The term "drug loading" refers to the average amount of cytotoxic drug loaded per antibody in formula I and can also be expressed as the ratio of drug amount to antibody amount, and the drug loading can range from 0 to 12, preferably 1 to 10 cytotoxic drugs (D) attached per antibody (Ab). In one embodiment, the drug loading is represented as n, which may be an exemplary mean value of 1,2,3,4,5,6,7,8,9, 10. The average amount of drug per ADC molecule after the conjugation reaction can be identified by conventional methods such as UV/visible spectroscopy, mass spectrometry, ELISA assays and HPLC characterization.

In one embodiment, the cytotoxic drug is conjugated to the open interchain cysteine thiol-SH group and/or site-directed mutated cysteine thiol-SH group of the antibody via a linker, and generally, the number of drug molecules capable of being conjugated to the antibody in the conjugation reaction will be less than or equal to the theoretical maximum.

The loading of the ligand cytotoxic drug conjugate can be controlled by the following non-limiting methods, including:
(1) controlling the molar ratio of the connecting reagent to the monoclonal antibody,
(2) the reaction time and the temperature are controlled,
(3) different reagents were selected.

The preparation of the conventional pharmaceutical composition is shown in Chinese pharmacopoeia.

The term "pharmaceutically acceptable salt" or "pharmaceutically acceptable salt" refers to salts of the ligand-drug conjugates as disclosed herein, or salts of the compounds described herein, which are safe and effective for use in the body of a mammal and which possess the requisite biological activity, and the ligand-drug conjugates disclosed herein contain at least one carboxyl group and thus may form salts with bases, non-limiting examples of which include: sodium, potassium, calcium or magnesium salts, and the like.

The term "pharmaceutically acceptable salt" or "pharmaceutically acceptable salt" refers to salts of the antibody-drug conjugates disclosed herein, or salts of the compounds described herein, which are safe and effective for use in a mammalian body and which possess the requisite biological activity, the ligand-drug conjugate compounds disclosed herein contain at least one amino group and thus can form salts with acids, non-limiting examples of which include: hydrochloride, hydrobromide, hydroiodide, sulphate, hydrogen sulphate, citrate, acetate, succinate, ascorbate, oxalate, nitrate, sorbate, hydrogen phosphate, dihydrogen phosphate, salicylate, hydrogen citrate, tartrate, maleate, fumarate, formate, benzoate, methanesulphonate, ethanesulphonate, benzenesulphonate, p-toluenesulphonate.

"Acidic amino acid" means that the isoelectric point of the amino acid is less than 7, and acidic amino acid molecules often have one or more acidic groups such as carboxyl groups, and can be effectively ionized into negative ions in the structure to increase the hydrophilicity. The acidic amino acid may be a natural amino acid or an unnatural amino acid.

"Natural amino acid" refers to an amino acid synthesized by a living organism. Natural amino acids are generally L-shaped, with a few exceptions, such as glycine, including both natural and biosynthetic.

"Unnatural amino acid" refers to an amino acid obtained by synthetic means.

The disclosure will now be further illustrated by reference to specific examples, which are intended to be illustrative only and not to be limiting of the scope of the disclosure. Test methods without specific conditions noted in the following examples are generally performed according to conventional conditions or according to conditions recommended by the manufacturer. All percentages, ratios, or parts are by weight unless otherwise specified.

Unless otherwise defined, all technical and scientific terms used herein have the same meaning as is familiar to those skilled in the art.

In addition, any methods and materials similar or equivalent to those described herein can be used in the methods disclosed herein. The preferred embodiments and materials described herein are intended to be exemplary only.

### Example 1

### Synthesis of compound M1:

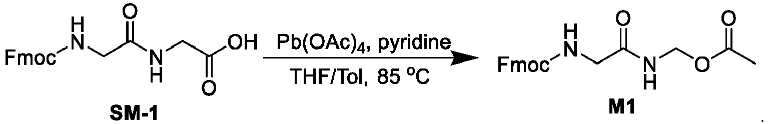

Add N-fluorenylmethoxycarbonyl-glycine (100g, 282mmol, 1.0eq), lead tetraacetate (175g, 553mmol, 1.4eq), 2000mL dry tetrahydrofuran and 670mL toluene into a 5000 mL single-neck flask; the reactants were stirred uniformly, protected by nitrogen, heated to 85 °C and reacted for 2.5 h; under TLC monitoring, after the reaction is finished, the mixture was cooled to room temperature and filtered. Concentrate the filtrate under reduced pressure, and purify the residue by column chromatography to obtain compound **M1** (87 g); LC-MS: [ M+NH₄]⁺=386.0.

### Example 2

### Synthesis of compound M3:

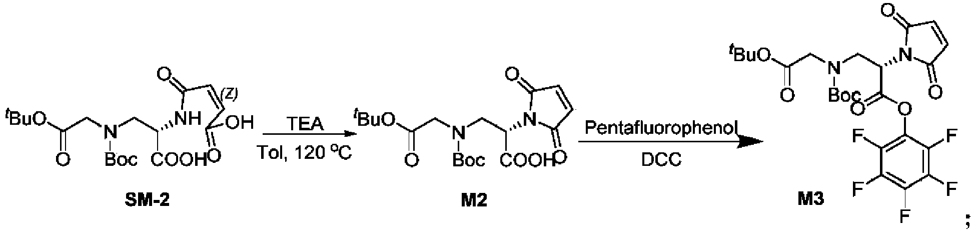

Add **SM-2** (synthesized according to the method disclosed in CN 108452321A) (40g, 96mmol, 1.0eq), triethylamine (26.7mL, 2.0eq), and toluene (400mL) to a 1000 mL single-neck flask and the mixture was heated to 120°C and refluxed for 2 hours. TLC monitoring almost entire reaction, cooling to 50 °C under, and removing solvent under reduced pressure. The mixture was dissolved in ethyl acetate (150mL) and water (40mL), and the pH was adjusted to 2-3 with 1M HCl while stirring in an ice bath, and liquid phases were separated. The aqueous layer was extracted once more with ethyl acetate, and the organic layers were combined and dried over anhydrous sodium sulfate. Filtration to concentrate to generate crude product as a paleyellow oil, which was purified by column chromatography (DCM : MeOH = 40:1) to yield compound **M2** (26.6 g); LC-MS: [ M + H ]⁺=399.3.

Add compound **M2** (26.5g, 60.5mmol, 1.0eq), pentafluorophenol (12.2g, 66.5mmol, 1.1eq), DCC (13.7g, 66.5mmol, 1.1eq) and THF (300mL) in a 1000mL single neck flask, react at room temperature for 30 minutes (monitored by TLC), and the insoluble material was filtered off by filtration. Directly prepare and purify the reaction liquid, concentrating the preparation liquid by a water pump at 35°C under reduced pressure in a water bath to remove acetonitrile, and freeze-drying to obtain a compound **M3** (31.5g), wherein the yield was 64%; LC-MS: [M+H]⁺=565.1.

### Example 3

### Synthesis of the Compound ent-M3:

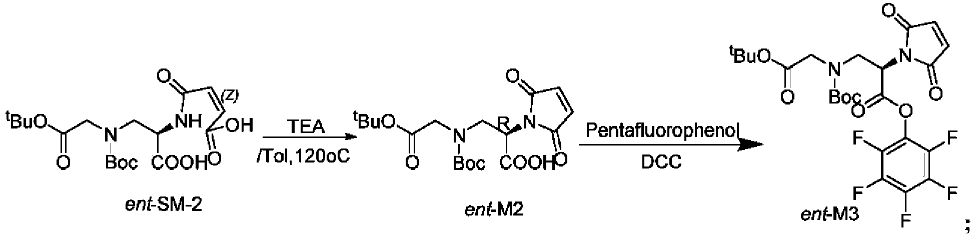

In reference to the synthetic route of example 2, compound ***ent*-M3** (27.8g) was obtained; LC-MS: [M+H]⁺=565.2.

### Example 4

### Synthesis of Compound 1:

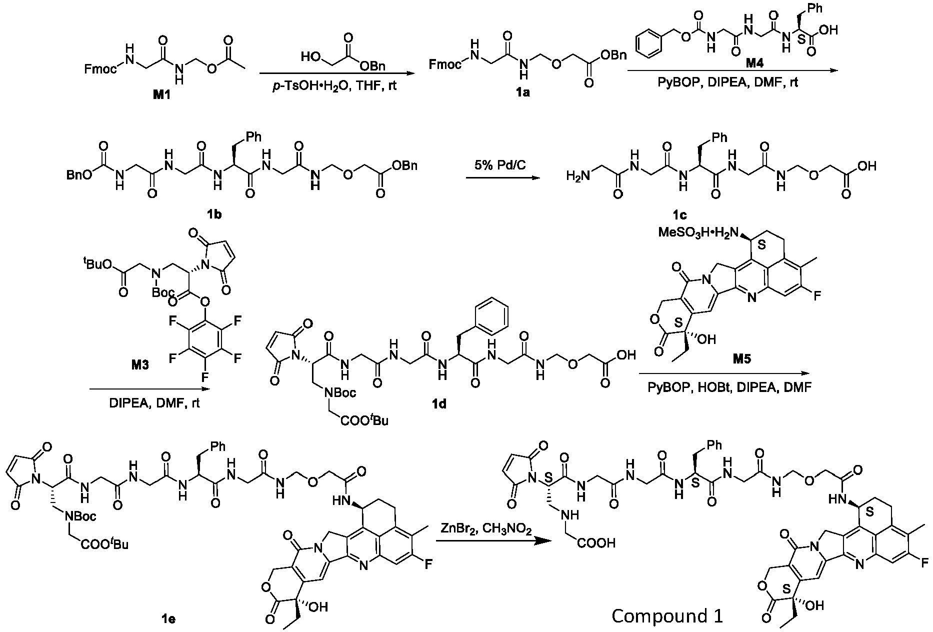

### Step 1: Compound 1a

Add **M1** (6g, 16.3mmol), 100mLTHF, p-toluenesulfonic acid monohydrate (0.31g, 1.63mmol) into a 250mL single-mouth flask, stir and cool to 0°C, add dropwise benzyl glycolate (5.4g, 32.6 mmol), after dripping, the temperature is naturally raised to room temperature for reaction (reaction is about 2-4h), monitored by TLC. After the reaction is over, add saturated NaHCO₃ solution, extract with ethyl acetate, wash with saturated sodium chloride solution, dry with anhydrous sodium sulfate, filter, and concentrate. The residue is purified by silica gel column (PE:EA=10:1-5:1 -1:1) to obtain **1a** (4g) with a yield of 52%; LC-MS: [M+H]⁺ =475.18.

### Step 2: Compound 1b

Add 1a (2g, 4.2mmol), 10mL DMF to a 25mL single-mouth flask, stir at 0°C, add DBU (766mg, 5.04mmol), react for 1h, TLC monitoring Fmoc deprotection is complete, set aside;

Take another 25mL single-mouth bottle and add **M4** (prepared with reference to the method published in patent CN111051330 A) (1.73g, 4.2mmol), PyBOP (2.61g, 5.04mmol), HOBt (680mg, 5.04mmol) and 10mL DMF, add under ice water bath DIPEA (830uL, 5.04mmol), continue to stir for 30 minutes, add the above reaction solution to the reaction flask, and warm to room temperature for reaction. After the reaction was finished while monitored by HPLC, the reaction solution was purified by the preparation liquid phase to obtain the product preparation solution. The preparation solution was extracted with dichloromethane, washed with saturated sodium chloride solution, dried with anhydrous sodium sulfate, filtered, and the filtrate was concentrated under reduced pressure to obtain solid **1b** (1.7g), the yield is 63%; LCMS: [M+H]⁺ =648.26.

### Step 3: Compound 1c

Add 1b (900mg, 1.39mmol) in a 25mL single-necked flask, dissolve in 15mL DMF, add 900mg 5% Pd/C, hydrogenation reaction for 2h, after the reaction is complete, filter to obtain the filtrate, directly used in the next reaction without purification.

### Step 4: Compound 1d

Place the crude product 1c in an ice-water bath, add DIPEA (235uL, 1.39mmol), and then add compound **M3** (784mg, 1.39mmol), after the addition, increase to room temperature and react for 1h. After the reaction was finished while being monitored by HPLC, the reaction solution was purified by high performance liquid phase to obtain a preparation solution, which was freeze-dried to obtain **1d** (504 mg); LC-MS: [M+H]⁺ =804.4.

### Step 5: Compound 1e

Add **1d** (500mg, 0.62mmol), **M5** (310mg, 0.62mmol), PyBOP (448mg, 0.86mmol), HOBt (116mg, 0.86mmol) and 15mL DMF to a 50mL single-mouth bottle, add DIPEA (378uL, 2.29 mmol) under ice water bath, warm to room temperature and react for 2h. After the reaction was finished while monitored by HPLC, the reaction solution was purified by high performance liquid phase to obtain a preparation solution of compound **1e,** which was freeze-dried to obtain **1e** (210 mg); LC-MS: [M+H]⁺ =1221.6.

### Step 6: Compound 1

Add **1e** (100mg, 0.081mmol), zinc bromide (368mg, 1.63mmol) and 5mL nitromethane to a 25mL single-necked flask, and react at 40°C for 1h. After the reaction was finished while monitored by HPLC, the solvent was removed by concentration under reduced pressure to obtain a crude product. The crude product was purified by high performance liquid phase to obtain a product preparation solution, which was freeze-dried to obtain solid compound **1** (60 mg); LC-MS: [M+H]⁺ =1065.3.

### Example 5

### Synthesis of compound 2:

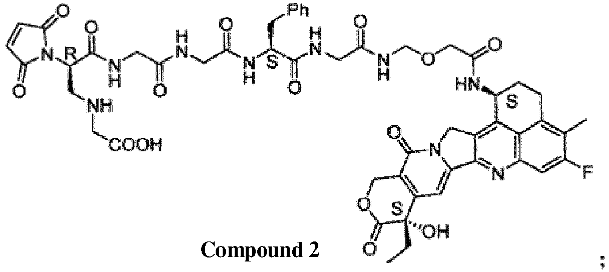

Refer to the synthetic route of Example 4 to obtain compound 2 (51 mg); LC-MS: [M+H]⁺ =1065.3.

### Example 6

### Synthesis of compound 3:

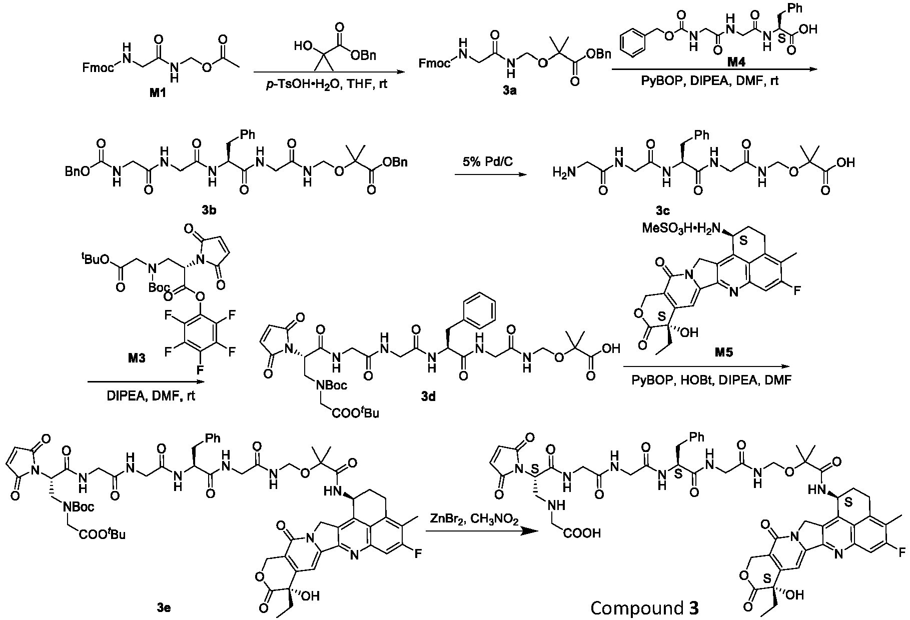

### Step 1: Compound 3a

Add **M1** (6g, 16.3mmol), 100mLTHF, p-toluenesulfonic acid monohydrate (0.31g, 1.63mmol) into a 250mL single-mouth flask, stir and cool to 0°C, add 2-hydroxy-2-methylpropane dropwise Benzyl acid ester (6.3g, 32.6mmol), after dripping, the temperature is naturally raised to room temperature for reaction (reaction is about 2-4h), monitored by TLC. After the reaction is over, add saturated NaHCO₃ solution, extract with ethyl acetate, wash with saturated sodium chloride solution, dry with anhydrous sodium sulfate, filter, and concentrate. The residue is purified by silica gel column (PE:EA=10:1-5:1 -2:1) to obtain **3a** (4.2g) with a yield of 52%; LC-MS: [M+H]⁺ =503.3.

### Step 2: Compound 3b

Add **3a** (2g, 4.0mmol), 10mL DMF to a 25mL single-mouth flask, stir at 0°C, add DBU (760mg, 5.0mmol), react for 1h, TLC monitoring until Fmoc deprotection is complete, set aside;

Add **M4** (1.65g, 4.0mmol), PyBOP (2.59g, 5.0mmol), HOBt (675mg, 5.0mmol) and 10mL DMF to another 25mL single-mouth bottle. Add DIPEA (823uL, 5.04mmol) under ice water bath and continue stirring For 30 minutes, the above reaction solution was added to the reaction flask, and the reaction was raised to room temperature. After the reaction was finished while monitored by HPLC, the reaction solution was purified by the preparation liquid phase to obtain the product preparation solution. The preparation solution was extracted with dichloromethane, washed with saturated sodium chloride solution, dried with anhydrous sodium sulfate, filtered, and the filtrate was concentrated under reduced pressure to obtain solid **3b** (1.4g), the yield is 53%; LC-MS: [M+H]⁺ =676.2.

### Step 3: Compound 3c

Add 3b (700mg, 1.04mmol) in a 25mL single-neck bottle, 10mL DMF dissolved, add 700mg 5% Pd/C, hydrogenation reaction for 1.5h, after the reaction is complete, filter to obtain the filtrate, directly used in the next reaction without purification .

### Step 4: Compound 3d

Place the crude product 3c in an ice-water bath, add DIPEA (210uL, 1.25mmol), and then add compound **M3** (704mg, 1.25mmol), after the addition, increase to room temperature and react for 1h. After the reaction was finished while monitored by HPLC, the reaction solution was purified by high performance liquid phase to obtain a preparation solution, which was freeze-dried to obtain **3d** (486 mg); LC-MS: [MH]⁻ =830.5.

### Step 5: Compound 3e

Add **3d** (300mg, 0.36mmol), **M5** (180mg, 0.36mmol), PyBOP (260mg, 0.5mmol), HOBt (67mg, 0.5mmol) and 10mL DMF to a 50mL single-mouth bottle, add DIPEA (219.5uL, 1.33mmol), warmed to room temperature and reacted for 3h. After the reaction was finished while monitored by HPLC, the reaction solution was purified by high performance liquid phase to obtain a preparation solution of compound 3e, which was freeze-dried to obtain **3e** (157 mg); LC-MS: [M+H]⁺ =1249.6.

### Step 6: Compound 3

Add **3e** (100mg, 0.08mmol), zinc bromide (360mg, 1.6mmol) and 5mL nitromethane into a 25mL single-necked flask, and react at 40°C for 1h. After the reaction was finished while monitored by HPLC, the solvent was removed by concentration under reduced pressure to obtain a crude product. The crude product was purified by high performance liquid phase to obtain a product preparation solution, which was freeze-dried to obtain solid compound 3 (64 mg); LC-MS: [M+H]⁺ =1093.1.

### Example 7

### Synthesis of compound 4:

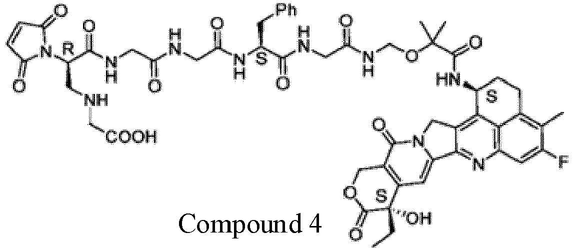

Refer to the synthetic route of Example 6 to obtain compound **4** (60 mg); LC-MS: [M+H]⁺ =1093.2.

### Example 8

### Synthesis of compound 5A:

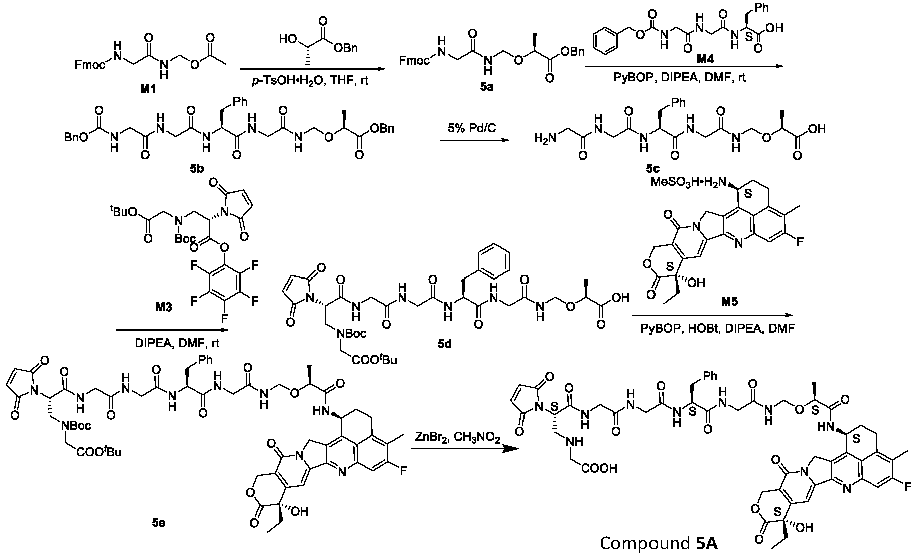

### Step 1: Compound 5a

Add M1 (500mg, 1.4mmol, 1.0eq), p-toluenesulfonic acid monohydrate (26mg, 0.1mmol, 0.1eq) and 10mL THF into a 25mL single-necked flask, stir well, reduce to 0°C, and then slowly add L -Benzyl lactate (1.2g, 7.0mmol, 5eq), after the addition, warm to room temperature for reaction. Monitoring by TLC, after the reaction, saturated NaHCO₃ solution was added, extracted with ethyl acetate, dried over anhydrous sodium sulfate, filtered, and concentrated. The residue was purified by reverse phase column to obtain 5a (400mg);
LC-MS: [M+NH ₄ ] ⁺ =506.2.
¹H NMR (400Mz, CDCl₃/CD₃OD): 1.39 (3H, d, J = 6.8 Hz), 3.78 (2H, t, J = 4.0 Hz), 4.17-4.27 (2H, m), 4.42 (2H,d, J=4.0Hz),4.72-4.85(2H,m), 5.11-5.58 (2H,m), 5.43 (1H,s), 7.06 (1H,t, J=8.0Hz), 7.25-7.33( 6H, m), 7.38 (2H, t, J = 8.0 Hz), 7.57 (2H, d, J = 8.0 Hz), 7.75 (2H, d, J = 8.0 Hz).

### Step 2: Compound 5b

Add compound **5a** (400mg, 0.8mmol, 1.0eq) and 4mL DMF into a 25mL single-necked flask, stir well, reduce to 0°C, and then slowly add DBU (137mg, 0.9mmol, 1.1eq), and warm to room temperature after the addition is complete reaction. TLC monitor until the end of the reaction, record it as reaction solution ①;
Add **M4** (372mg, 0.9mmol, 1.1eq), PyBOP (852mg, 1.6mmol, 2.0eq) and 3mL DMF to another 25mL single-necked flask, stir at room temperature for 5 minutes, add the reaction solution ①, react at room temperature, and monitor by HPLC. After the reaction was completed, the reaction solution was purified by HPLC to obtain compound 5b (326 mg); LC-MS: [M+NH ₄ ] ⁺ =679.2.

### Step 3: Compound 5c

**5b** (4.0 g, 6.05 mmol, 1.0 eq) was added to a 100 mL single-necked flask, DMF (60 mL) was dissolved, and 5% Pd/C (4 g) was added, and the hydrogenation reaction was carried out at room temperature for 4 h (using HPLC to monitor the progress of the reaction). The Pd/C was filtered, and the filtrate was not concentrated, and was directly placed in an ice-water bath (about 0°C) for later use.

### Step 4: Compound 5d

Place the crude product 5c in an ice-water bath, add DIPEA (1.1 mL, 1.1 eq), and then add compound **M3** (3.4 g, 6.05 mmol), and increase to room temperature for 2 hours after the addition. The reaction was monitored by HPLC, and the reaction solution was purified by high performance liquid phase to obtain a preparation solution. The preparation solution was lyophilized to obtain 5d (3.15 g); LC-MS: [MH]⁻ =816.3.

### Step 5: Compound 5e

Add 5d (2.07g, 2.53mmol, 1.0eq), **M5** (1.35g, 2.53mmol, 1.0eq), PyBOP (1.98g, 3.79mmol, 1.5eq), HOBt (0.51g, 3.79mmol, 1.5 eq) and DMF (40 mL), DIPEA (1.05 mL, 1.5 eq) was added under ice-water bath, and heated to room temperature for 2 h (monitored by HPLC). The reaction solution was directly purified by preparation, the preparation solution was concentrated in a vacuum water bath at 35° C. to remove acetonitrile, and lyophilized to obtain compound 5e (1.92 g) with a yield of 61%; LC-MS: [M+H]⁺ =1235.4.

### Step 6: Compound 5A

Add compound **5e** (1.0g, 0.8mmol, 1.0eq), 35mL of nitromethane to a 100mL single-necked flask, and then add zinc bromide (3.64g, 16mmol, 20.0eq) after dissolution, oil bath at 40°C (preheat and stabilize in advance) ) After reacting for 30 minutes, the nitromethane was removed by concentration in a water pump under reduced pressure water bath at 45°C. to obtain a yellow residue solid (monitored by HPLC). Prepared by acid method to obtain the preparation solution of compound 5A. The preparation solution was concentrated in a water pump under reduced pressure water bath at 35°C. to remove acetonitrile, and lyophilized to obtain compound 5A (786 mg) with a yield of 90%.

LC-MS: [M+H]⁺ =1079.4;
¹H NMR(400MHz,DMSO-d6)δ9.39-9.02(m,1H), 8.70(t, J=6.5Hz,1H), 8.64 (t, J=5.7Hz,1H), 8.56(d, J = 8.8Hz,1H),8.34(t, J = 5.7Hz,1H), 8.16(d, J = 8.2Hz,1H), 8.01(t, J = 5.5Hz,1H),7.71(d, J=10.9 Hz, 1H), 7.30 (s, 1H), 7.28-7.15 (m, 4H), 7.14 (s, 2H), 5.53 (dd, J = 14.5, 6.4 Hz, 1H), 5.49-5.34 (m, 2H) ,5.22(d, J=18.8Hz,1H),5.09(d, J=18.7Hz,1H),5.03(dd, J=9.6,3.9Hz,1H),4.73(dd, J=9.9,6.9Hz, 1H), 4.59 (dd, J = 10.1, 6.5 Hz, 1H), 4.49 (ddd, J = 13.2, 8.6, 4.4 Hz, 1H), 4.14 (dd, J = 13.3, 6.6 Hz, 2H), 3.93 (s, 2H), 3.84 (dd, J = 16.5, 6.3 Hz, 1H), 3.76 (dd, J = 16.9, 5.7 Hz, 2H), 3.70 (d, J = 5.2 Hz, 2H), 3.60 (dd, J = 16.7, 5.4 Hz, 1H), 3.52 (dd, J = 16.4, 5.1 Hz, 1H), 3.45 (dd, J = 12.8, 10.1 Hz, 1H), 3.25-3.15 (m, 1H), 3.14-3.05 (m ,1H),3.01(dd, J=13.7,4.1Hz,1H),2.73(dd, J=13.5,9.8Hz,1H),2.54-2.47(m,1H),2.33(s,2H),2.17( d, J = 5.5 Hz, 2H), 1.91-1.79 (m, 2H), 1.33 (d, J = 6.6 Hz, 2H), 0.87 (t, J = 7.3 Hz, 2H).

### Example 9

### Synthesis of compound 5B:

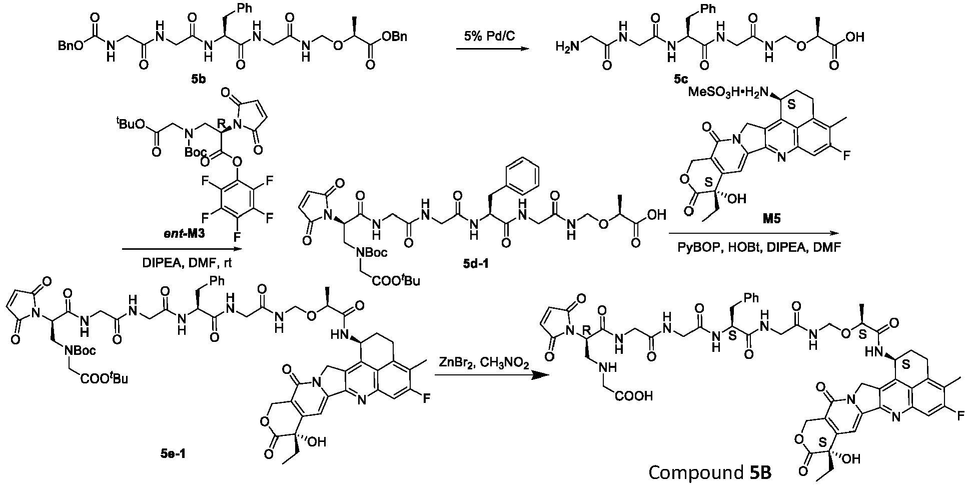

### Step 1: Compound 5d-1

Compound **5b** (300 mg, 0.45 mmol, 1.0 eq) and DMF (3 mL) were added to a 25 mL single-necked flask, and the mixture was stirred to clear the solution, and 5% Pd/C (300 mg) was added. Hydrogen replacement was performed three times. The hydrogenation reaction was 2h, and the reaction was monitored by HPLC. After the reaction, Pd/C was removed by filtration, the filtrate was cooled to 0-5°C, DIPEA (65mg, 0.5mmol, 1.1eq) was added, and **ent-M3** (255mg, 0.45mmol) was added to the filtrate. After the addition, the temperature was raised to The reaction was conducted at 20±5°C for 1 hour, and the end of the reaction was monitored by HPLC. After the reaction, the product was prepared and purified by HPLC, and the product preparation was collected and lyophilized to obtain compound 5d-1 (200 mg) with a yield of 54%; LC-MS: [MH]⁻ =816.3.

### Step 2: Compound Se-1

Add compound 5d-1 (200mg, 0.24mmol, 1.0eq), **M5** (127mg, 0.24mmol, 1.0eq), PyBOP (187mg, 0.36mmol, 1.2eq), HOBt (48mg, 0.36mmol, 1.2eq) into a 25mL single-mouth flask eq) and DMF (6mL), cooled to 0-5°C in an ice-water bath, add DIPEA (62mg, 0.48mmol, 2.0eq), after the addition, the temperature was raised to 20±5°C and reacted for 2h. HPLC monitored the completion of the reaction. The reaction solution was directly prepared and purified by HPLC, and the product preparation solution was collected and lyophilized to obtain compound 5e-1 (162.8 mg); LC-MS: [M+H]⁺ =1235.4.

### Step 3: Compound 5B

Add compound 5e-1 (110mg, 0.089mmol, 1.0eq), ZnBr ₂ (400mg, 1.78mmol, 20.0eq) and CH₃NO₂ (10mL) into a 25mL single-necked flask in sequence. After the addition, the temperature is raised to 40°C for 0.5 h, stop the reaction, the reaction solution was directly rotary dried under reduced pressure at 45°C to obtain a yellow solid, and a sample was taken by HPLC to monitor the reaction. The spin-dried solid was directly prepared and purified by HPLC. The product preparation was collected and lyophilized to obtain compound 5B (73.4 mg) with a yield of 76.5%; LC-MS: [M+H]⁺ =1079.4.

### Example 10

### Preparation of compound 6A:

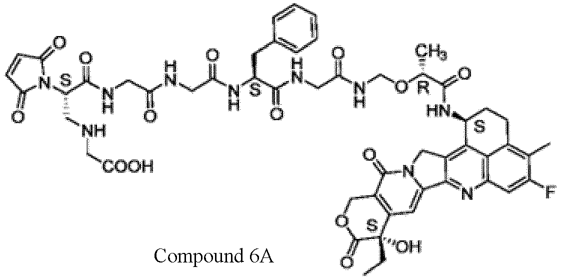

Refer to the synthetic route of Example 8 to obtain compound **6A** (71 mg); LC-MS: [M+H]⁺ =1079.4.

### Example 11

### Preparation of compound 6B:

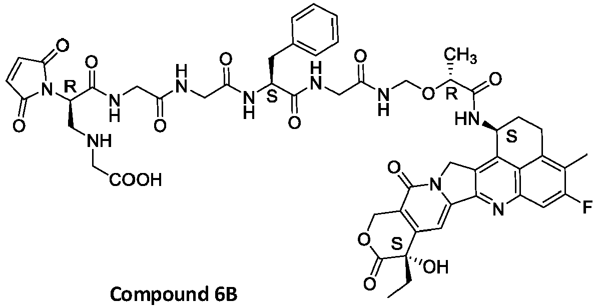

Refer to the synthetic route of Example 9 to obtain compound **6B** (59 mg); LC-MS: [M+H]⁺ =1079.4.

### Example 12

### Preparation of compound 7A and 7B:

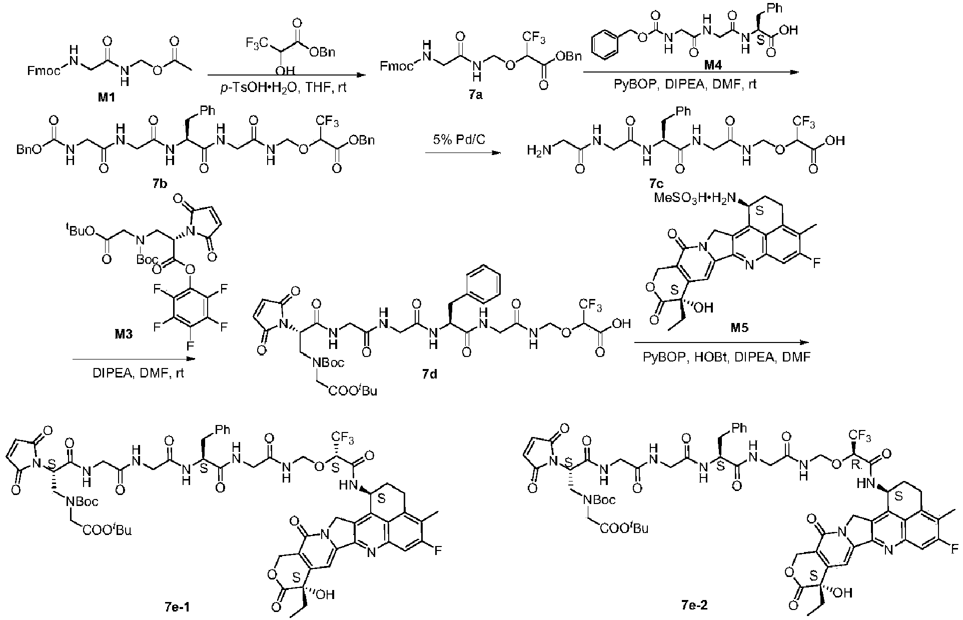

### Step 1: Compound 7a

Add **M1** (10g, 27.1mmol), 3,3,3-trifluoro benzyl lactate (prepared with reference to the method published in patent WO2020063673A1) (12.7g, 54.3mmol), zinc acetate (9.96g, 54.3mmol) in a 250mL single-mouth bottle ), and 100mL of toluene, heated to 100°C for 4h. After the reaction was completed, the temperature was lowered to room temperature, the insoluble matter was removed by filtration, and the filtrate was concentrated to obtain a crude product. The crude product was purified by silica gel column chromatography (PE:EA=10:1-5:1-2:1) to obtain 5.15g of the target product, with a yield of 35.1%; LC-MS: [M+H]+ =543.17.

### Step 2: Compound 7b

Add **7a** (5g, 9.2mmol) and 15mL DMF to a 50mL single-necked flask, after dissolving it, add DBU (1.68g, 11mmol) in an ice-water bath, and react for 1 hour, which is recorded as reaction solution①;
Take another 50mL single-mouth bottle, add **M4** (3.8g, 9.2mmol), PyBOP (5.75g, 11mmol), HOBt (1.49g, 11mmol) and 10mL DMF. After dissolving, add DIPEA (1.82mL, 11mmol) under ice water bath. ), continue the reaction for 30 minutes, add the reaction solution ①, and warm to room temperature for 2 hours. The reaction progress was monitored by HPLC. After the reaction was completed, the reaction solution was purified by high performance liquid phase to obtain a preparation solution. The preparation liquid was extracted with dichloromethane, washed with saturated sodium chloride solution, dried with anhydrous sodium sulfate, filtered, and concentrated to obtain 4.1g of solid, with a yield of 62.3%; LC-MS: [M+H]⁺ =716.25.

### Step 3: Compound 7d

Add **7b** (900mg, 1.26mmol) in a 25mL single-necked flask, after 15mL DMF is dissolved, add 900mg 5% Pd/C, hydrogenation reaction for 2h, the reaction is complete, filter, place the filtrate in an ice water bath, add DIPEA (228uL, 1.38mmol), then **M3** (712mg, 1,26mmol) was added, and after the addition, the temperature was raised to room temperature and reacted for 1h. The reaction was monitored by HPLC, and the reaction solution was purified by high performance liquid phase to obtain a preparation solution. The preparation solution was lyophilized to obtain 525 mg of the product with a yield of 47.9%; LC-MS: [MH]⁻ =870.33.

### Step 4: Compound 7e

Add **7d** (500mg, 0.57mmol), **M5** (305mg, 0.57mmol), PyBOP (448mg, 0.86mmol), HOBt (116mg, 0.86mmol) and 15mL DMF to a 50mL single-mouth bottle, add DIPEA (378uL, 2.29) under ice water bath mmol), warm to room temperature and react for 2h. After the reaction was finished while monitored by HPLC, the reaction solution was purified by high performance liquid phase to obtain the preparation solutions of compound **7e-1** and compound **7e-2.** The preparation solutions were respectively lyophilized to obtain 150 mg of compound **7e-1**, LC-MS: [M+H]⁺ =1289.46; 220 mg of compound **7e-2,** LC-MS: [M+H]⁺ =1289.46.

### Step 5: Compound 7A

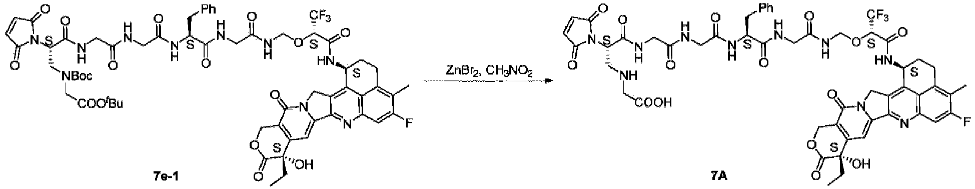

Add **7e-1** (100mg, 0.077mmol), zinc bromide (349mg, 1.55mmol) and 5mL nitromethane into a 25mL single-mouth flask, and react at 40°C for 1h. After the reaction was finished while monitored by HPLC, the solvent was removed by concentration under reduced pressure to obtain a crude product. The crude product was purified by high performance liquid phase to obtain a product preparation solution, which was freeze-dried to obtain a solid (52mg); TOF result: 1133.3613.

### Step 6: Compound 7B

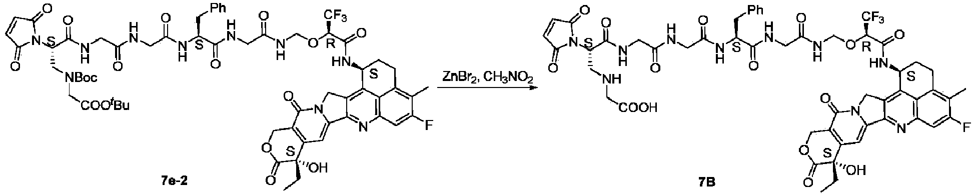

Add **7e-2** (100mg, 0.077mmol), zinc bromide (349mg, 1.55mmol) and 5mL nitromethane into a 25mL single-necked flask, and react at 40°C for 1h. After the reaction was finished while monitored by HPLC, the solvent was removed by concentration under reduced pressure to obtain a crude product. The crude product was purified by high performance liquid phase to obtain a product preparation solution, which was freeze-dried to obtain a solid (63mg); TOF result: 1133.3668.

### Example 13

### Synthesis of compounds 8A and 8B:

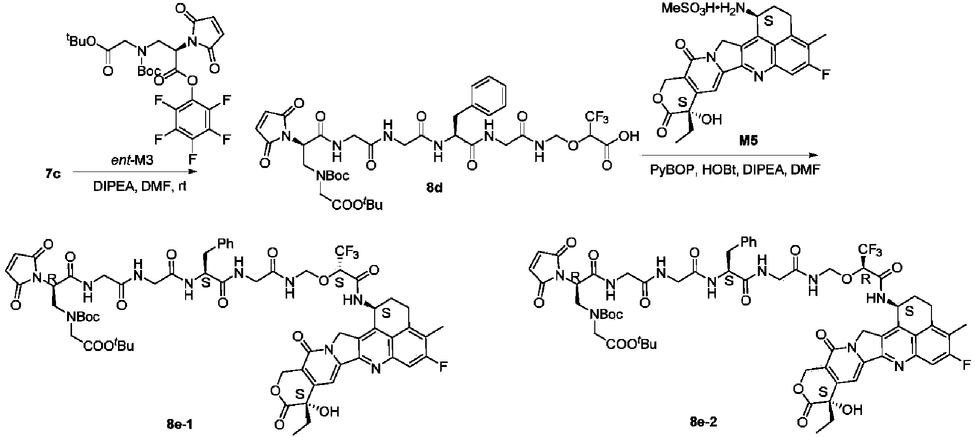

### Step 1: Compound 8d

7c (900mg, 1.83mmol) was added to a 25mL single-necked flask. After 20mL of DMF was dissolved, DIPEA (303uL, 1.83mmol) was added, then **ent-M3** (1034mg, 1.83mmol) was added, and the mixture was heated to room temperature and reacted for 1h. After the reaction was finished while monitored by HPLC, the reaction solution was purified by high performance liquid phase to obtain a preparation solution. The preparation solution was lyophilized to obtain 613 mg of the product with a yield of 38.5%; LC-MS: [MH]⁻ =870.32.

### Step 2: Compound 8e-1 and Compound 8e-2

Add 8d (500mg, 0.57mmol), **M5** (305mg, 0.57mmol), PyBOP (448mg, 0.86mmol), HOBt (116mg, 0.86mmol) and 15mL DMF to a 50mL single-mouth bottle, add DIPEA (378uL, 2.29 mmol) under ice water bath, warm to room temperature and react for 2h. After the reaction was finished while monitored by HPLC, the reaction solution was purified by high performance liquid phase to obtain the preparation solutions of compound **8e-1** and compound **8e-2**. The preparation solutions were respectively freeze-dried to obtain 140 mg of compound **8e-1** and 210 mg of compound 8e-2. LC-MS of compound **8e-1:** [M+H]⁺ =1289.47; LC-MS of compound **8e-2:** [M+H]⁺ =1289.47.

### Step 3: Compound 8A

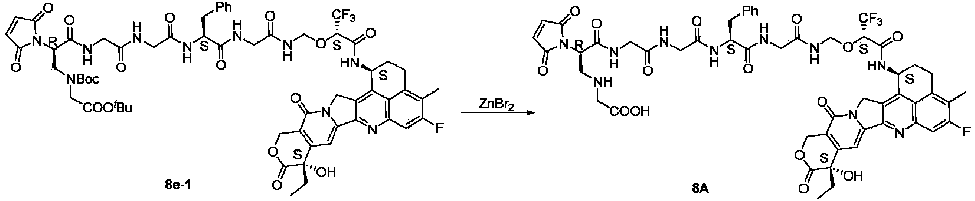

Compound **8e-1** (100 mg, 0.077 mmol), zinc bromide (349 mg, 1.55 mmol) and 5 mL of nitromethane were added to a 25 mL single-necked flask, and reacted at 40° C. for 1 h. After the reaction was finished while monitored by HPLC, the solvent was removed by concentration under reduced pressure to obtain a crude product. The crude product was purified by high performance liquid phase to obtain a product preparation solution, which was freeze-dried to obtain a solid (50mg); TOF result: 1133.3623.

### Step 4: Compound 8B

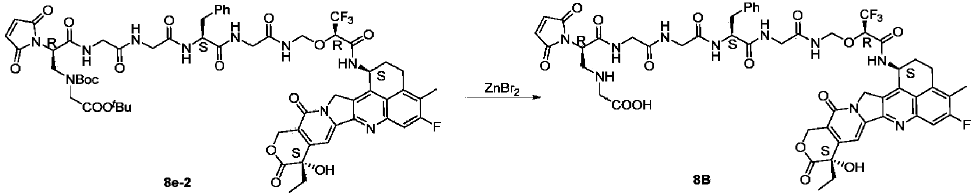

Compound **8e-2** (100 mg, 0.077 mmol), zinc bromide (349 mg, 1.55 mmol) and 5 mL of nitromethane were added to a 25 mL single-necked flask, and reacted at 40°C. for 1 h. After the reaction was finished while monitored by HPLC, the solvent was removed by concentration under reduced pressure to obtain a crude product. The crude product was purified by high performance liquid phase to obtain a product preparation solution, which was freeze-dried to obtain 58mg solid; TOF result: 1133.3653.

### Example 14

### Synthesis of compound 9A:

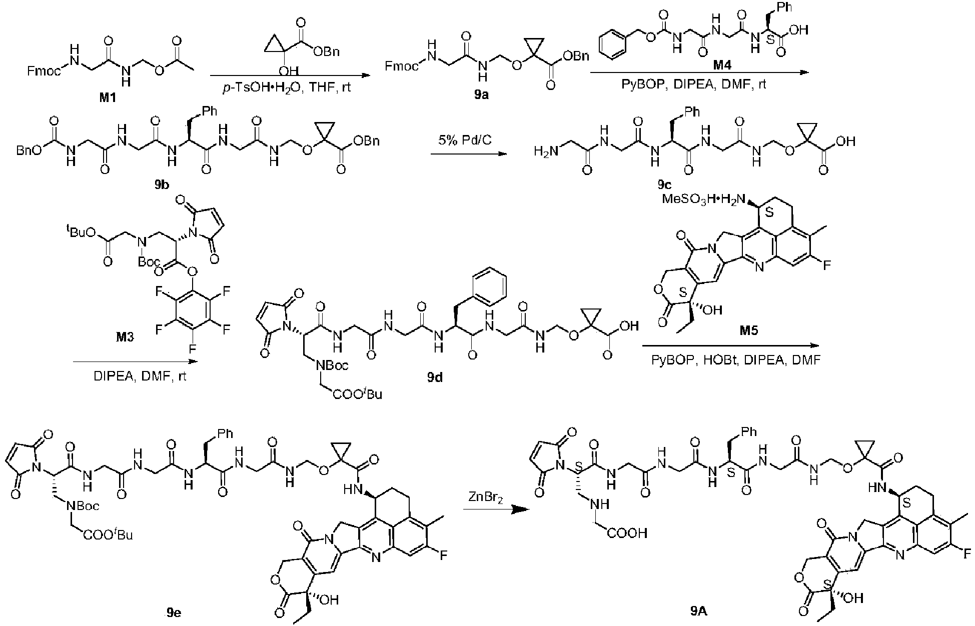

### Step 1: Compound 9a

Add **M1** (6g, 16.3mmol), 100mLTHF, p-toluenesulfonic acid monohydrate (0.31g, 1.63mmol) into a 250mL single-mouth flask, stir and cool to 0°C, add 2-hydroxy-2-cyclopropyl dropwise Benzyl acetate (prepared with reference to the method published in the patent US20050020645A1) (6.3g, 32.6mmol), after dripping, the temperature is naturally raised to room temperature for reaction (reaction is about 2-4h), and TLC is monitored. After the reaction is over, add saturated NaHCO₃ solution, extract with ethyl acetate, wash with saturated sodium chloride solution, dry with anhydrous sodium sulfate, filter, and concentrate. The residue is purified by silica gel column (PE:EA=10:1-5:1 -2:1) Obtain 9a (3.7g) with a yield of 45%; LC-MS: [M+H]⁺ =501.5.

### Step 2: Compound 9b

Add 9a (2g, 4.0mmol), 10mL DMF to a 25mL single-mouth flask, stir at 0°C, add DBU (760mg, 5.0mmol), react for 1 hour, TLC monitoring Fmoc deprotection is complete, set aside;
Add **M4** (1.65g, 4.0mmol), PyBOP (2.59g, 5.0mmol), HOBt (675mg, 5.0mmol) and 10mL DMF to another 25mL single-mouth bottle. Add DIPEA (823uL, 5.04mmol) under ice water bath and continue stirring For 30 minutes, the above reaction solution was added to the reaction flask, and the reaction was raised to room temperature. After the reaction was finished while monitored by HPLC, the reaction solution was purified by the preparation liquid phase to obtain the product preparation solution. The preparation solution was extracted with dichloromethane, washed with saturated sodium chloride solution, dried with anhydrous sodium sulfate, filtered, and the filtrate was concentrated under reduced pressure to obtain 1.5g solid, yield 56%; LC-MS: [M+H]⁺ =674.7.

### Step 3: Compound 9c

Add 9b (900mg, 1.3mmol) in a 25mL single-neck bottle, 10mL DMF, add 900mg 5% Pd/C, hydrogenation reaction for 1.5h, after the reaction is complete, filter to obtain the filtrate, directly used in the next reaction without purification .

### Step 4: Compound 9d

Place the crude product 9c in an ice-water bath, add DIPEA (223 uL, 1.3 mmol), and then add compound **M3** (750 mg, 1.3 mmol), and increase to room temperature to react for 1 h after the addition. The reaction was monitored by HPLC, and the reaction solution was purified by high performance liquid phase to obtain a preparation solution, which was freeze-dried to obtain **9d** (529 mg); LC-MS: [MH]⁻ =828.4.

### Step 5: Compound 9e

Add 9d (500mg, 0.6mmol), **M5** (300mg, 0.6mmol), PyBOP (416mg, 0.8mmol), HOBt (108mg, 0.5mmol) and 15mL DMF to a 50mL single-mouth bottle, add DIPEA (351uL, 2.13) under ice water bath mmol), warm to room temperature and react for 3h. After the reaction was finished while monitored by HPLC, the reaction solution was purified by high performance liquid phase to obtain a preparation solution of compound 9e, which was freeze-dried to obtain **9e** (257 mg); LC-MS: [M+H]⁺ =1247.5.

### Step 6: Compound 9A

Add **9e** (100mg, 0.08mmol), zinc bromide (360mg, 1.6mmol) and 5mL nitromethane into a 25mL single-necked flask, and react at 40°C for 1h. After the reaction was finished while monitored by HPLC, the solvent was removed by concentration under reduced pressure to obtain a crude product. The crude product was purified by high performance liquid phase to obtain a product preparation solution, which was freeze-dried to obtain solid compound **9A** (55 mg); LC-MS: [M+H]⁺ =1091.3.

### Example 15

### Synthesis of compound 9B:

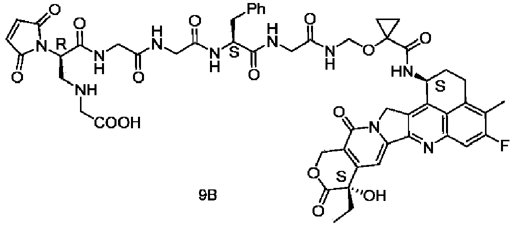

Refer to the synthetic route in Example 14 to obtain compound **9B** (44 mg); LC-MS: [M+H]⁺ =1091.3.

### Example 16

### Synthesis of compound 10A:

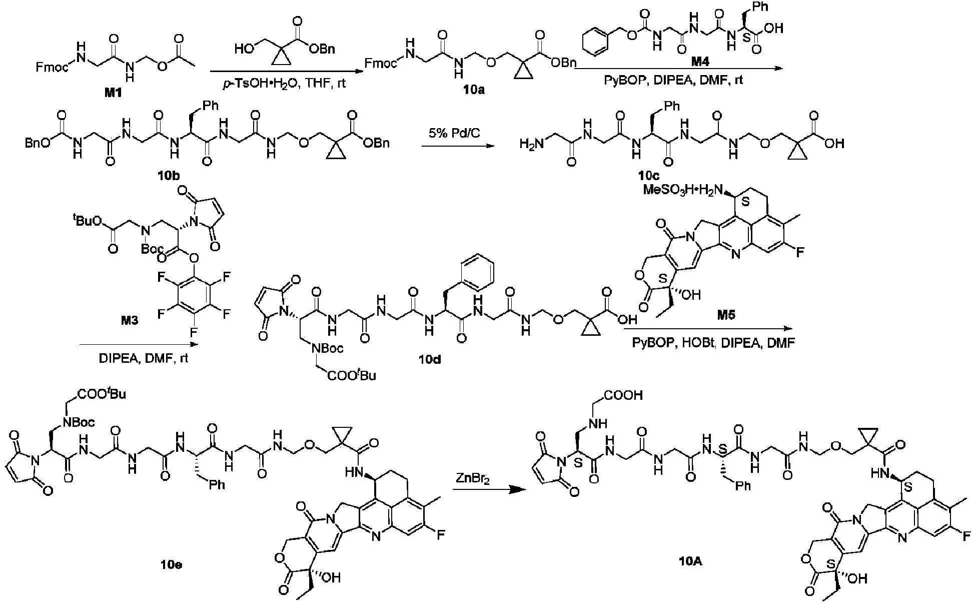

### Step 1: Compound 10a

Add **M1** (6g, 16.3mmol), 100mLTHF, p-toluenesulfonic acid monohydrate (0.31g, 1.63mmol) into a 250mL single-neck flask, stir and cool to 0°C, add 3-hydroxy-2-cyclopropyl dropwise benzyl propionate (prepared with reference to the method published in the patent WO2013187496A1) (6.7g, 32.6mmol), after dripping, the temperature is naturally raised to room temperature for reaction (reaction is about 2-4h), and TLC is monitored. After the reaction is over, add saturated NaHCO₃ solution, extract with ethyl acetate, wash with saturated sodium chloride solution, dry with anhydrous sodium sulfate, filter, and concentrate. The residue is purified by silica gel column (PE:EA=10:1-5:1 -2:1) to obtain 10a (4.9g) with a yield of 58%; LC-MS: [M+H]⁺ =515.4.

### Step 2: Compound 10b

Add **10a** (4g, 7.8mmol), 10mL DMF to a 25mL single-mouth flask, stir at 0°C, add DBU (1.2g, 8.0mmol), react for 1h, TLC monitoring Fmoc deprotection is complete, set aside;
Take another 25mL single-mouth bottle and add **M4** (3.3g, 8.0mmol), PyBOP (5.2g, 10.0mmol), HOBt (1.35g, 10.0mmol) and 10mL DMF, add DIPEA (1.65mL, 10.1mmol) under ice water bath, Stirring is continued for 50 minutes, the above reaction solution is added to the reaction flask, and the reaction is raised to room temperature. After the reaction was finished while monitored by HPLC, the reaction solution was purified by the preparation liquid phase to obtain the product preparation solution. The preparation solution was extracted with dichloromethane, washed with saturated sodium chloride solution, dried with anhydrous sodium sulfate, filtered, and the filtrate was concentrated under reduced pressure to obtain 2.3g solid, yield 42%; LC-MS: [M+H]⁺ =688.8.

### Step 3: Compound 10c

Add **10b** (1.0g, 1.45mmol) in a 25mL single-necked flask, after 15mL DMF is dissolved, add 1.0g 5% Pd/C, hydrogenation reaction for 1.5h, after the reaction is complete, filter to obtain the filtrate, and use it directly without purification. One step response.

### Step 4: Compound 10d

Place the crude product **10c** in an ice-water bath, add DIPEA (258uL, 1.5mmol), and then add compound **M3** (837mg, 1.45mmol), and increase to room temperature to react for 1h after the addition. After the reaction was finished while monitored by HPLC, the reaction solution was purified by high performance liquid phase to obtain a preparation solution, which was freeze-dried to obtain 10d (499 mg); LC-MS: [MH]⁻ =842.4.

### Step 5: Compound 10e

Add **10d** (400mg, 0.48mmol), **M5** (240mg, 0.48mmol), PyBOP (250mg, 0.48mmol), HOBt (104mg, 0.48mmol) and 15mL DMF to a 50mL single-mouth bottle. Add DIPEA (330uL, 2.0 mmol), warm to room temperature and react for 3h. After the reaction was finished while monitored by HPLC, the reaction solution was purified by high performance liquid phase to obtain a preparation solution of compound 10e, which was freeze-dried to obtain 10e (188 mg); LC-MS: [M+H]⁺ =1261.5.

### Step 6: Compound 10A

Add **10e** (100mg, 0.08mmol), zinc bromide (360mg, 1.6mmol) and 5mL nitromethane to a 25mL single-necked flask, and react at 40°C for 1h. After the reaction was finished while monitored by HPLC, the solvent was removed by concentration under reduced pressure to obtain a crude product. The crude product was purified by high performance liquid phase to obtain a product preparation solution, which was freeze-dried to obtain solid compound **10A** (61 mg); LC-MS: [M+H]⁺ =1105.4.

### Example 17

### Synthesis of compound 10B:

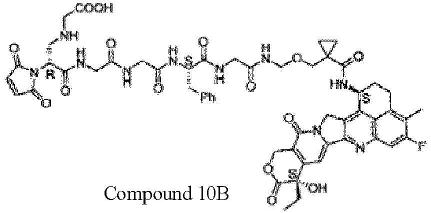

Refer to the synthetic route in Example 16 to obtain compound **10B** (75 mg); LC-MS: [M+H]⁺ =1105.4.

### Example 18

### Synthesis of compound 11A:

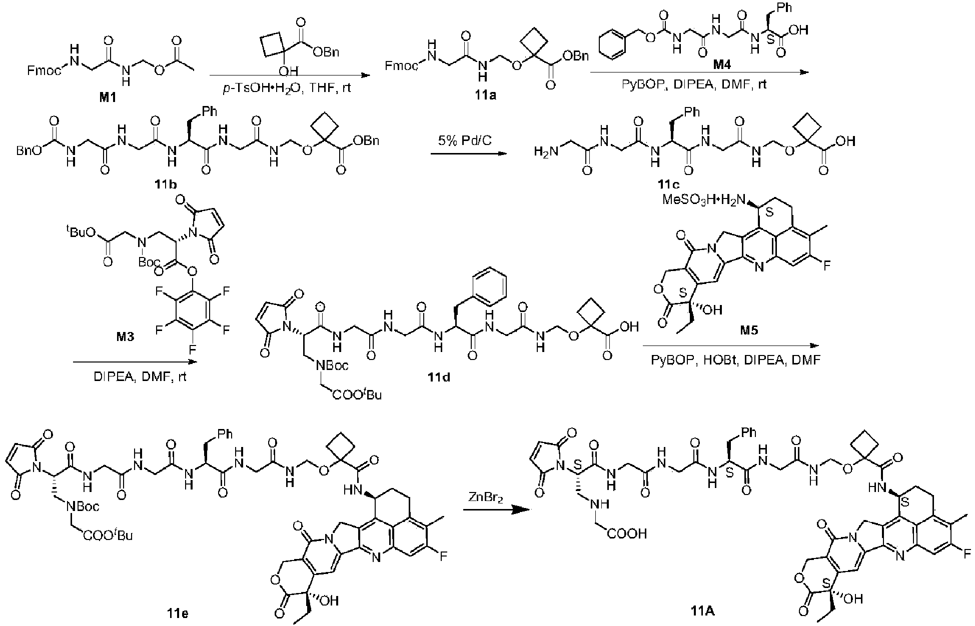

### Step 1: Compound 11a

Add **M1** (6g, 16.3mmol), 100mLTHF, p-toluenesulfonic acid monohydrate (0.31g, 1.63mmol) into a 250mL single-neck flask, stir and cool to 0°C, add dropwise 2-hydroxy-2-cyclobutyl Benzyl acetate (synthesized by referring to the method published in Journal of Medicinal Chemistry, 2013, 56(13), 5541-5552.) (6.7g, 32.6mmol), after dripping, the reaction is naturally heated to room temperature (reaction is about 2-4h), TLC monitoring. After the reaction is over, add saturated NaHCO₃ solution, extract with ethyl acetate, wash with saturated sodium chloride solution, dry with anhydrous sodium sulfate, filter, and concentrate. The residue is purified by silica gel column (PE:EA=10:1-5:1 -2:1) 11a (5.1g) was obtained with a yield of 62%; LC-MS: [M+H]⁺ =515.7.

### Step 2: Compound 11b

Add **11a** (4g, 7.8mmol), 10mL DMF to a 25mL single-mouth flask, stir at 0°C, add DBU (1.2g, 8.0mmol), react for 1 hour, TLC monitoring Fmoc deprotection is complete, set aside;
Add **M4** (3.3g, 8.0mmol), PyBOP (5.2g, 10.0mmol), HOBt (1.35g, 10.0mmol) and 10mL DMF to another 25mL single-mouth bottle. Add DIPEA (1.63mL, 10.0mmol) under ice water bath, Stirring is continued for 40 minutes, the above reaction solution is added to the reaction flask, and the reaction is raised to room temperature. After the reaction was finished while monitored by HPLC, the reaction solution was purified by the preparation liquid phase to obtain the product preparation solution. The preparation solution was extracted with dichloromethane, washed with saturated sodium chloride solution, dried with anhydrous sodium sulfate, filtered, and the filtrate was concentrated under reduced pressure to obtain 2.3g solid, yield 42%; LC-MS: [M+H]⁺ =688.3.

### Step 3: Compound 11c

Add **11b** (2.0g, 2.9mmol) in a 25mL single-necked flask, after 25mL DMF is dissolved, add 2.0g 5% Pd/C, hydrogenation reaction 3h, after the reaction is complete, filter to obtain the filtrate, directly used in the next step without purification reaction.

### Step 4: Compound 11d

Place the crude product 11c in an ice-water bath, add DIPEA (516uL, 3.0mmol), and then add compound **M3** (1.7g, 2.9mmol), and increase to room temperature to react for 2h after the addition. The reaction was monitored by HPLC, and the reaction solution was purified by high performance liquid phase to obtain a preparation solution, which was freeze-dried to obtain **11d** (934mg); LC-MS: [MH]⁻ =842.4.

### Step 5: Compound 11e

Add 1**1d** (800mg, 0.96mmol), **M5** (480mg, 0.96mmol), PyBOP (500mg, 0.96mmol), HOBt (208mg, 0.96mmol) and 30mL DMF into a 50mL single-mouth bottle, add DIPEA (660uL, 4.0 mmol), warm to room temperature and react for 4h. After the reaction was finished while monitored by HPLC, the reaction solution was purified by high performance liquid phase to obtain a preparation solution of compound **11e**, which was freeze-dried to obtain **11e** ( 401 mg); LC-MS: [M+H]⁺ =1261.4.

### Step 6: Compound 11A

Add **11e** (150mg, 0.12mmol), zinc bromide (532mg, 2.4mmol) and 10mL nitromethane into a 25mL single-necked flask, and react at 40°C for 1h. After the reaction was finished while monitored by HPLC, the solvent was removed by concentration under reduced pressure to obtain a crude product. The crude product was purified by high performance liquid phase to obtain a product preparation solution, which was freeze-dried to obtain solid compound **11A** (86 mg); LC-MS: [M+H]⁺ =1105.4.

### Example 19

### Synthesis of compound 11B

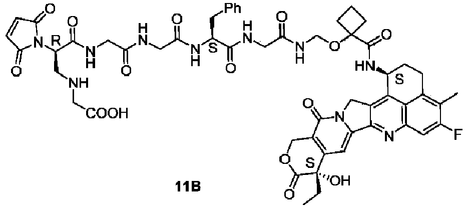

According to the synthetic route of Example 18, compound **11B** (50 mg) was obtained. LC-MS: [M+H]⁺ 1105.4.

### Example 20

### Synthesis of compound 12A:

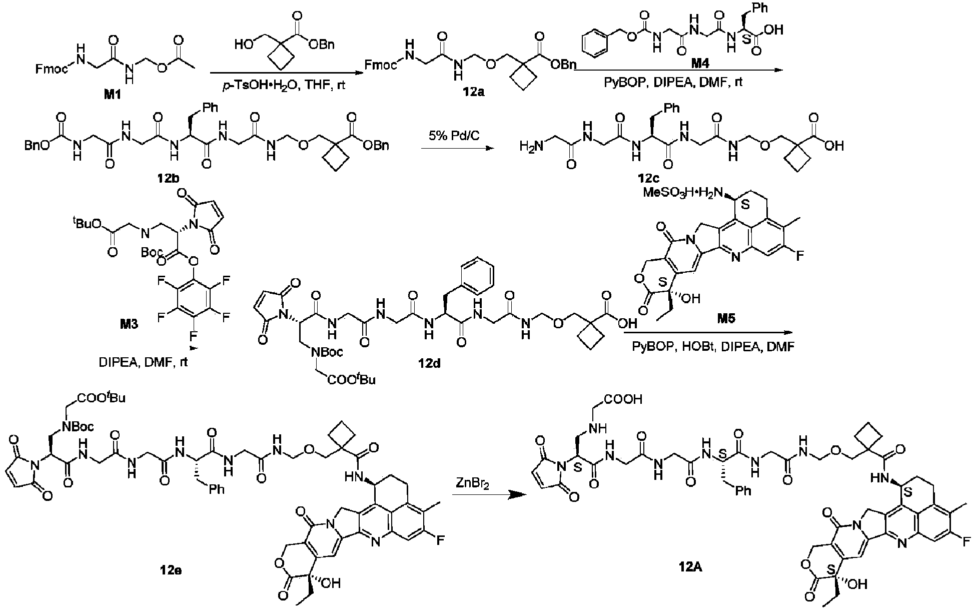

### Step 1: Compound 12a

Add **M1** (6g, 16.3mmol), 100mLTHF, p-toluenesulfonic acid monohydrate (0.31g, 1.63mmol) into a 250mL single-mouth flask, stir and cool to 0°C, add 3-hydroxy-2-cyclobutyl dropwise Benzyl propionate (prepared with reference to the method published in patent WO2009011285A1) (7.2g, 32.6mmol), after dripping, the temperature is naturally raised to room temperature for reaction (reaction is about 2-4h), monitored by TLC. After the reaction is over, add saturated NaHCO₃ solution, extract with ethyl acetate, wash with saturated sodium chloride solution, dry with anhydrous sodium sulfate, filter, and concentrate. The residue is purified by silica gel column (PE:EA=10:1-5:1 -2:1) 12a (4.5g) was obtained, the yield was 52%; LC-MS: [M+H]⁺ =529.4.

### Step 2: Compound 12b

In a 25mL single-mouth flask, add 12a (4g, 7.6mmol), 10mL DMF, stir at 0°C, add DBU (1.2g, 8.0mmol), react for 1 hour, TLC monitoring Fmoc deprotection is complete, set aside;
Add **M4** (3.2g, 7.6mmol), PyBOP (4.7g, 9.0mmol), HOBt (1.22g, 9.0mmol) and 10mL DMF to another 25mL single-mouth bottle, add DIPEA (1.49mL, 0.9mmol) under ice water bath, Stirring was continued for 30 minutes, the above reaction solution was added to the reaction flask, and the reaction was raised to room temperature. After the reaction was finished while monitored by HPLC, the reaction solution was purified by the preparation liquid phase to obtain the product preparation solution. The preparation solution was extracted with dichloromethane, washed with saturated sodium chloride solution, dried with anhydrous sodium sulfate, filtered, and the filtrate was concentrated under reduced pressure to obtain 2.0g solid, yield 37%; LC-MS: [M+H]⁺ =702.8.

### Step 3: Compound 12c

Add **12b** (1.0g, 1.43mmol) in a 25mL single-necked flask, after 15mL DMF is dissolved, add 1.0g 5% Pd/C, hydrogenation reaction for 1.5h, after the reaction is complete, filter to obtain the filtrate, and use it directly without purification. One step response.

### Step 4: Compound 12d

Place the crude product **12c** in an ice-water bath, add DIPEA (258uL, 1.5mmol), and then add compound **M3** (825mg, 1.43mmol), after the addition, increase to room temperature and react for 1h. The reaction was monitored by HPLC, and the reaction solution was purified by high performance liquid phase to obtain a preparation solution. The preparation solution was lyophilized to obtain **12d** (522 mg); LC-MS: [MH]⁻ =856.4.

### Step 5: Compound 12e

Add 12d (400mg, 0.47mmol), **M5** (240mg, 0.47mmol), PyBOP (250mg, 0.47mmol), HOBt (101mg, 0.47mmol) and 15mL DMF to a 50mL single-mouth bottle, add DIPEA (330uL, 2.0 mmol), warm to room temperature and react for 3h. After the reaction was finished while monitored by HPLC, the reaction solution was purified by high performance liquid phase to obtain a preparation solution of compound **12e,** which was freeze-dried to obtain **12e** (198 mg); LC-MS: [M+H]⁺ =1275.4.

### Step 6: Compound 12A

Add **12e** (100mg, 0.08mmol), zinc bromide (360mg, 1.6mmol) and 5mL nitromethane to a 25mL single-necked flask, and react at 40°C for 1h. After the reaction was finished while monitored by HPLC, the solvent was removed by concentration under reduced pressure to obtain a crude product. The crude product was purified by high performance liquid phase to obtain a product preparation solution, which was freeze-dried to obtain solid compound **12A** (55 mg); LC-MS: [M+H]⁺ =1119.4.

### Example 21

### Synthesis of compound 12B:

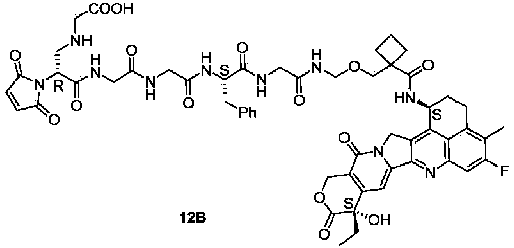

Refer to the synthetic route of Example 20 to obtain compound **12B** (50 mg); LC-MS: [M+H]⁺ =1119.4.

### Example 22

### Synthesis of compound 13A:

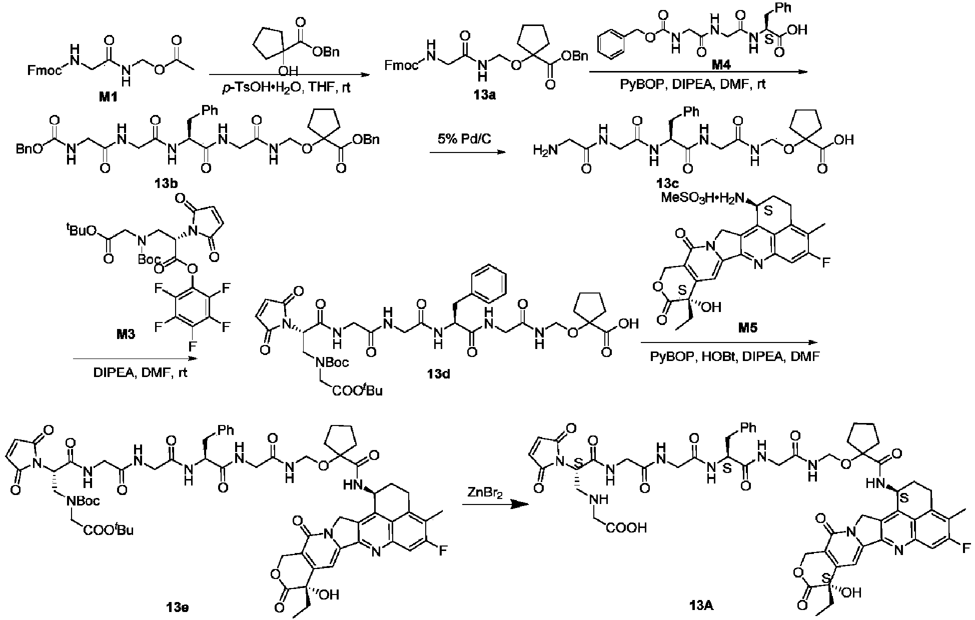

### Step 1: Compound 13a

Add **M1** (6g, 16.3mmol), 100mLTHF, p-toluenesulfonic acid monohydrate (0.31g, 1.63mmol) into a 250mL single-mouth flask, stir and cool to 0°C, add 2-hydroxy-2-cyclopentyl dropwise Benzyl acetate (synthesized by referring to the method published in Journal of Medicinal Chemistry, 2013, 56(13), 5541-5552.) (7.2g, 32.6mmol), after dripping, the reaction is naturally heated to room temperature (reaction is about 2-4h), TLC monitoring. After the reaction is over, add saturated NaHCO₃ solution, extract with ethyl acetate, wash with saturated sodium chloride solution, dry with anhydrous sodium sulfate, filter, and concentrate. The residue is purified by silica gel column (PE:EA=10:1-5:1 -2:1) **13a** (4.6g) was obtained, the yield was 53%; LC-MS: [M+H]⁺ =529.5.

### Step 2: Compound 13b

In a 25mL single-mouth flask, add **13a** (4g, 7.6mmol), 10mL DMF, stir at 0°C, add DBU (1.17g, 7.8mmol), react for 1 hour, TLC monitoring Fmoc deprotection is complete, set aside;
Add **M4** (3.14g, 7.6mmol), PyBOP (4.42g, 8.5mmol), HOBt (1.15g, 8.5mmol) and 10mL DMF to another 25mL single-mouth bottle, add DIPEA (1.39mL, 0.85mmol) under ice water bath, Stirring was continued for 30 minutes, the above reaction solution was added to the reaction flask, and the reaction was raised to room temperature. After the reaction was finished while monitored by HPLC, the reaction solution was purified by the preparation liquid phase to obtain the product preparation solution. The preparation solution was extracted with dichloromethane, washed with saturated sodium chloride solution, dried with anhydrous sodium sulfate, filtered, and the filtrate was concentrated under reduced pressure to obtain 2.1g solid, yield 39%; LC-MS: [M+H]⁺ =702.8.

### Step 3: Compound 13c

Add **13b** (1.5g, 1.87mmol) in a 25mL single-necked flask, after 25mL DMF is dissolved, add 1.5g 5% Pd/C, hydrogenation reaction for 3h, after the reaction is complete, filter to obtain the filtrate, directly used in the next step without purification reaction.

### Step 4: Compound 13d

Place the crude product **13c** in an ice-water bath, add DIPEA (333uL, 1.93mmol), and then add compound **M3** (1.1g, 1.87mmol), and increase to room temperature to react for 1h after the addition. The reaction was monitored by HPLC, and the reaction solution was purified by high performance liquid phase to obtain a preparation solution. The preparation solution was lyophilized to obtain **13d** (519 mg); LC-MS: [MH]⁻ =856.6.

### Step 5: Compound 13e

Add **13d** (400mg, 0.47mmol), **M5** (240mg, 0.48mmol), PyBOP (250mg, 0.48mmol), HOBt (103mg, 48mmol) and 15mL DMF into a 50mL single-mouth bottle, add DIPEA (330uL, 2.0mmol) under ice water bath ), warm to room temperature and react for 4h. After the reaction was finished while monitored by HPLC, the reaction solution was purified by high performance liquid phase to obtain a preparation solution of compound **13e,** which was freeze-dried to obtain **13e** (187 mg); LC-MS: [M+H]⁺ =1275.5.

### Step 6: Compound 13A

Add **13e** (100mg, 0.08mmol), zinc bromide (355mg, 0.16mmol) and 5mL nitromethane into a 25mL single-neck flask, and react at 40°C for 1h. After the reaction was finished while monitored by HPLC, the solvent was removed by concentration under reduced pressure to obtain a crude product. The crude product was purified by high performance liquid phase to obtain a product preparation solution, which was freeze-dried to obtain solid compound **13A** (60 mg); LC-MS: [M+H]⁺ =1119.6.

### Example 23

### Synthesis of compound 13B:

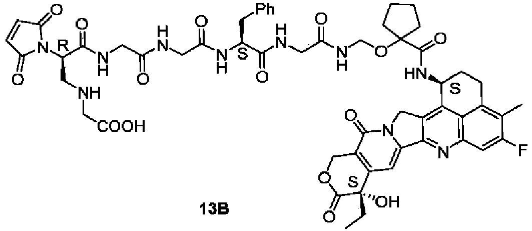

Refer to the synthetic route in Example 22 to obtain compound **13B** (51 mg); LC-MS: [M+H]⁺ =1119.6.

### Example 24

### Synthesis of compound 14A:

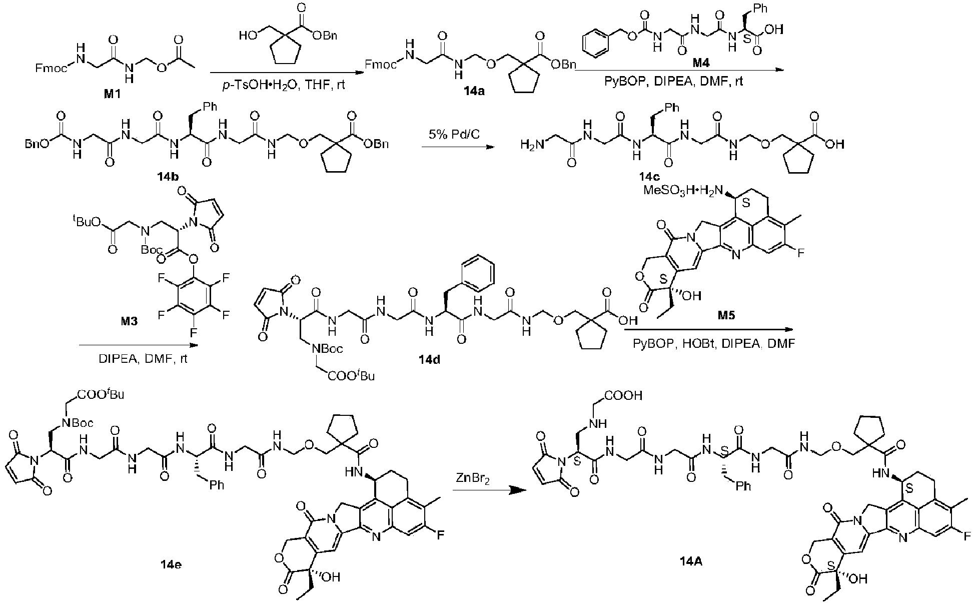

### Step 1: Compound 14a

Add **M1** (6g, 16.3mmol), 100mLTHF, p-toluenesulfonic acid monohydrate (0.31g, 1.63mmol) into a 250mL single-mouth flask, stir and cool to 0°C, add 3-hydroxy-2-cyclopentyl dropwise Benzyl propionate (synthesized with reference to the method published in the patent WO2009011285A1) (7.6g, 32.6mmol), after dripping, the reaction was naturally raised to room temperature (the reaction was about 2-4h), and TLC monitored. After the reaction is over, add saturated NaHCO₃ solution, extract with ethyl acetate, wash with saturated sodium chloride solution, dry with anhydrous sodium sulfate, filter, and concentrate. The residue is purified by silica gel column (PE:EA=10:1-5:1 -2:1) 14a (4.4g) was obtained with a yield of 49%; LC-MS: [M+H]⁺ =543.6.

### Step 2: Compound 14b

Add **14a** (4g, 7.4mmol), 10mL DMF to a 25mL single-mouth flask, stir at 0°C, add DBU (1.2g, 8.0mmol), react for 1 hour, TLC monitoring Fmoc deprotection is complete, set aside;
Add **M4** (3.1g, 7.4mmol), PyBOP (4.6g, 8.8mmol), HOBt (1.19g, 8.8mmol) and 10mL DMF to another 25mL single-mouth bottle. Add DIPEA (1.49mL, 9.0mmol) under ice water bath, Stirring was continued for 30 minutes, the above reaction solution was added to the reaction flask, and the reaction was raised to room temperature. After the reaction was finished while monitored by HPLC, the reaction solution was purified by the preparation liquid phase to obtain the product preparation solution. The preparation solution was extracted with dichloromethane, washed with saturated sodium chloride solution, dried with anhydrous sodium sulfate, filtered, and the filtrate was concentrated under reduced pressure to obtain 2.6g solid, yield 49%; LC-MS: [M+H]⁺ =716.4.

### Step 3: Compound 14c

Add **14b** (1.0g, 1.4mmol) in a 25mL single-necked bottle, 15mL DMF dissolved, add 1.0g 5% Pd/C, hydrogenation reaction for 1.5h, after the reaction is complete, filter to obtain the filtrate, directly used without purification One step response.

### Step 4: Compound 14d

Place the crude product **14c** in an ice-water bath, add DIPEA (248uL, 1.5mmol), and then add compound M3 (808mg, 1.4mmol), after the addition, the temperature is raised to room temperature and reacted for 1h. After the reaction was finished while monitored by HPLC, the reaction solution was purified by high performance liquid phase to obtain a preparation solution, which was freeze-dried to obtain **14d** (500 mg); LC-MS: [MH]⁻ =870.5.

### Step 5: Compound 14e

Add 14d (400mg, 0.46mmol), **M5** (235mg, 0.46mmol), PyBOP (245mg, 0.46mmol), HOBt (99mg, 0.46mmol) and 15mL DMF into a 50mL single-mouth bottle, add DIPEA (331uL, 2.0 mmol), warm to room temperature and react for 3h. After the reaction was finished while monitored by HPLC, the reaction solution was purified by high performance liquid phase to obtain a preparation solution of compound **14e,** which was freeze-dried to obtain **14e** (146 mg); LC-MS: [M+H]⁺ =1289.5.

### Step 6: Compound 14A

Add **14e** (100mg, 0.08mmol), zinc bromide (360mg, 1.6mmol) and 5mL nitromethane into a 25mL single-necked flask, and react at 40°C for 1h. After the reaction was finished while monitored by HPLC, the solvent was removed by concentration under reduced pressure to obtain a crude product. The crude product was purified by high performance liquid phase to obtain a product preparation solution, which was freeze-dried to obtain solid compound **14A** (52 mg); LC-MS: [M+H]⁺ =1133.4.

### Example 25

### Synthesis of compound 14B:

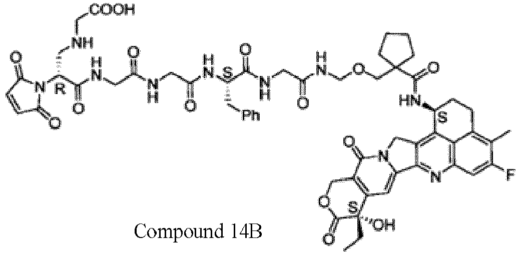

Refer to the synthetic route in Example 24 to obtain compound **14B** (48 mg); LC-MS: [M+H]⁺ =1133.4.

### Example 26

### Synthesis of compounds 15A and 15B:

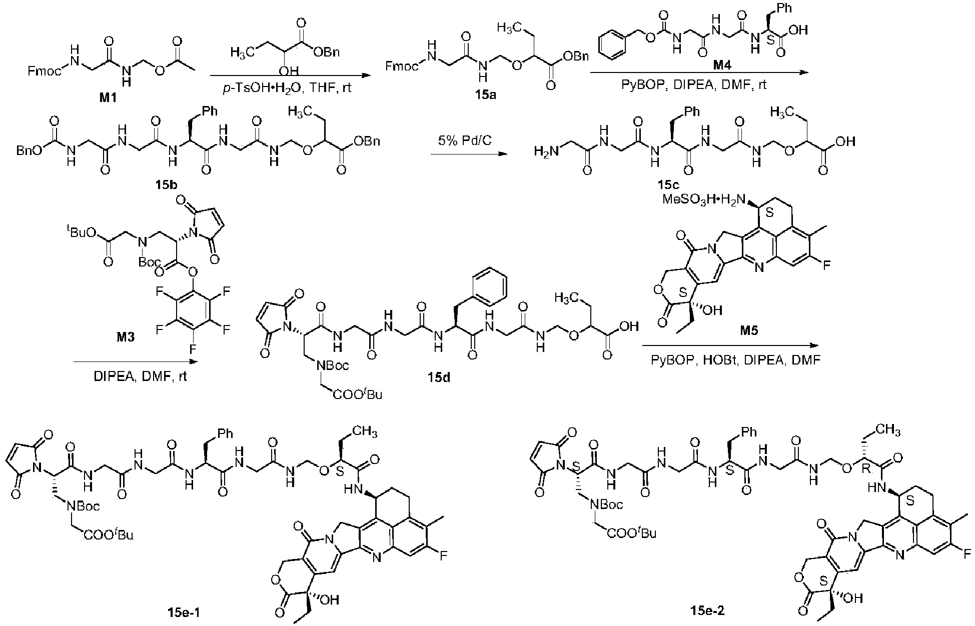

### Step 1: Compound 15a

Add **M1** (10g, 27.1mmol), 2-hydroxy-butyric acid benzyl ester (refer to the document Chemical Communications, 2019, 55(53), 7699-7702. Prepared by the published method) (10.5g, 54.3mmol) in a 250mL single-mouth bottle. ), zinc acetate (9.96g, 54.3mmol) and 100mL of toluene, heated to 100°C and reacted for 4h. After the reaction was completed, the temperature was lowered to room temperature, the insoluble matter was removed by filtration, and the filtrate was concentrated to obtain a crude product. The crude product was purified by silica gel column chromatography (PE:EA=10:1-5:1-2:1) to obtain 5.67 g of the target product with a yield of 42%; LC-MS: [M+H]⁺ =503.5.

### Step 2: Compound 15b

Add **15a** (5g, 9.95mmol) and 15mL DMF to a 50mL single-necked flask. After dissolving it, add DBU (1.68g, 11mmol) in an ice-water bath, and react for 1 hour, which is recorded as reaction solution①;
Take another 50mL single-mouth bottle, add **M4** (4.1g, 10.0mmol), PyBOP (5.75g, 11mmol), HOBt (1.49g, 11mmol) and 10mL DMF. After dissolving, add DIPEA (1.82mL, 11mmol) under ice water bath. ), continue the reaction for 40 minutes, add the reaction solution ①, and warm to room temperature to react for 2 hours. The reaction progress was monitored by HPLC. After the reaction was completed, the reaction solution was purified by high performance liquid phase to obtain a preparation solution. The preparation solution was extracted with dichloromethane, washed with saturated sodium chloride solution, dried with anhydrous sodium sulfate, filtered, and concentrated to obtain 4.6g of solid, with a yield of 68%; LC-MS: [M+H]⁺ =676.7.

### Step 3: Compound 15d

Add **15b** (2.0g, 2.96mmol) in a 25mL single-necked flask, after 15mL DMF is dissolved, add 2.0g 5% Pd/C, hydrogenation reaction for 2h, after the reaction is complete, filter, place the filtrate in an ice water bath, add DIPEA ( 496uL, 3.0mmol), then M3 (1.7g, 2.96mmol) was added, and after the addition, the temperature was raised to room temperature and reacted for 1h. After the reaction was finished while monitored by HPLC, the reaction solution was purified by high performance liquid phase to obtain a preparation solution. The preparation solution was lyophilized to obtain 1120.0 mg of the product with a yield of 45%; LC-MS: [MH]⁻ = 830.3.

### Step 4: Compound 15e

Add **15d** (500mg, 0.60mmol), **M5** (321mg, 0.60mmol), PyBOP (469mg, 0.90mmol), HOBt (121mg, 0.90mmol) and 15mL DMF to a 50mL single-mouth bottle. Add DIPEA (446uL, 2.7 mmol), warm to room temperature and react for 2h. After the reaction was finished while monitored by HPLC, the reaction solution was purified by high performance liquid phase to obtain preparations of compound **15e-1** and compound **15e-2.** The preparations were freeze-dried to obtain 138 mg of compound **15e-1,** LC-MS: [M+H]⁺ =1249.5; 140mg of compound **15e-2,** LC-MS: [M+H]⁺ =1249.5.

### Step 5: Compound 15A

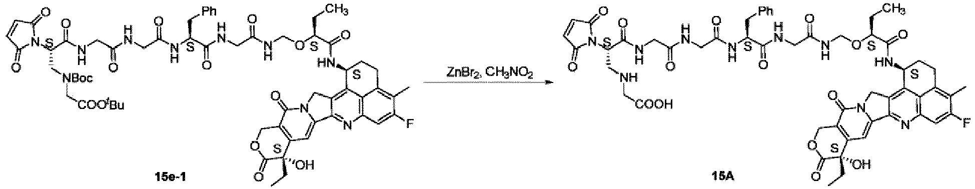

Add **15e-1** (100mg, 0.08mmol), zinc bromide (360mg, 1.6mmol) and 5mL nitromethane into a 25mL single-necked flask, and react at 40°C for 1h. After the reaction was finished while monitored by HPLC, the solvent was removed by concentration under reduced pressure to obtain a crude product. The crude product was purified by high performance liquid phase to obtain a product preparation solution, which was freeze-dried to obtain a solid (59mg); LC-MS: [M+H]⁺ =1093.4.

### Step 6: Compound 15B

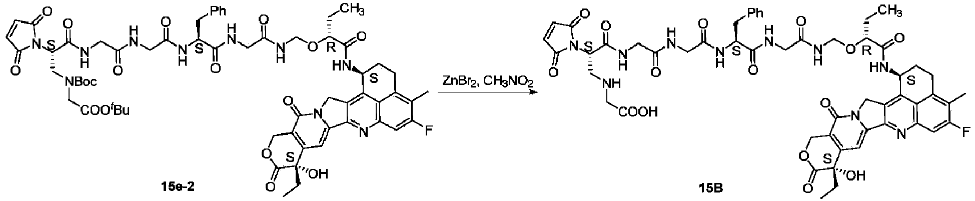

Add **15e-2** (100mg, 0.08mmol), zinc bromide (360mg, 1.6mmol) and 5mL nitromethane to a 25mL single-necked flask, and react at 40°C for 1h. After the reaction was finished while monitored by HPLC, the solvent was removed by concentration under reduced pressure to obtain a crude product. The crude product was purified by high performance liquid phase to obtain a product preparation solution, which was freeze-dried to obtain a solid (60mg); LC-MS: [M+H]⁺ =1093.4.

### Example 27

### Synthesis of compound 16A and 16B:

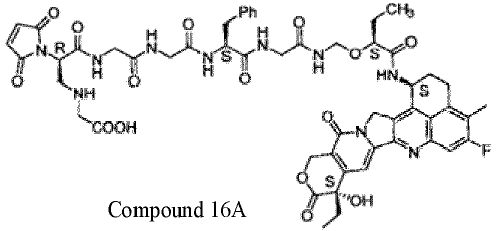

Refer to the synthetic route of Example 26 to obtain compound **16A** (55 mg); LC-MS: [M+H]⁺ =1093.4.

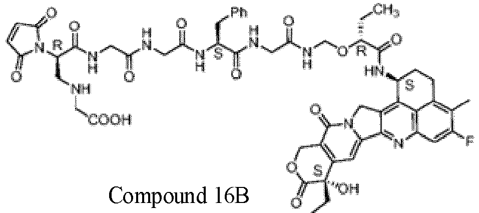

Refer to the synthetic route in Example 26 to obtain compound **16B** (54 mg); LC-MS: [M+H]⁺ =1093.4.

### Example 28

### Synthesis of compounds 17A and 17B:

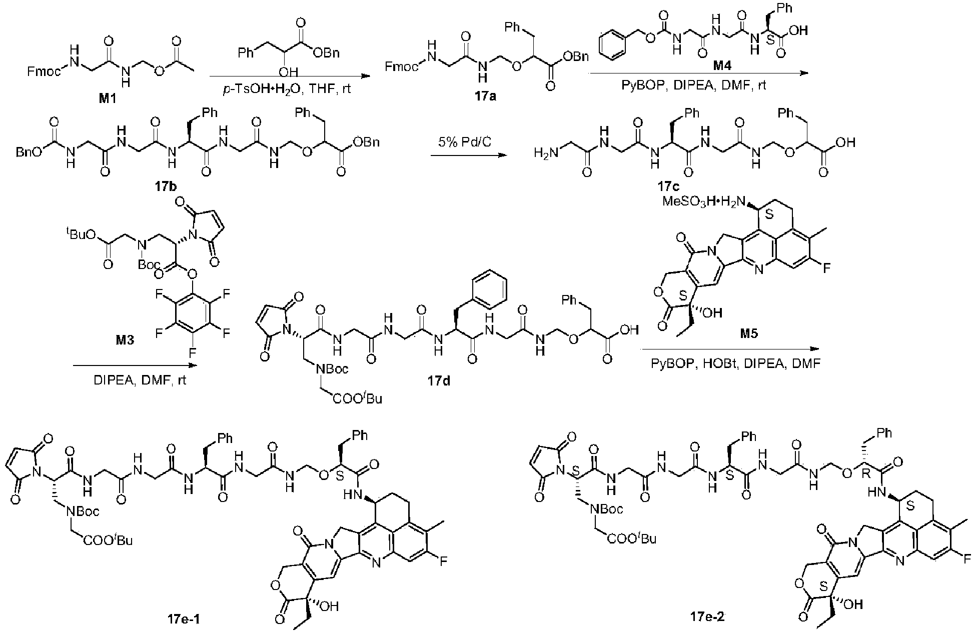

### Step 1: Compound 17a

Add **M1** (10g, 27.1mmol), 2-hydroxy-phenylpropionic acid benzyl ester (referenced Nature Communications, 2020.11(1), 56. published method) (14.7g, 54.3mmol), acetic acid Zinc (9.96g, 54.3mmol) and 100mL toluene were heated to 100°C to react for 4h. After the reaction was completed, the temperature was lowered to room temperature, the insoluble matter was removed by filtration, and the filtrate was concentrated to obtain a crude product. The crude product was purified by silica gel column chromatography (PE:EA=10:1-5:1-2:1) to obtain 6.13g of the target product, with a yield of 40%; LC-MS: [M+H]+ =565.6.

### Step 2: Compound 17b

Add **17a** (5g, 8.86mmol) and 15mL DMF to a 50mL single-necked flask. After dissolving, add DBU (1.53g, 10mmol) in an ice-water bath and react for 1 hour, which is recorded as reaction solution ①;
Take another 50mL single-mouth bottle, add **M4** (3.6g, 8.86mmol), PyBOP (5.23g, 10mmol), HOBt (1.36g, 10mmol) and 10mL DMF. After dissolving, add DIPEA (1.65mL, 10mmol) under ice water bath. ), continue the reaction for 30 minutes, add the reaction solution ①, and warm to room temperature for 2 hours. The reaction progress was monitored by HPLC. After the reaction was completed, the reaction solution was purified by high performance liquid phase to obtain a preparation solution. The preparation liquid was extracted with dichloromethane, washed with saturated sodium chloride solution, dried with anhydrous sodium sulfate, filtered, and concentrated to obtain a solid (5.0g) with a yield of 77%; LC-MS: [M+H]⁺ =738.3.

### Step 3: Compound 17d

Add **17b** (3.0g, 4.07mmol) in a 25mL single-necked flask, after 15mL DMF is dissolved, add 3.0g 5% Pd/C, hydrogenation reaction for 2h, the reaction is complete, filter, place the filtrate in an ice water bath, add DIPEA ( 744uL, 4.5mmol), then **M3** (2.34g, 4.07mmol) was added, and after the addition, the temperature was raised to room temperature and reacted for 1h. After the reaction was finished while monitored by HPLC, the reaction solution was purified by high performance liquid phase to obtain a preparation solution. The preparation solution was lyophilized to obtain 1.2 g of the product with a yield of 33%; LC-MS: [MH]⁻ = 892.4.

### Step 4: Compound 17e

Add **17d** (500mg, 0.56mmol), **M5** (300mg, 0.56mmol), PyBOP (438mg, 0.84mmol), HOBt (113mg, 0.84mmol) and 15mL DMF into a 50mL single-mouth bottle, add DIPEA (330uL, 2.0 mmol), warm to room temperature and react for 2h. After the reaction was finished while monitored by HPLC, the reaction solution was purified by high performance liquid phase to obtain preparations of compound **17e-1** and compound **17e-2.** The preparations were freeze-dried to obtain 156 mg of compound **17e-1,** LC-MS: [M+H]⁺ =1311.4; 150 mg of compound **17e-2,** LC-MS: [M+H]⁺ =1311.7.

### Step 5: Compound 17A

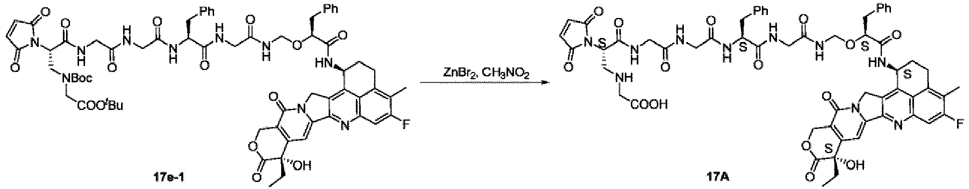

Add **17e-1** (100mg, 0.08mmol), zinc bromide (360mg, 1.6mmol) and 5mL nitromethane into a 25mL single-necked flask, and react at 40°C for 1h. After the reaction was finished while monitored by HPLC, the solvent was removed by concentration under reduced pressure to obtain a crude product. The crude product was purified by high performance liquid phase to obtain a product preparation solution, which was freeze-dried to obtain a solid (43mg); LC-MS: [M+H]⁺ =1155.4.

### Step 6: Compound 17B

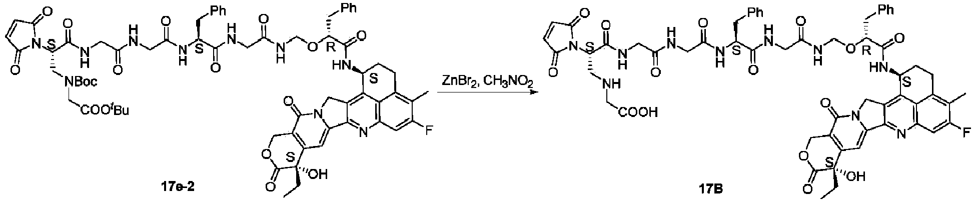

Add **17e-2** (100mg, 0.08mmol), zinc bromide (360mg, 1.6mmol) and 5mL nitromethane into a 25mL single-necked flask, and react at 40°C for 1h. After the reaction was finished while monitored by HPLC, the solvent was removed by concentration under reduced pressure to obtain a crude product. The crude product was purified by high performance liquid phase to obtain a product preparation solution, which was freeze-dried to obtain a solid (40 mg); LC-MS: [M+H]⁺ =1155.4.

### Example 29

### Synthesis of compound 18A and 18B:

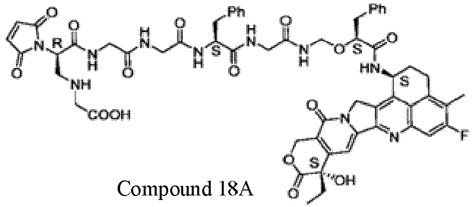

Refer to the synthetic route in Example 28 to obtain compound **18A** (54 mg); LC-MS: [M+H]⁺ =1155.4.

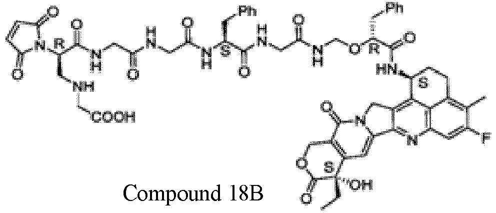

Refer to the synthetic route in Example 28 to obtain compound **18B** (55 mg); LC-MS: [M+H]⁺ =1155.4.

### Example 30

### Synthesis of compounds 19A and 19B:

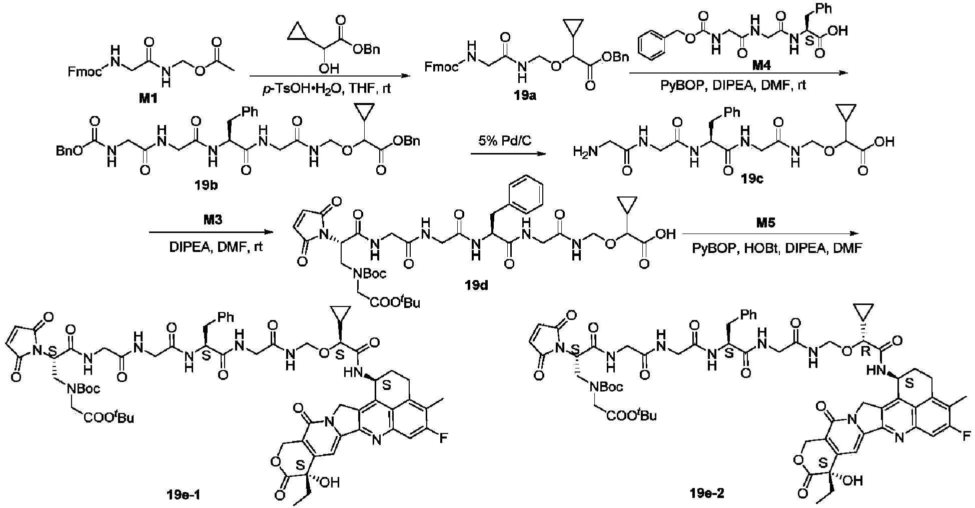

### Step 1: Compound 19a

Add **M1** (10g, 27.1mmol), 2-cyclopropyl-2-hydroxyacetate benzyl ester (prepared with reference to the method published in patent WO2020244657A1) (11.2g, 54.3mmol), zinc acetate (9.96g, 54.3 mmol) and 100 mL of toluene, heated to 100° C. and reacted for 4 hours. After the reaction was completed, the temperature was lowered to room temperature, the insoluble matter was removed by filtration, and the filtrate was concentrated to obtain a crude product. The crude product was purified by silica gel column chromatography (PE:EA=10:1-5:1-2:1) to obtain 4.97 g of the target product, with a yield of 36%; LC-MS: [M+H]⁺ =515.2.

### Step 2: Compound 19b

Add **19a** (4g, 7.8mmol) and 10mL DMF to a 50mL single-necked flask. After dissolving, add DBU (1.42g, 9.3mmol) in an ice-water bath, and react for 1 hour, which is recorded as reaction solution ①;
Take another 50mL single-mouth bottle, add **M4** (3.2g, 7.8mmol), PyBOP (4.5g, 8.6mmol), HOBt (1.16g, 8.6mmol) and 10mL DMF. After dissolving, add DIPEA (1.65mL, 10mmol), continue the reaction for 30 minutes, add the reaction solution ①, warm to room temperature and react for 2 hours. The reaction progress was monitored by HPLC. After the reaction was completed, the reaction solution was purified by high performance liquid phase to obtain a preparation solution. The preparation liquid was extracted with dichloromethane, washed with saturated sodium chloride solution, dried with anhydrous sodium sulfate, filtered, and concentrated to obtain a solid (4.2g) with a yield of 78%; LC-MS: [M+H]⁺ =688.3.

### Step 3: Compound 19d

Add **19b** (1000mg, 1.45mmol) in a 25mL single-necked flask, after 15mL DMF is dissolved, add 1000mg5% Pd/C, hydrogenation reaction for 2h, the reaction is complete, filter, place the filtrate in an ice water bath, add DIPEA (248uL, 1.5 mmol), then **M3** (720mg, 1.45mmol) was added, and after the addition, the temperature was raised to room temperature and reacted for 1h. The reaction was monitored by HPLC, and the reaction solution was purified by high performance liquid phase to obtain a preparation solution. The preparation solution was freeze-dried to obtain 503 mg of the product with a yield of 41%; LC-MS: [MH]⁻ =842.3.

### Step 4: Compound 19e-1 and 19e-2

Add **19d** (500mg, 0.59mmol), **M5** (317mg, 0.59mmol), PyBOP (339mg, 0.65mmol), HOBt (88mg, 0.86mmol) and 10mL DMF into a 50mL single-mouth bottle, add DIPEA (292uL, 1.77) under ice water bath mmol), warm to room temperature and react for 2h. After the reaction was finished while monitored by HPLC, the reaction solution was purified by high performance liquid phase to obtain the preparation solutions of compound **19e-1** and compound **19e-2.** The preparation solutions were lyophilized to obtain 112 mg of compound **19e-1.** LC-MS: [M+H]⁺ =1261.5; 131 mg of compound **19e-2,** LC-MS: [M+H]⁺ =1261.5.

### Step 5: Compound 19A

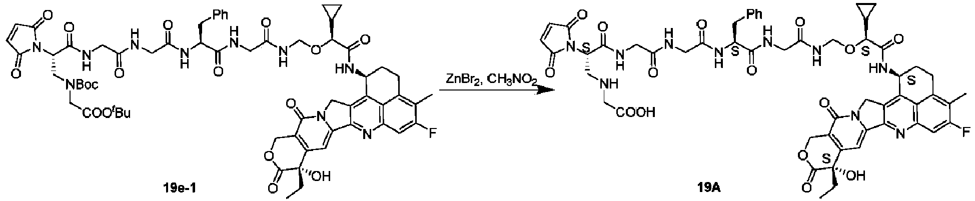

Add **19e-1** (100mg, 0.079mmol), zinc bromide (357mg, 1.59mmol) and 5mL nitromethane into a 25mL single-necked flask, and react at 40°C for 1h. After the reaction was finished while monitored by HPLC, the solvent was removed by concentration under reduced pressure to obtain a crude product. The crude product was purified by high performance liquid phase to obtain a product preparation solution, which was freeze-dried to obtain 55 mg solid; LC-MS: [M+H]⁺ =1105.4.

### Step 6: Compound 19B

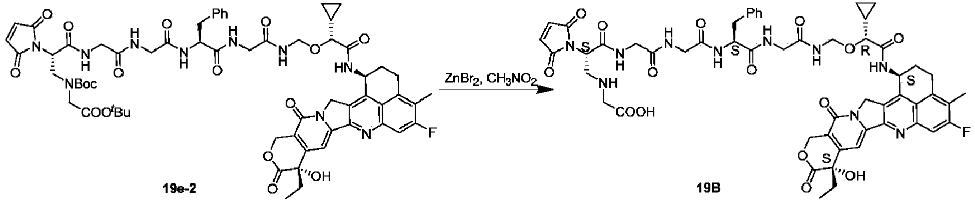

Add **19e-2** (100mg, 0.079mmol), zinc bromide (357mg, 1.59mmol) and 5mL nitromethane into a 25mL single-necked flask, and react at 40°C for 1h. After the reaction was finished while monitored by HPLC, the solvent was removed by concentration under reduced pressure to obtain a crude product. The crude product was purified by high performance liquid phase to obtain a product preparation solution, which was freeze-dried to obtain 58 mg solid; LC-MS: [M+H]⁺ =1105.4.

### Example 31

### Synthesis of compounds 20A and 20B:

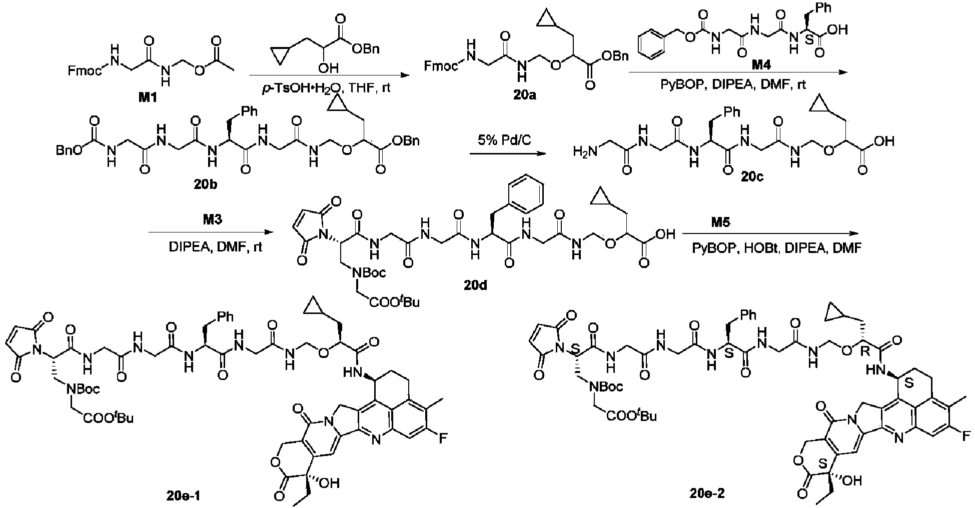

### Step 1: Compound 20a

Add **M1** (10g, 27.1mmol), 2-hydroxy-cyclopropylpropionic acid benzyl ester (synthesized with reference to the method published in patent WO2020063676A) (12.0g, 54.3mmol), zinc acetate (9.96g, 54.3mmol) in a 250mL single-mouth bottle ) and 100mL of toluene, heated to 100°C for 4h. After the reaction was completed, the temperature was lowered to room temperature, the insoluble matter was removed by filtration, and the filtrate was concentrated to obtain a crude product. The crude product was purified by silica gel column chromatography (PE:EA=10:1-5:1-2:1) to obtain the target product 5.09g; LC-MS: [M+H]⁺ =529.2.

### Step 2: Compound 20b

Add **20a** (4g, 7.6mmol) and 10mL DMF to a 50mL single-necked flask. After dissolving, add DBU (1.39g, 9.1mmol) in an ice-water bath, and react for 1 hour, which is recorded as reaction solution ①;
Take another 50mL single-mouth bottle, add **M4** (3.12g, 7.6mmol), PyBOP (4.5g, 8.6mmol), HOBt (1.16g, 8.6mmol) and 10mL DMF. After dissolving, add DIPEA (1.65mL, 10mmol), continue the reaction for 30 minutes, add the reaction solution ①, warm to room temperature and react for 2 hours. The reaction progress was monitored by HPLC. After the reaction was completed, the reaction solution was purified by high performance liquid phase to obtain a preparation solution. The preparation liquid was extracted with dichloromethane, washed with saturated sodium chloride solution, dried with anhydrous sodium sulfate, filtered, and concentrated to obtain 4.5g solid with a yield of 84%; LC-MS: [M+H]⁺ =702.3.

### Step 3: Compound 20d

Add **20b** (1000mg, 1.42mmol) in a 25mL single-necked flask, after 15mL DMF is dissolved, add 1000mg5% Pd/C, hydrogenation reaction for 2h, the reaction is complete, filter, place the filtrate in an ice water bath, add DIPEA (248uL, 1.5 mmol), then **M5** (708mg, 1.42mmol) was added, and after the addition, the temperature was raised to room temperature and reacted for 1h. After the reaction was finished while monitored by HPLC, the reaction solution was purified by high performance liquid phase to obtain a preparation solution. The preparation solution was lyophilized to obtain 443 mg of the product with a yield of 36%; LC-MS: [MH]⁻ =856.4.

### Step 4: Compound 20e-1 and 20e-2

Add **20d** (400mg, 0.47mmol), exatecan mesylate (250mg, 0.47mmol), PyBOP (223mg, 0.56mmol), HOBt (83mg, 0.56mmol) and 10mL DMF to a 50mL single-mouth bottle, ice water bath DIPEA (248uL, 1.5mmol) was added, and the mixture was heated to room temperature and reacted for 2h. After the reaction was finished while monitored by HPLC, the reaction solution was purified by high performance liquid phase to obtain the preparation solutions of compound **20e-1** and compound **20e-2.** The preparation solutions were respectively freeze-dried to obtain 103 mg of compound **20e-1,** LC-MS: [M+H]⁺ =1275.5; 103mg of compound 20e-2, LC-MS: [M+H]⁺ =1275.5.

### Step 5: Compound 20A

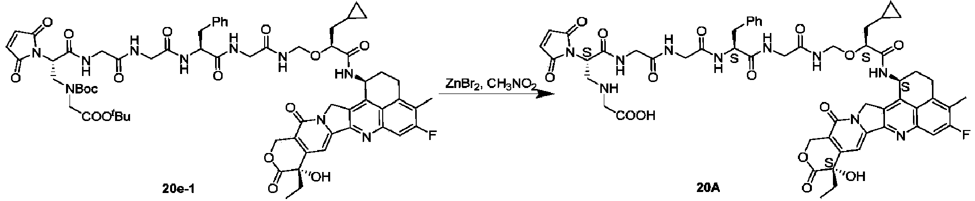

Add **8A** (100mg, 0.078mmol), zinc bromide (352mg, 1.57mmol) and 5mL nitromethane into a 25mL single-neck flask, and react at 40°C for 1h. After the reaction was finished while monitored by HPLC, the solvent was removed by concentration under reduced pressure to obtain a crude product. The crude product was purified by high-performance liquid phase to obtain a product preparation solution, which was freeze-dried to obtain a solid (51mg); LC-MS: [M+H]⁺ =1119.4.

### Step 6: Compound 20B

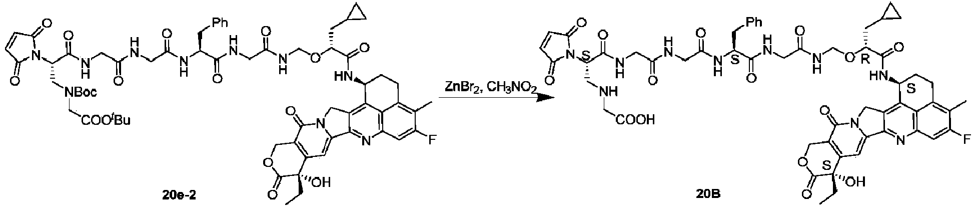

Add **20e-2** (100mg, 0.079mmol), zinc bromide (357mg, 1.59mmol) and 5mL nitromethane into a 25mL single-necked flask, and react at 40°C for 1h. After the reaction was finished while monitored by HPLC, the solvent was removed by concentration under reduced pressure to obtain a crude product. The crude product was purified by high performance liquid phase to obtain a product preparation solution, which was freeze-dried to obtain a solid (47 mg); LC-MS: [M+H]⁺ =1119.4.

### Example 32

### Synthesis of compound 21:

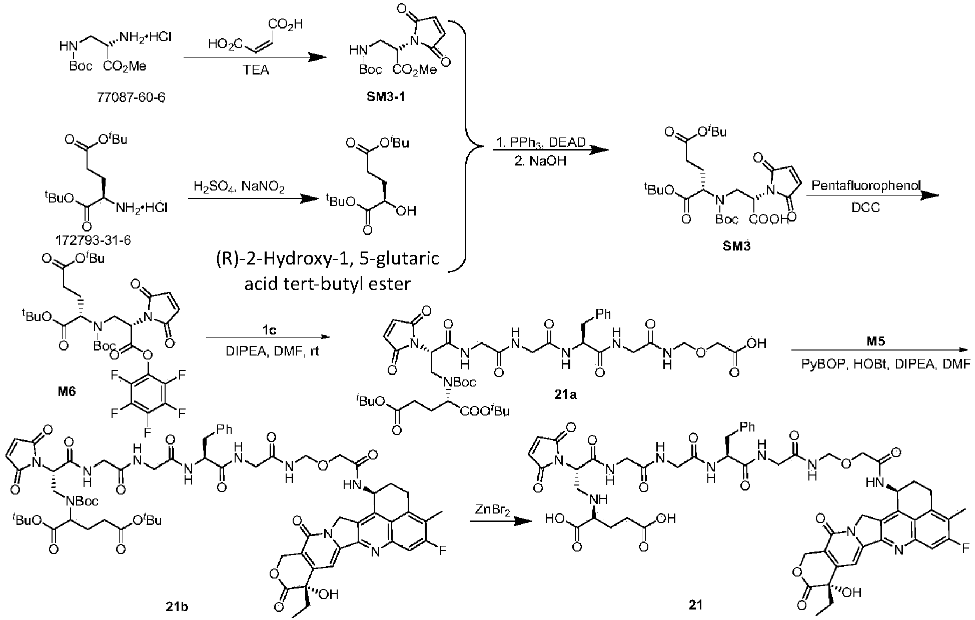

### Step 1: Compound SM3-1

Add 77087-60-6 (100g, 458mmol), maleic acid (53.4g, 460mmol), TEA (64mL, 460mmol) and 1000mL toluene into a 2000mL single-necked flask, and heat to 100°C for 5h to react. After the reaction was completed, the temperature was lowered to room temperature, the insoluble matter was removed by filtration, and the filtrate was concentrated to obtain a crude product. The crude product was purified by silica gel column chromatography (PE:EA=100:1-50:1-20:1) to obtain 75.6 g of the target; LC-MS: [M+H]⁺ =299.1.

### Step 2: Compound (R)-2-hydroxy-1,5-glutaric acid tert-butyl ester

Add 172793-31-6 (100g, 338mmol) and 1000mL of water into a 2000mL single-necked flask, add sodium nitrite (35g, 507mmol), concentrated sulfuric acid (32mL, 35mmol) in turn, slowly warm up to room temperature and react for 24h. After the reaction was completed, 500 mL of ethyl acetate was extracted three times, the organic phase was dried over anhydrous sodium sulfate, filtered, and concentrated under reduced pressure to remove the solvent to obtain a crude product. The crude product was purified by silica gel column chromatography (PE:EA=50:1-30:1-2:1) to obtain 91.2 g of the target product; LC-MS: [M+H]⁺ =261.4.

### Step 3: Compound SM3

Add (R)-2-hydroxy-1,5-glutaric acid tert-butyl ester (50g, 192mmol) and 1000mL anhydrous tetrahydrofuran into a 2000mL single-neck bottle, cool down to 0°C in an ice-water bath, and add PPh ₃ (87.7g, 288mmol), DEAD (50.2g, 288mmol) and SM3-1 (57.3,192mmol), slowly warm up to room temperature and react for 13h. After the reaction was completed, the insoluble matter was removed by filtration, and the filtrate was concentrated to obtain a crude product. The crude product was purified by silica gel column chromatography (PE:EA=50:1-30:1-1:1) to obtain 68.6g of product;
Dissolve the above product in 500mL methanol, cool to 0°C in an ice-water bath, add NaOH (64mL, 190mmol, 3M/L) dropwise at this temperature, maintain the temperature for 12h, add HCl (6M/L) to adjust the pH To 3, extract five times with 500 mL of dichloromethane, dry with anhydrous sodium sulfate, filter, and concentrate the filtrate under reduced pressure. The resulting crude product is purified by column chromatography (DCM/MeOH=50/1-20/1-2/1). Obtained **SM3** 50.4g; LC-MS: [MH]⁻ =525.5.

### Step 4: Compound M6

In a 2000mL single-mouth flask, add compound **SM3** (50g, 95mmol, 1.0eq), pentafluorophenol (19.2g, 104.5mmol, 1.1eq), DCC (21.5g, 104.5mmol, 1.1eq) and THF (600mL), and react at room temperature 1h (monitoring by TLC), filter to remove insoluble matter. The reaction solution was directly purified by preparation, and the preparation solution was concentrated by a water pump under reduced pressure at 35° C. to remove acetonitrile, and lyophilized to obtain compound **M6** (51.9 g) with a yield of 79%; LC-MS: [M+H]⁺ =693.3.

### Step 5: Compound 21a

Add **1c** (1g, 2.36mmol) to a 25mL single-necked flask. After 25mL of DMF is dissolved, DIPEA (430uL, 2.6mmol) is added, and then **M6** (1177mg, 2.36mmol) is added. After the addition, it is heated to room temperature and reacted for 1h. After the reaction was finished while monitored by HPLC, the reaction solution was purified by high performance liquid phase to obtain a preparation solution, which was freeze-dried to obtain 555 mg of product; LC-MS: [MH]⁻ =931.0.

### Step 6: Compound 21b

Add **21a** (500mg, 0.54mmol), exatecan mesylate **M5** (285mg, 0.54mmol), PyBOP (239mg, 0.6mmol), HOBt (239mg, 0.6mmol) and 10mL DMF, ice DIPEA (248uL, 1.5mmol) was added under a water bath and heated to room temperature to react for 2h. After the reaction was finished while monitored by HPLC, the reaction solution was purified by high performance liquid phase to obtain a preparation solution of compound **21b,** and the preparation solution was lyophilized to obtain compound 231 mg; LC-MS: [M+H]⁺ =1349.5.

### Step 7: Compound 21

Compound **21b** (200mg, 0.1488mmol), zinc bromide (665mg, 2.96mmol) and 10mL nitromethane were added to a 25mL single-necked flask, and reacted at 40°C for 1h. After the reaction was finished while monitored by HPLC, the solvent was removed by concentration under reduced pressure to obtain a crude product. The crude product was purified by high performance liquid phase to obtain a product preparation solution, which was freeze-dried to obtain a solid (103mg); LC-MS: [M+H]⁺ =1137.5.

### Example 33

### Synthesis of compound 22:

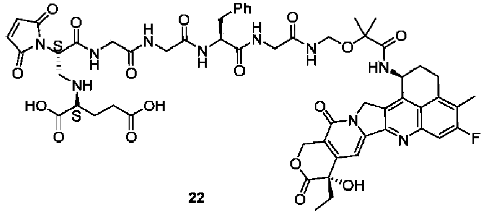

Using compounds **M6** and **3c** as starting materials, referring to the synthetic route of Example 32, compound **22** (91 mg) was obtained; LC-MS: [M+H]⁺ =1165.5.

### Example 34

### Synthesis of compounds 23 and 24:

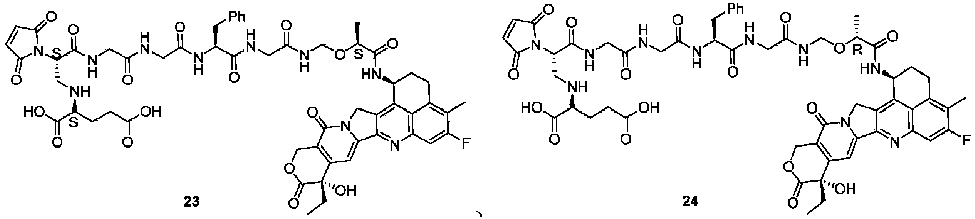

Using compounds **M6** and 5c as starting materials, referring to the synthetic route of Example 32, 102 mg of compound **23** was obtained, LC-MS: [M+H]⁺ =1151.4; 99 mg of compound **24** was obtained, LC-MS: [M+H]⁺ = 1151.4.

### Example 35

### Synthesis of compounds 25 and 26:

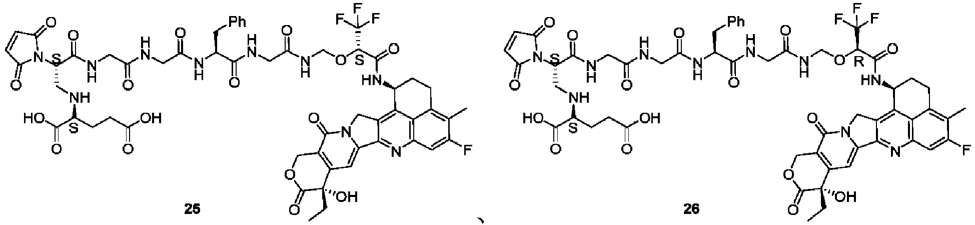

Using compounds **M6** and **7c** as starting materials and referring to the synthetic route of Example 32, 83mg of compound **25** was obtained, LC-MS: [M+H]⁺ =1205.7; 80mg of compound **26** was obtained, LC-MS: [M+H]⁺ =1205.7.

### Example 36

### Synthesis of compounds 27 and 28:

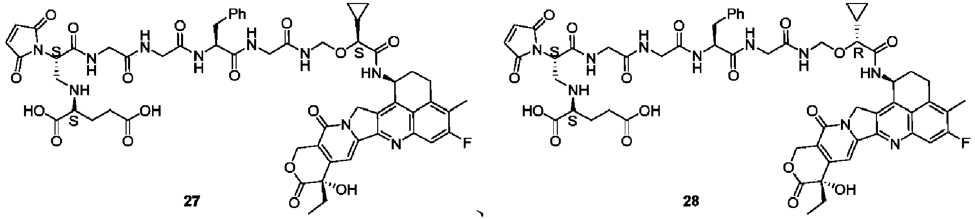

Using compounds **M6** and **19c** as starting materials and referring to the synthetic route of Example 32, 100 mg of compound **27** was obtained, LC-MS: [M+H]⁺ = 1177.5; 101 mg of compound **28** was obtained, LC-MS: [M+H]⁺ = 1177.5.

### Example 37

### Synthesis of compound 29:

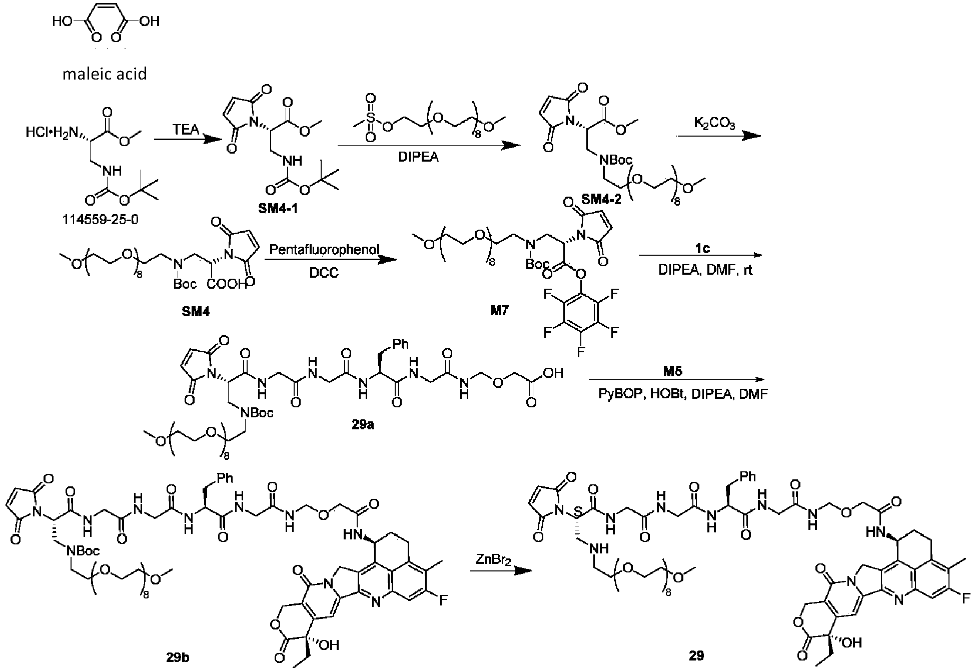

### Step 1: Compound SM4-1

In a 5000mL single-mouth flask, add maleic acid (50g, 431mmol, 1.0eq), 114559-25-0 (110g, 431mmol, 1eq), TEA (263g, 2.16mol, 5eq) and toluene (2000mL), and heat to reflux for reaction 5h (monitoring by TLC), filter to remove insoluble matter. The reaction solution was directly spinned under reduced pressure to remove the solvent, and the residue was subjected to silica gel column chromatography (PE/EA=50/1-20/1-1/1) to obtain **SM4-1** (64.7g) with a yield of 50%; LC- MS: [M+H]⁺ =299.2.

### Step 2: Compound SM4-2

Add **SM4-1** (64g, 215mmol) to a 2000mL single-necked flask. After 1000mL DMF is dissolved, add DIPEA (71mL, 430mmol), then add nonethylene glycol monomethyl ether methanesulfonate (111.5g, 220mmol), add After rising to room temperature, react for 2h. The reaction was monitored by HPLC, and the reaction solution was purified by silica gel column chromatography (PE/EA=50/1-20/1-1/1) to obtain 59.9 g of the product; LC-MS: [M+H]⁺ =709.4.

### Step 3: Compound SM4

**SM4-2** (59 g, 83 mmol) was added to a 2000 mL single-mouth flask, and after 1000 mL of MeOH was dissolved, K ₂ CO ₃ (11.75 g, 85 mmol) was added, and the addition was completed and the reaction was carried out at room temperature for 4 hours. The reaction was monitored by HPLC and the insoluble matter was removed by filtration. The reaction solution was directly purified by preparation. The preparation solution was concentrated in a water pump under reduced pressure water bath at 35°C to remove acetonitrile, and lyophilized to obtain compound **SM4** (27g); LC-MS: [MH]⁻ =693.5.

### Step 4: Compound M7

In a 500mL single-mouth flask, add compound **SM4** (25g, 36mmol, 1.0eq), pentafluorophenol (7.3g, 40mmol, 1.1eq), DCC (8.2g, 40mmol, 1.1eq) and THF (200mL), and react at room temperature for 1h ( Use TLC to monitor), filter to remove insoluble matter. The reaction solution was directly purified by preparation, the preparation solution was concentrated in a vacuum water bath at 35° C. to remove acetonitrile, and lyophilized to obtain compound **M7** (23.3 g) with a yield of 93%; LC-MS: [M+H]⁺ =695.8.

### Step 5: Compound 29a

Add 1c (1g, 2.36mmol) to a 25mL single-necked flask. After 25mL of DMF is dissolved, DIPEA (430uL, 2.6mmol) is added, then **M7** (1640mg, 2.36mmol) is added, and the mixture is heated to room temperature and reacted for 1h. After the reaction was finished while monitored by HPLC, the reaction solution was purified by high performance liquid phase to obtain a preparation solution, which was freeze-dried to obtain 609 mg of product; LC-MS: [MH]-=1098.5.

### Step 6: Compound 29b

Add **29a** (500mg, 0.45mmol), exatecan mesylate **M5** (240mg, 0.45mmol), PyBOP (215mg, 0.54mmol), HOBt (215mg, 0.54mmol) and 10mL DMF, ice DIPEA (248uL, 1.5mmol) was added under a water bath and heated to room temperature to react for 2h. After the reaction was finished while monitored by HPLC, the reaction solution was purified by high performance liquid phase to obtain a preparation solution of compound **29b,** and the preparation solution was freeze-dried to obtain compound 187 mg; LC-MS: [M+H]⁺ =1517.6.

### Step 7: Compound 29

Compound **29b** (150 mg, 0.988 mmol), zinc bromide (223 mg, 0.988 mmol) and 10 mL of nitromethane were added to a 25 mL single-necked flask, and reacted at 40°C for 1 h. After the reaction was finished while monitored by HPLC, the solvent was removed by concentration under reduced pressure to obtain a crude product. The crude product was purified by high performance liquid phase to obtain a product preparation solution, which was freeze-dried to obtain a solid (114mg); LC-MS: [M+H]⁺ =1517.9.

### Example 38

### Synthesis of compound 30:

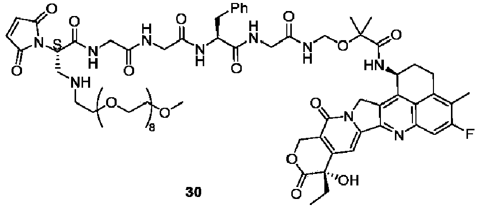

Using compounds **M7** and **3c** as starting materials, referring to the synthetic route of Example 37, compound **30** (125 mg) was obtained; LC-MS: [M+H]⁺ =1445.6.

### Example 39

### Synthesis of compound 31 and 32:

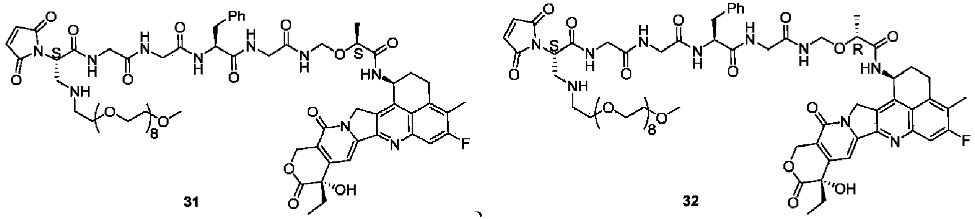

Using compound **M7** and **5c** as starting materials, referring to the synthetic route of Example 37, 61mg of compound **31** was obtained, LC-MS: [M+H]⁺ =1431.7; 63 mg of compound **32** was obtained, LC-MS: [M+H]⁺ =1431.7.

### Example 40

### Synthesis of compounds 33 and 34:

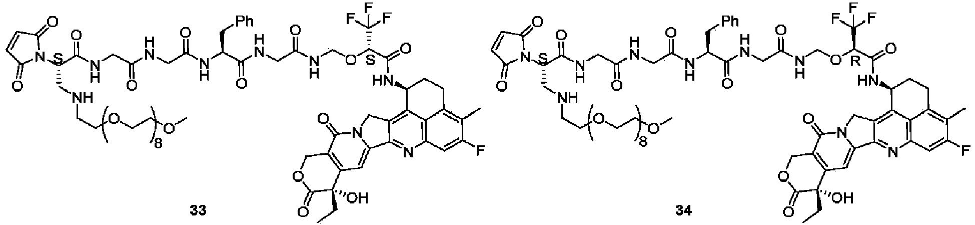

Using compounds **M7** and 7c as starting materials, referring to the synthetic route of Example 37, 60 mg of compound 33 was obtained, LC-MS: [M+H]⁺ =1485.6; 58 mg of compound 34 was obtained, LC-MS: [M+H]⁺ = 1485.6.

### Example 41

### Synthesis of compounds 35 and 36:

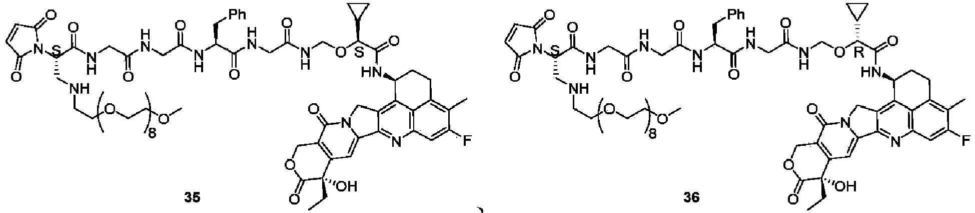

Using compound **M7** and 19c as starting materials, referring to the synthetic route of Example 37, 102 mg of compound **35** was obtained, LC-MS: [M+H]⁺ =1457.8; 102 mg of compound **36** was obtained, LC-MS: [M+H]⁺ =1457.8.

### Example 42

### Synthesis of compound 37:

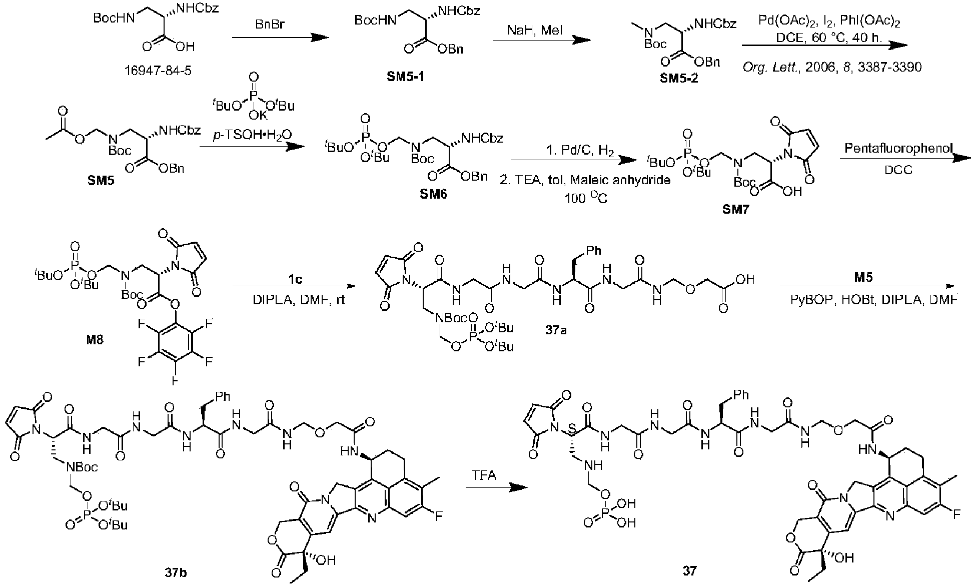

### Step 1: Compound SM5-1

In a 2000mL single-mouth flask, add compound 16947-84-5 (100g, 295mmol, 1.0eq), DIPEA (50mL, 300mmol), benzyl bromide (51.3g, 300mmol) and THF (1000mL), and react at room temperature for 12h (monitored byTLC)), filter to remove insoluble matter. The reaction solution was directly rotated under reduced pressure to remove the solvent, and the residue was subjected to silica gel column chromatography (PE/EA=50/1-20/1-2/1) to obtain **SM5-1** (110.1g) with a yield of 87%; LC- MS: [M+H]⁺ =429.2.

### Step 2: Compound SM5-2

In a 2000mL single-mouth flask, add compound **SM5-1** (100g, 233.4mmol, 1.0eq) and THF (1000mL), cool to 0°C in an ice water bath, add NaH (37.4g, 933.5mmol), Mel (132.5g, 933.5mmol), the reaction was maintained at 0°C for 24h (monitored byTLC), 500 mL of saturated NH 4 Cl aqueous solution was added to quench the reaction, 500 mL of ethyl acetate was extracted three times, the organic phase was dried with anhydrous sodium sulfate, and filtered. The filtrate was directly rotated under reduced pressure to remove the solvent, and the residue was subjected to silica gel column chromatography (PE/EA=100/1-50/1-10/1) to obtain SM5-2 (37.1g); LC-MS: [M+H ] ⁺ =443.3.

The third step: compound **SM5** (refer to the literature Org. Lett., 2006, 8, 3387-3390.)

In a 1000mL single-mouth flask, add compound **SM5-2** (35g, 79mmol, 1.0eq) and DCE (500mL), add palladium diacetate (180mg, 0.8mmol), I ₂ (20g, 79mmol), diacetate iodobenzene (40.8 g, 126.4 mmol), heated to 60°C and reacted for 40h (monitored by TLC), quenched by adding 500 mL of saturated sodium thiosulfate aqueous solution, extracted three times with 500 mL of dichloromethane, dried the organic phase with anhydrous sodium sulfate, and filtered. The filtrate was directly rotated under reduced pressure to remove the solvent, and the residue was subjected to silica gel column chromatography (PE/EA=100/1-50/1-10/1) to obtain **SM5** (28g); LC-MS: [M+H]⁺ = 501.3.

### Step 4: Compound SM6

In a 500mL single-mouth flask, add compound **SM5** (25g, 50mmol, 1.0eq), di-tert-butyl phosphate potassium salt (13.66g, 55mmol, 1.1eq), monohydrate p-toluenesulfonic acid (951mg, 5mmol, 0.1eq) and THF (200 mL), react at room temperature for 1 h (monitored byTLC), and filter to remove insoluble materials. The reaction solution was directly purified by preparation. The preparation solution was concentrated in a vacuum water bath at 35° C. to remove acetonitrile, and lyophilized to obtain compound **SM6** (15.1 g) with a yield of 46%; LC-MS: [M+H]⁺ =651.4.

### Step 5: Compound SM7

Add **SM6** (15g, 23mmol) and 100mL DMF into a 250mL single-necked flask. After dissolving it, add 15g 5% Pd/C in an ice-water bath, and replace the atmosphere in the system with hydrogen three times. React at room temperature for 12 hours, and filter to remove Pd/C. , The oil pump decompresses and evaporates to remove the solvent, set aside;
Take another 250mL single-necked flask, add the above crude product and 100mL toluene, triethylamine (6.4mL, 46mmol), maleic anhydride (2.4g, 24mmol), after dissolving, raise to 100°C to react for 2h. The reaction progress was monitored by HPLC. After the reaction was completed, the reaction solution was purified by high performance liquid phase to obtain a preparation solution. The preparation liquid was extracted with dichloromethane, washed with saturated sodium chloride solution, dried with anhydrous sodium sulfate, filtered, and concentrated to obtain a solid (4.2g) with a yield of 36%; LC-MS: [M+H]⁺ =507.3.

### Step 6: Compound M8

In a 100mL single-mouth flask, add compound SM7 (4g, 7.9mmol, 1.0eq), pentafluorophenol (1.6g, 8.7mmol, 1.1eq), DCC (1.8g, 8.7mmol, 1.1eq) and THF (60mL), room temperature The reaction was carried out for 1 hour (monitored byTLC), and the insoluble matter was filtered off. The reaction solution was directly purified by preparation. The preparation solution was concentrated in a vacuum water bath at 35° C. to remove acetonitrile, and lyophilized to obtain compound **M8** (3.7 g) with a yield of 70%; LC-MS: [M+H]⁺ =673.2.

### Step 7: Compound 37a

Add 1c (1g, 2.36mmol) to a 25mL single-necked flask. After 25mL of DMF is dissolved, DIPEA (430uL, 2.6mmol) is added, and then **M8** (1.2g, 2.36mmol) is added. After the addition, it is heated to room temperature and reacted for 1h. After the reaction was finished while monitored by HPLC, the reaction solution was purified by high performance liquid phase to obtain a preparation solution, which was freeze-dried to obtain 488 mg of product; LC-MS: [MH]⁻ =911.0.

### Step 8: Compound 37b

Add 37a (400mg, 0.44mmol), exatecan mesylate **M5** (235mg, 0.44mmol), PyBOP (199mg, 0.5mmol), HOBt (69mg, 0.5mmol) and 10mL DMF in a 100mL single-mouth bottle. DIPEA (218uL, 1.32mmol) was added under a water bath and heated to room temperature to react for 2h. After the reaction was finished while monitored by HPLC, the reaction solution was purified by high performance liquid phase to obtain a preparation solution of compound 37b. The preparation solution was lyophilized to obtain compound 201 mg; LC-MS: [M+H]⁺ =1329.6.

### Step 9: Compound 37

Compound **37b** (130mg, 0.098mmol), zinc bromide (221mg, 0.98mmol) and 10mL nitromethane were added to a 25mL single-necked flask, and reacted at 40°C for 1h. After the reaction was finished while monitored by HPLC, the solvent was removed by concentration under reduced pressure to obtain a crude product. The crude product was purified by high performance liquid phase to obtain a product preparation solution, which was freeze-dried to obtain a solid (96mg); LC-MS: [M+H]⁺ =1117.4.

### Example 43

### Synthesis of compound 38:

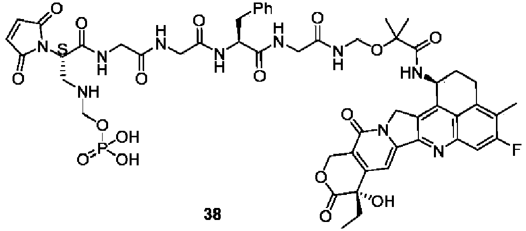

Using compounds **M8** and 3c as starting materials, referring to the synthetic route of Example 42, compound **38** (51 mg) was obtained; LC-MS: [M+H]⁺ =1145.6.

### Example 44

### Synthesis of compound 39 and 40:

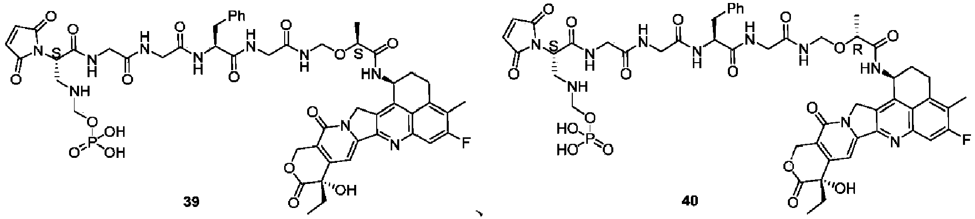

Using compound **M8** and 5c as starting materials, referring to the synthetic route of Example 42, 57 mg of compound **39** was obtained, LC-MS: [M+H]⁺ =1131.4; 60 mg of compound **40** was obtained, LC-MS: [M+H]⁺ =1131.4.

### Example 45

### Synthesis of compound 41 and 42:

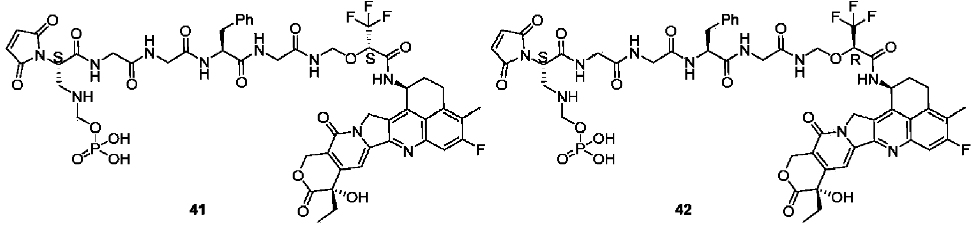

Using compounds **M7** and **7c** as starting materials, referring to the synthetic route of Example 42, 44 mg of compound **41** was obtained, LC-MS: [M+H]⁺ =1185.3; 44 mg of compound **42,** LC-MS: [M+H]⁺ =1185.3.

### Example 46

### Synthesis of compounds 43 and 44:

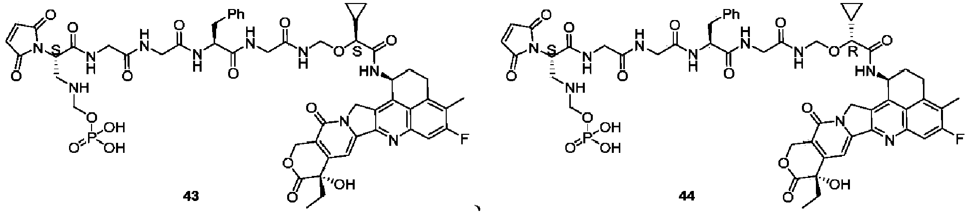

Using compounds **M8** and **19c** as starting materials, referring to the synthetic route of Example 42, 62 mg of compound **43** was obtained, LC-MS: [M+H]⁺ =1157.4; 59 mg of compound **44** was obtained, LC-MS: [M+H]⁺ = 1157.4.

### Example 47 (comparative example)

### Synthesis of compound 45:

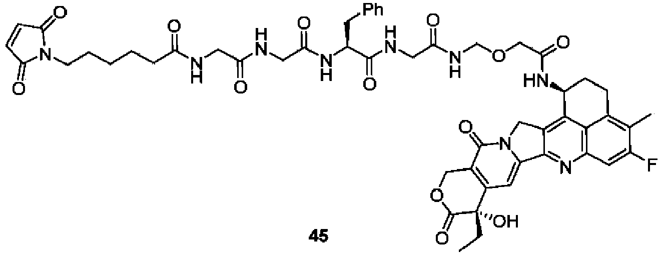

Compound **45** was synthesized with reference to the method provided in Example 58 of the patent "CN104755494A".

The following is the sequence of Trastuzumab:
Light chain 
MDMRVPAQLLGLLLLWLRGARCDIQMTQSPSSLSASVGDRVTITCRASQDVNTAVAWYQQKPGKAPKLLIYS
ASFLYSGVPSRFSGSRSGTDFTLTISSLQPEDFATYYCQQHYTTPPTFGQGTKVEIKRTVAAPSVFIFPPSDEQLKS
GTASVVCLLNNFYPREAKVQWKVDNALQSGNSQESVTEQDSKDSTYSLSSTLTLSKADYEKHKVYACEVTHQ
GLSSPVTKSFNRGEC*
Heavy chain

MDMRVPAQLLGLLLLWLRGARCEVQLVESGGGLVQPGGSLRLSCAASGFNIKDTYIHWVRQAPGKGLEWVA
RIYPTNGYTRYADSVKGRFTISADTSKNTAYLQMNSLRAEDTAVYYCSRWGGDGFYAMDYWGQGTLVTVSSA
STKGPSVFPLAPSSKSTSGGTAALGCLVKDYFPEPVTVSWNSGALTSGVHTFPAVLQSSGLYSLSSVVTVPSSSL
GTQTYICNVNHKPSNTKVDKKVEPKSCDKTHTCPPCPAPELLGGPSVFLFPPKPKDTLMISRTPEVTCVVVDVS
HEDPEVKFNWYVDGVEVHNAKTKPREEQYNSTYRVVSVLTVLHQDWLNGKEYKCKVSNKALPAPIEKTISKA
KGQPREPQVYTLPPSREEMTKNQVSLTCLVKGFYPSDIAVEWESNGQPENNYKTTPPVLDSDGSFFLYSKLTV
DKSRWQQGNVFSCSVMHEALHNHYTQKSLSLSPG

### Preparation of ligand-drug conjugate:

### 1) General coupling method

After the preliminary purification, the antibody molecules whose monomer ratio is greater than 95% are exchanged into a phosphate buffer solution with an ultrafiltration centrifuge tube at a concentration of 10 mg/mL. Add 20 times the number of moles of antibody TCEP, and react for 4 hours at room temperature to open the disulfide bond between antibody chains. The linker-drug compound (payload) was added 20 times the number of mole molecules of the antibody, and reacted for 2 hours at room temperature. After the reaction is over, use an ultrafiltration centrifuge tube with a molecular weight cut-off of 30KDa to exchange the liquid into PBS, and remove uncoupled payload. After changing the liquid, the ADC sample is filtered with a 0.22 micron sterile filter for use.

### 2) Determination of DAR value of ligand-drug conjugate

Single rate detection conditions:
The sample was centrifuged at 14000 rpm for 5 minutes, and the supernatant was taken for analysis;
Instrument: Waters e2695 (2489UV/Vis);
Chromatographic column: TSKgel G3000SWXL (7.8×300mm, 5µm);
Mobile phase: A: 50mM PB, 300mM NaCl, 200mM Arg, 5% IPA, pH 6.5;
The mobile phase A was eluted isocratically for 30 min, flow rate: 0.714 mL/min, column temperature 25°C, detection wavelength: 280 nm.

DAR detection conditions:
The sample was centrifuged at 14000 rpm for 5 minutes, and the supernatant was taken for analysis;
Instrument: Waters H-class (TUV);
Chromatographic column: Proteomix HIC Butyl-NP5 (4.6×35mm, 5µm);
Mobile phase: A: 1.5M ammonium sulfate, 0.025M anhydrous sodium phosphate, pH 7.0, B: 0.025M anhydrous sodium phosphate, 25% IPA, pH 7.0;
The mobile phase A equilibrates the chromatographic column, the mobile phase A and B are gradient eluted, the flow rate is 0.8mL/min; the column temperature is 25°C, and the detection wavelength is 214nm.

### Example 48: ADC-1

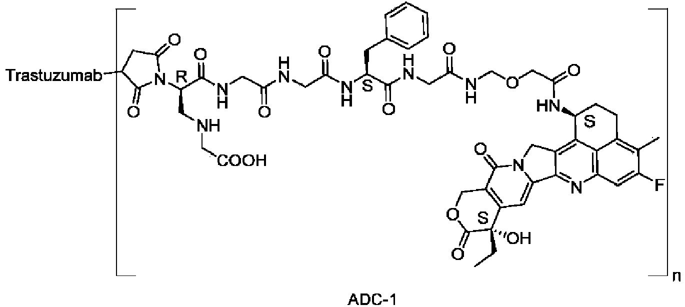

ADC-1 was prepared according to the general coupling method.

### Example 49: ADC-2

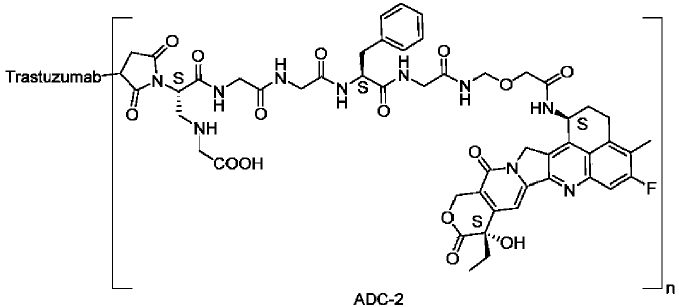

ADC-2 was prepared according to the general coupling method.

### Example 50: ADC-3

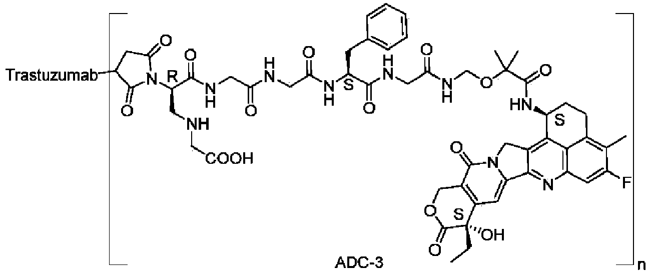

ADC-3 was prepared according to the general coupling method.

### Example 51: ADC-4

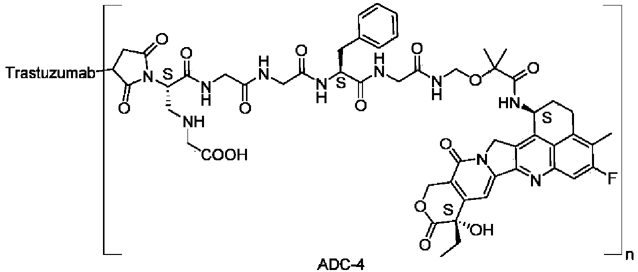

ADC-4 was prepared according to the general coupling method.

### Example 52: ADC-5

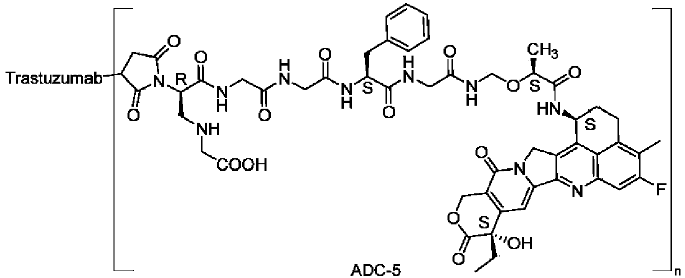

ADC-5 was prepared according to the general coupling method.

### Example 53: ADC-6

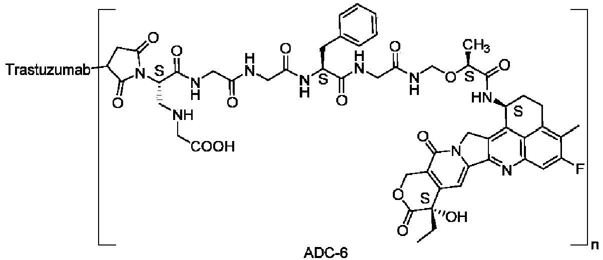

ADC-6 was prepared according to the general coupling method.

### Example 54: ADC-7

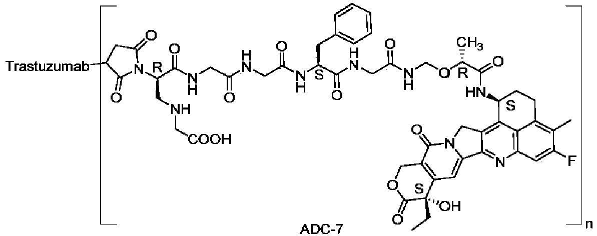

ADC-7 was prepared according to the general coupling method.

### Example 55: ADC-8

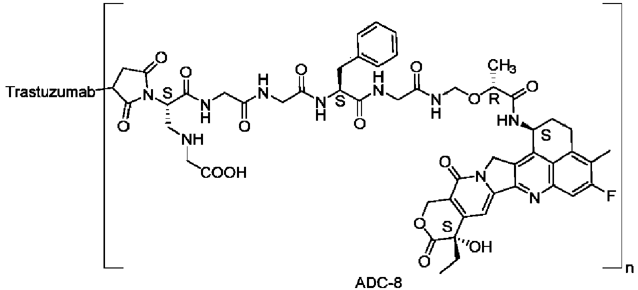

ADC-8 was prepared according to the general coupling method.

### Example 56: ADC-9

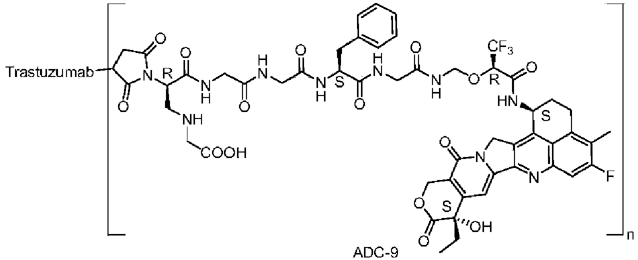

ADC-9 was prepared according to the general coupling method.

### Example 57: ADC-10

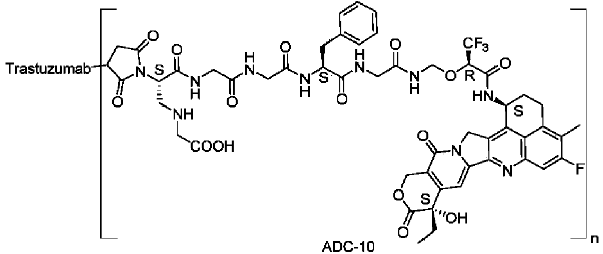

ADC-10 was prepared according to the general coupling method.

### Example 58: ADC-11

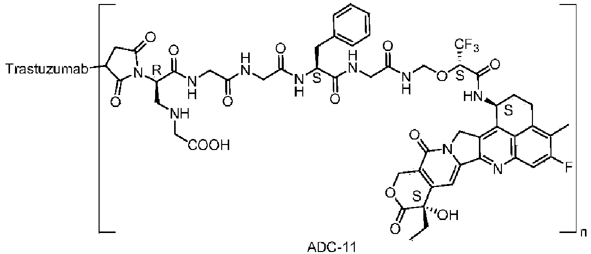

ADC-11 was prepared according to the general coupling method.

### Example 59: ADC-12

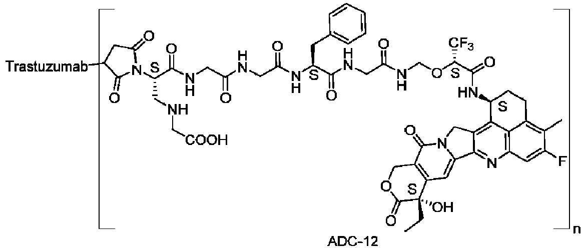

ADC-12 was prepared according to the general coupling method.

### Example 60: ADC-13

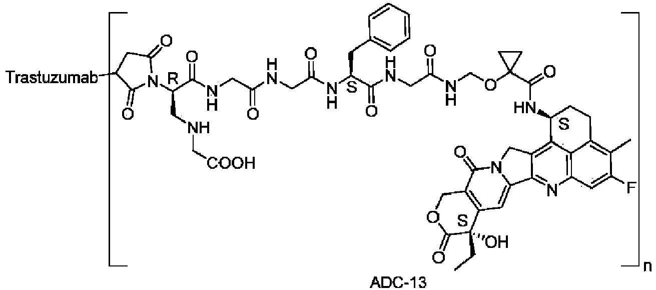

ADC-13 was prepared according to the general coupling method.

### Example 61: ADC-14

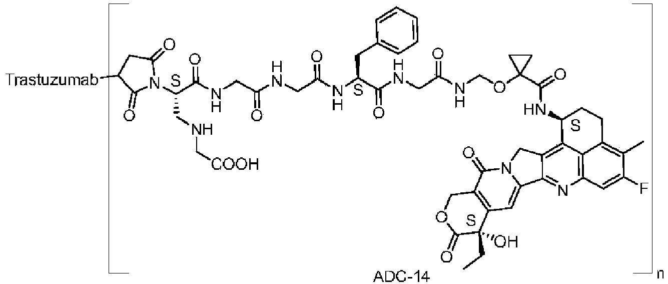

ADC-14 was prepared according to the general coupling method.

### Example 62: ADC-15

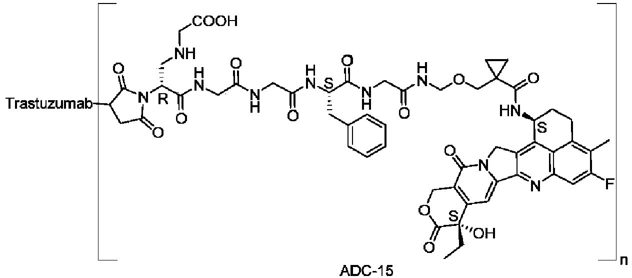

ADC-15 was prepared according to the general coupling method.

### Example 63: ADC-16

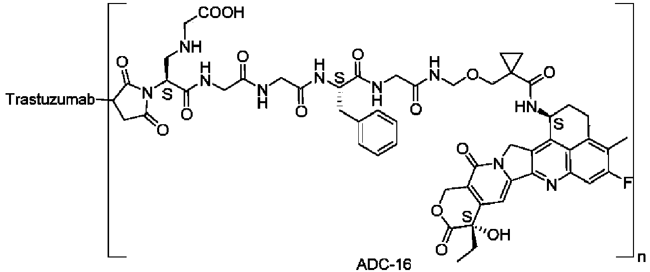

ADC-16 was prepared according to the general coupling method.

### Example 64: ADC-17

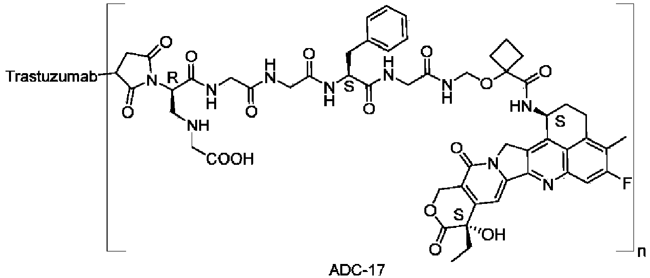

ADC-17 was prepared according to the general coupling method.

### Example 65: ADC-18

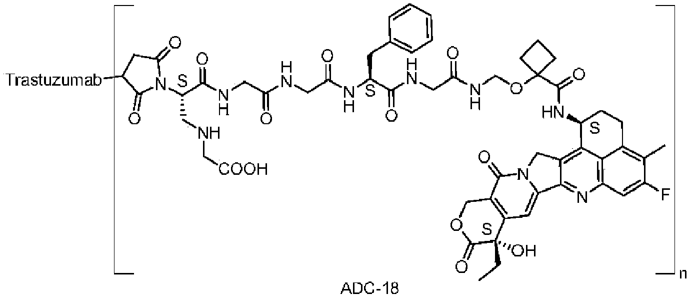

ADC-18 was prepared according to the general coupling method.

### Example 66: ADC-19

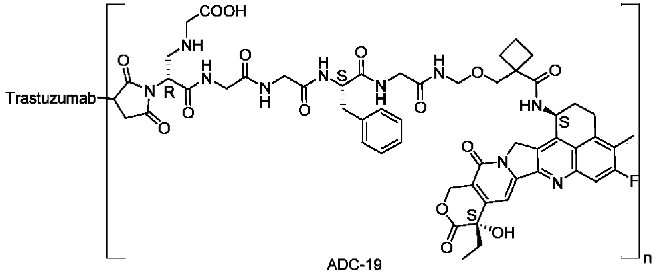

ADC-19 was prepared according to the general coupling method.

### Example 67: ADC-20

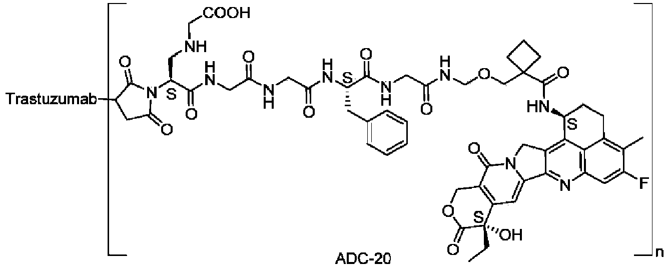

ADC-20 was prepared according to the general coupling method.

### Example 68: ADC-21

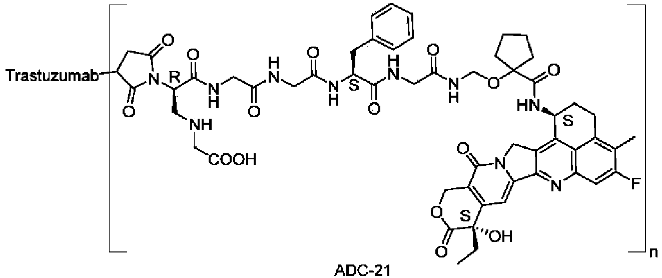

ADC-21 was prepared according to the general coupling method.

### Example 69: ADC-22

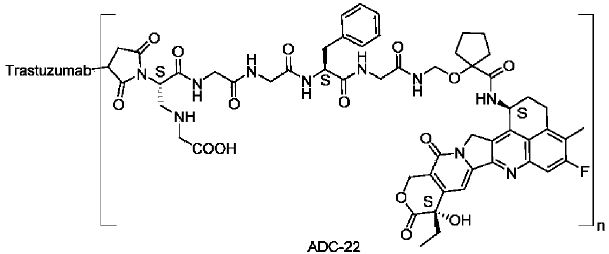

ADC-22 was prepared according to the general coupling method.

### Example 70: ADC-23

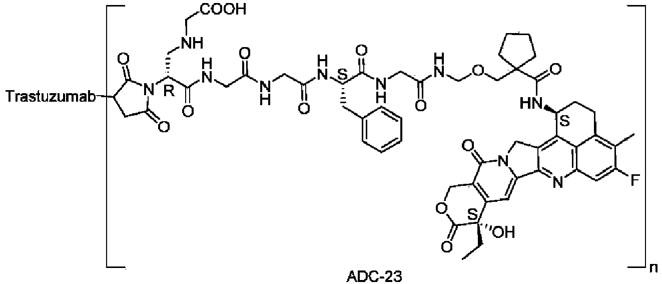

ADC-23 was prepared according to the general coupling method.

### Example 71: ADC-24

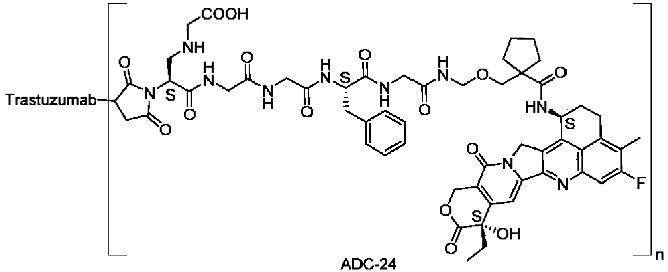

ADC-24 was prepared according to the general coupling method.

### Example 72: ADC-25

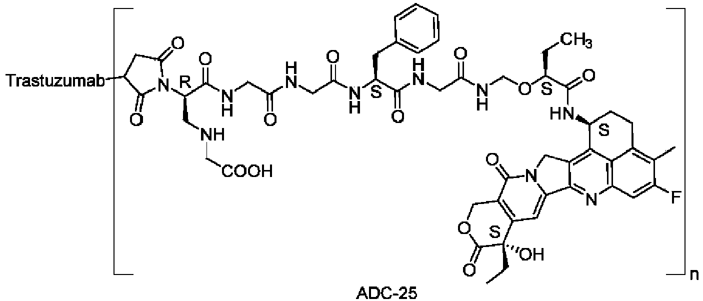

ADC-25 was prepared according to the general coupling method.

### Example 73: ADC-26

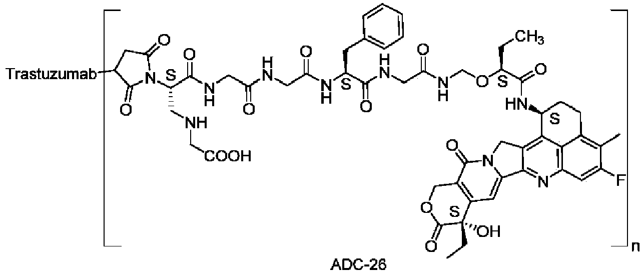

ADC-26 was prepared according to the general coupling method.

### Example 74: ADC-27

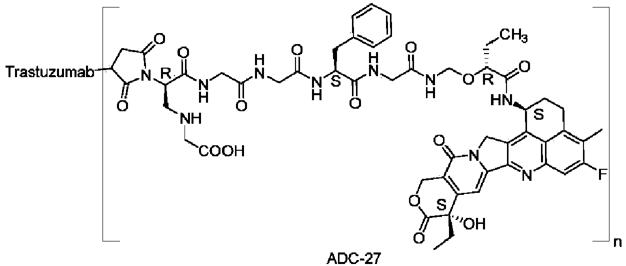

ADC-27 was prepared according to the general coupling method.

### Example 75: ADC-28

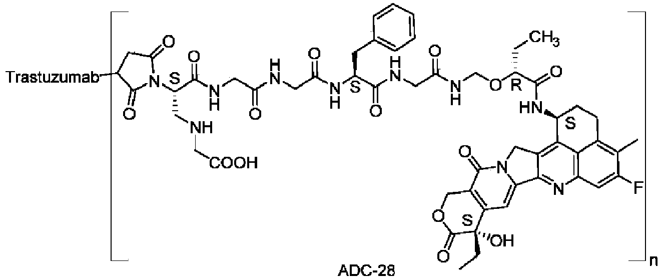

ADC-28 was prepared according to the general coupling method.

### Example 76: ADC-29

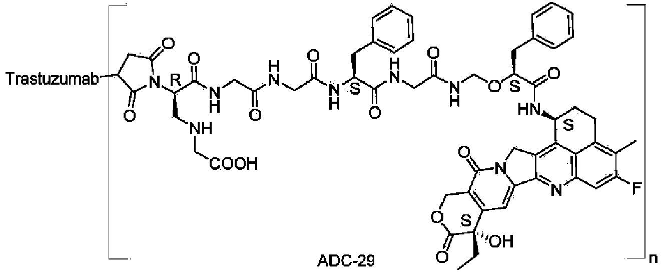

ADC-29 was prepared according to the general coupling method.

### Example 77: ADC-30

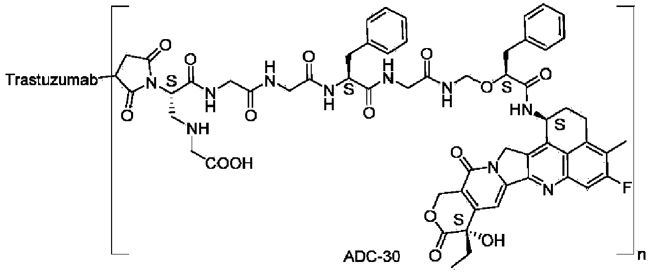

ADC-30 was prepared according to the general coupling method.

### Example 78: ADC-31

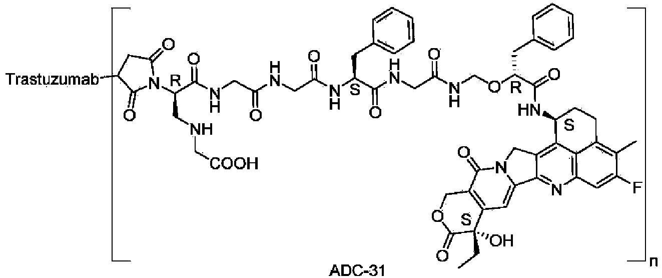

ADC-31 was prepared according to the general coupling method.

### Example 79: ADC-32

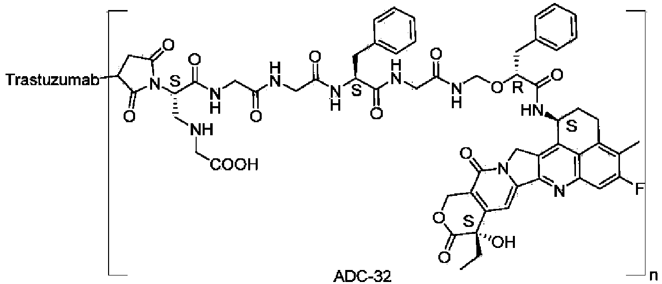

ADC-32 was prepared according to the general coupling method.

### Example 80: ADC-33

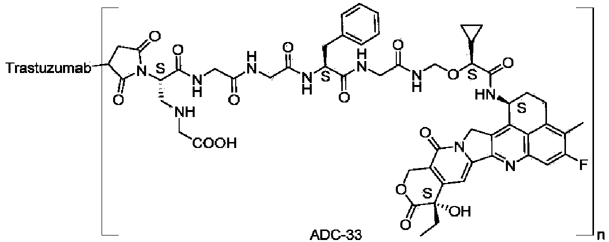

ADC-33 was prepared according to the general coupling method.

### Example 81: ADC-34

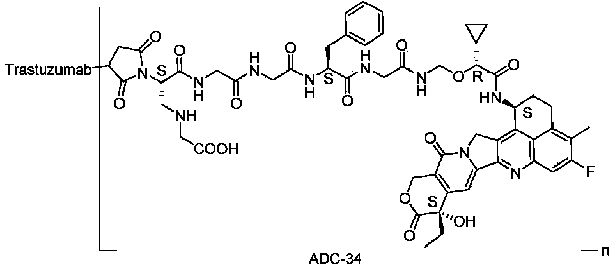

ADC-34 was prepared according to the general coupling method.

### Example 82: ADC-35

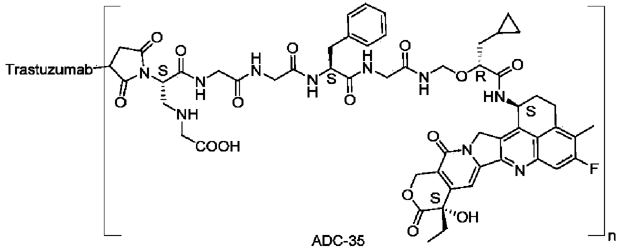

ADC-35 was prepared according to the general coupling method.

### Example 83: ADC-36

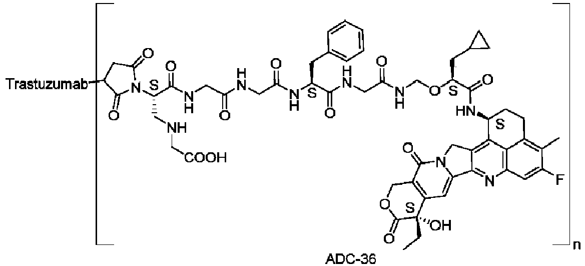

ADC-36 was prepared according to the general coupling method.

### Example 84: ADC-37

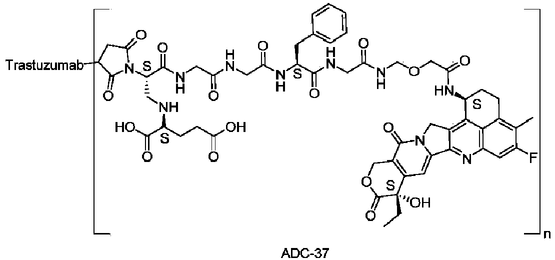

ADC-37 was prepared according to the general coupling method.

### Example 85: ADC-38

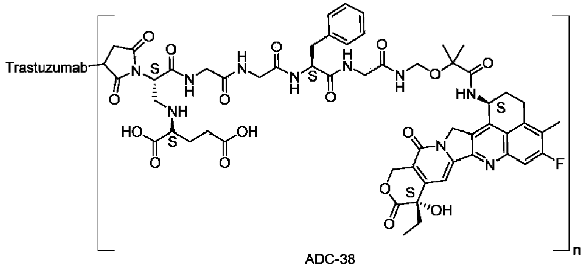

ADC-38 was prepared according to the general coupling method.

### Example 86: ADC-39

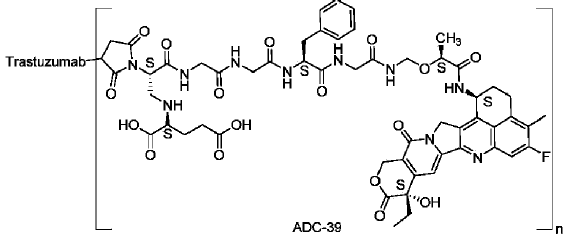

ADC-39 was prepared according to the general coupling method.

### Example 87: ADC-40

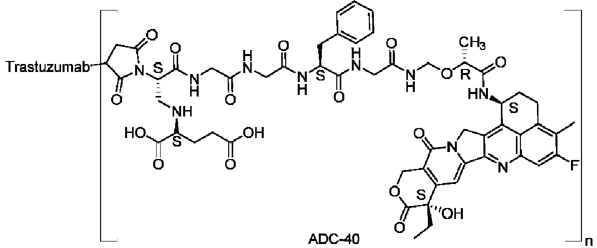

ADC-40 was prepared according to the general coupling method.

### Example 88: ADC-41

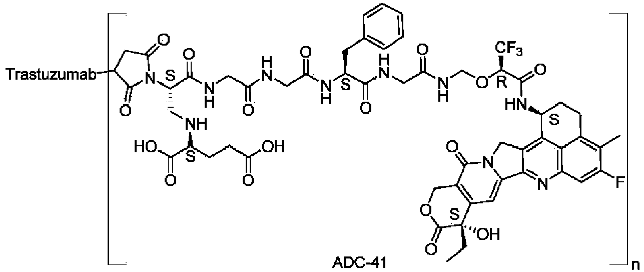

ADC-41 was prepared according to the general coupling method.

### Example 89: ADC-42

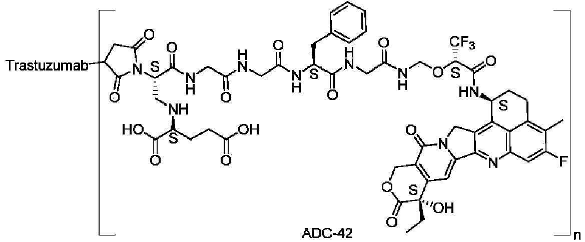

ADC-42 was prepared according to the general coupling method.

### Example 90: ADC-43

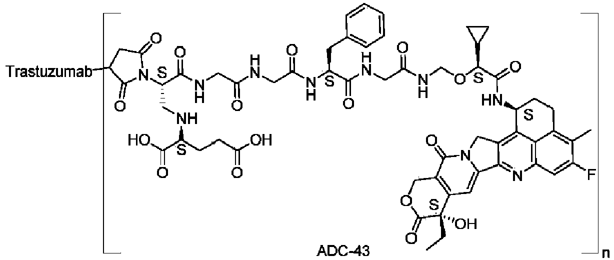

ADC-43 was prepared according to the general coupling method.

### Example 91: ADC-44

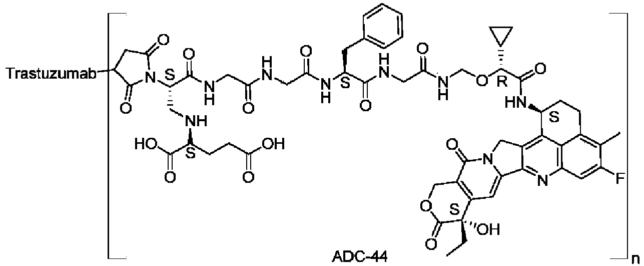

ADC-44 was prepared according to the general coupling method.

### Example 92: ADC-45

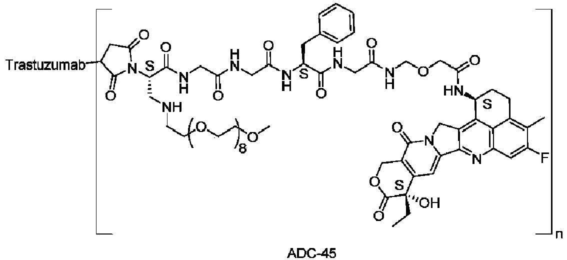

ADC-45 was prepared according to the general coupling method.

### Example 93: ADC-46

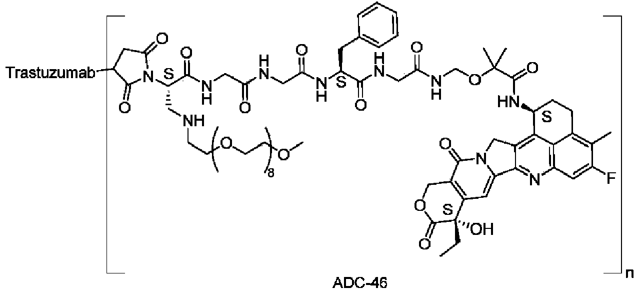

ADC-46 was prepared according to the general coupling method.

### Example 94: ADC-47

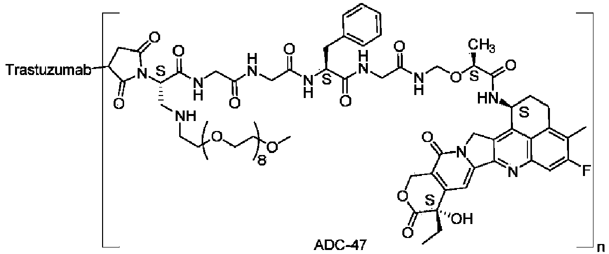

ADC-47 was prepared according to the general coupling method.

### Example 95: ADC-48

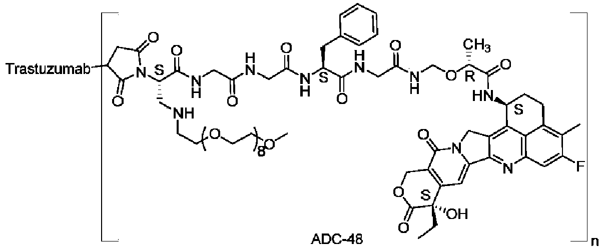

ADC-48 was prepared according to the general coupling method.

### Example 96: ADC-49

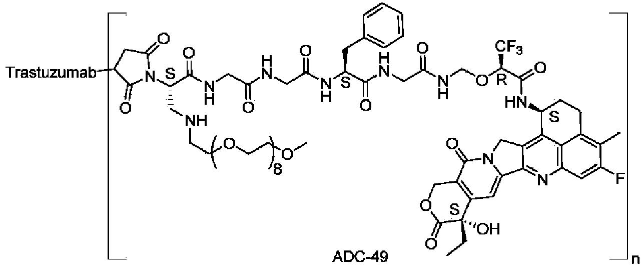

ADC-49 was prepared according to the general coupling method.

### Example 97: ADC-50

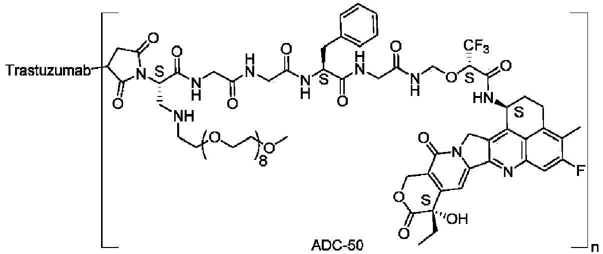

ADC-50 was prepared according to the general coupling method.

### Example 98: ADC-51

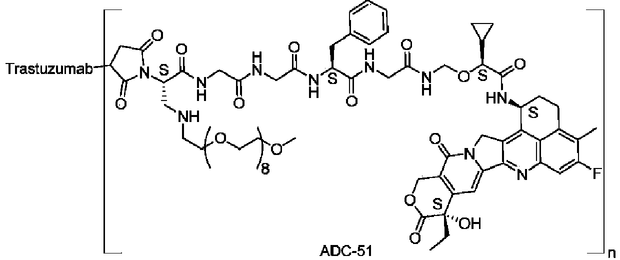

ADC-51 was prepared according to the general coupling method.

### Example 99: ADC-52

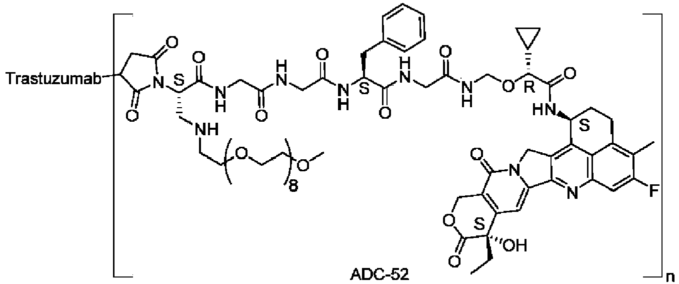

ADC-52 was prepared according to the general coupling method.

### Example 100: ADC-53

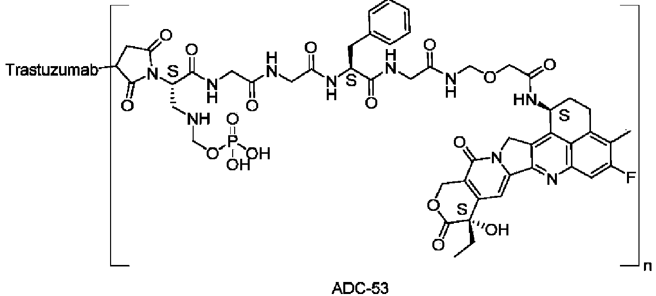

ADC-53 was prepared according to the general coupling method.

### Example 101: ADC-54

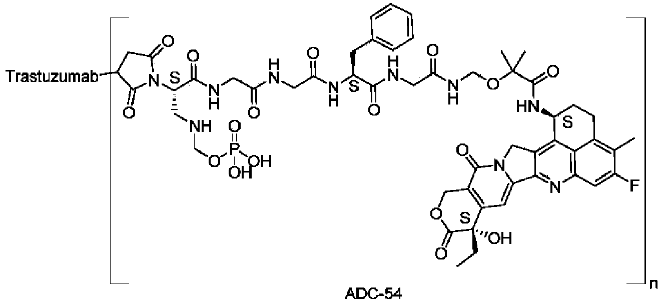

ADC-54 was prepared according to the general coupling method.

### Example 102: ADC-55

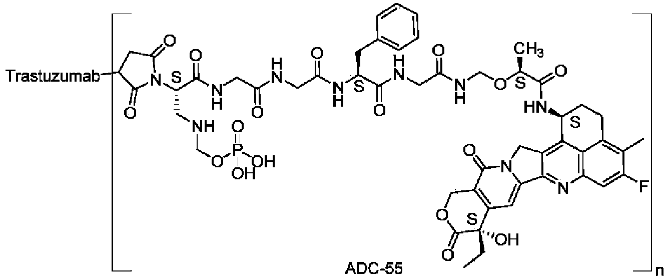

ADC-55 was prepared according to the general coupling method.

### Example 103: ADC-56

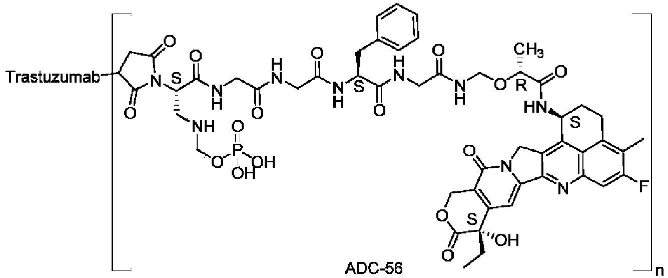

ADC-56 was prepared according to the general coupling method.

### Example 104: ADC-57

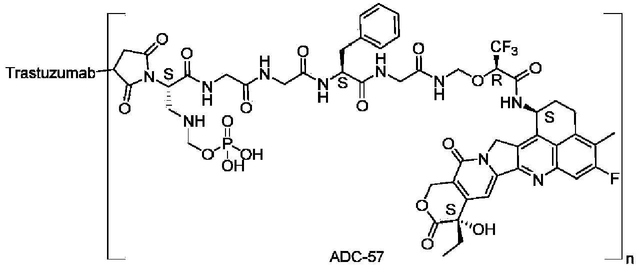

ADC-57 was prepared according to the general coupling method.

### Example 105: ADC-58

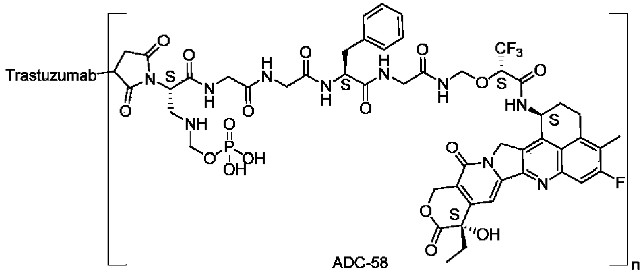

ADC-58 was prepared according to the general coupling method.

### Example 106: ADC-59

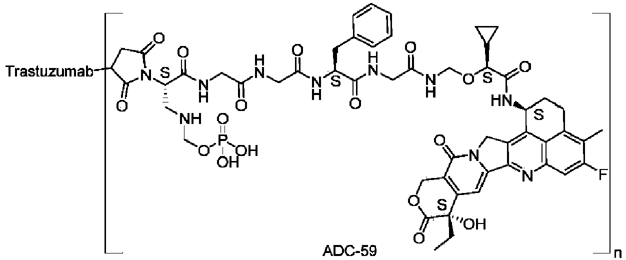

ADC-59 was prepared according to the general coupling method.

### Example 107: ADC-60

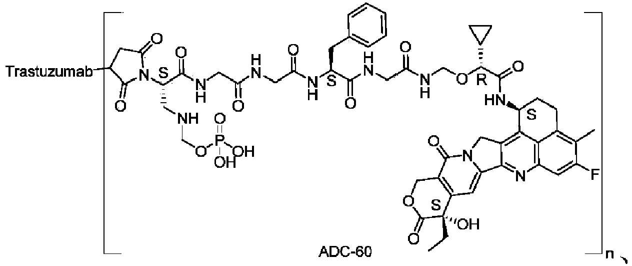

ADC-60 was prepared according to the general coupling method.

### Example 108: ADC-61 (control group)

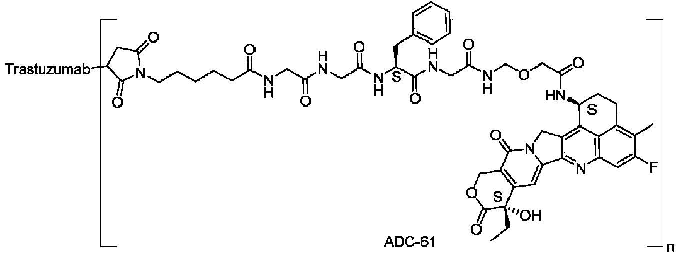

ADC-61 was prepared according to the general coupling method.

### Example 109: Plasma stability

1) Operation

Take a certain amount of ADC samples and add them to human plasma from which human IgG has been removed. Repeat three tubes of each ADC and place them in a 37°C water bath. After incubating for 72h and 144h respectively, take out the ADC samples and add to each tube 100uL ProteinA resin (MabSelect SuRe^{™} LX Lot:#10221479GE and washed with PBS), shaken in a vertical mixer and adsorbing for 2h, washing and elution to obtain the ADC samples. The ADC samples incubated for a specific time were measured by RP-HPLC.

2) Results

**Table 1. Ligand-drug conjugate (ADC) DAR value and monomer rate data of the disclosed ligand-drug conjugate (ADC), in one embodiment**

| Molecular name | DAR | Aggregates% | monomer% |
|---|---|---|---|
| Trastuzumab | NA | 1.61 | 98.39 |
| ADC-2 | 7.67 | 1.51 | 98.49 |
| ADC-6 | 7.55 | 1.61 | 98.39 |
| ADC-10 | 7.66 | 1.45 | 98.55 |
| ADC-12 | 7.64 | 2.28 | 97.72 |
| ADC-15 | 7.63 | 1.44 | 98.56 |
| ADC-20 | 7.60 | 1.40 | 98.60 |
| ADC-29 | 7.66 | 1.62 | 98.38 |
| ADC-35 | 7.59 | 1.67 | 98.33 |
| ADC-36 | 7.68 | 1.38 | 98.62 |
| ADC-41 | 7.64 | 1.51 | 98.49 |
| ADC-48 | 7.67 | 1.77 | 98.23 |
| ADC-52 | 7.58 | 1.61 | 98.39 |
| ADC-56 | 7.60 | 1.61 | 98.39 |
| ADC-61 (control) | 7.59 | 8.21 | 91.79 |

**Table 2. Plasma stability data of the ligand-drug conjugate (ADC) disclosed in one embodiment.**

| Molecules | DAR | | | |
|---|---|---|---|---|
| | Unincubated | Incubate 0 day | Incubate 3 days | Incubate 7 days |
| ADC-2 | 7.67 | 7.51 | 6.77 | 6.43 |
| ADC-6 | 7.55 | 7.52 | 7.46 | 7.44 |
| ADC-10 | 7.66 | 7.48 | 6.43 | 6.21 |
| ADC-12 | 7.64 | 7.43 | 6.73 | 6.11 |
| ADC-29 | 7.66 | 7.60 | 6.91 | 6.65 |
| ADC-36 | 7.68 | 7.64 | 7.02 | 6.96 |
| ADC-48 | 7.67 | 7.65 | 7.63 | 7.48 |
| ADC-56 | 7.60 | 7.49 | 7.08 | 6.33 |
| ADC-61 (control) | 7.59 | 7.48 | 5.31 | 5.02 |

3) Conclusion

As shown in Table 1, the camptothecin ADCs with highly stable hydrophilic linking units disclosed in the disclosure have excellent properties of high DAR value (>7.5) and high monomer ratio (>97%), compared to the control ADC-61 has a significantly higher monomer rate.

As shown in Table 2, after 7 days of incubation in the ADC plasma of the disclosure, the DAR value can still maintain a higher level compared to the control ADC-61, which proves that the ADC of the disclosure has excellent stability in plasma.

### Example 110: In vitro activity test

### 1) Experimental materials

Cells: from the cell bank of the Chinese Academy of Sciences;
Tumor cell culture medium: Gibco;
FBS: BIOWEST;

### 2) Preparation of medium

Growth medium (with 10% FBS, Penicillin/streptomycin (100U/mL);
Detection medium (with 1% FBS, Penicillin/streptomycin (100U/mL);

### 3) Operation

Turn on the UV lamp in the biological safety cabinet 30 minutes in advance, and ventilate for 3 minutes. Put the growth medium, detection medium, D-PBS and pancreatin into a 37°C constant temperature water bath to preheat, then disinfect the surface with alcohol and put it in a biological safety cabinet. Select cells with a confluence of ^{~}80% (logarithmic growth phase), put them in a biological safety cabinet, aspirate the old medium, rinse with D-PBS, aspirate and discard, digest with trypsin for 2 to 3 minutes, and then add to growth Stop trypsin in the medium, and centrifuge at 500×g for 5 min. Aspirate the centrifugal supernatant, mix well with 4mL detection medium, take 100uL for counting (take out 50uL cell fluid, add 50µL 0.4% Trypan Blue Stain and mix well, and count after mixing). Plate the plate according to the number of cells set before, and plate 80uL/well in a 96-well plate. Only add 80uL detection medium to wells E11, F11, and G11, and add 200uL DPBS to the edge holes to seal the edges. After the plated cells are completely attached to the wall (usually at least 4 hours), prepare and dilute the test sample: prepare 1.0mL, 2.5µM (5×Top Dose) test sample with the detection medium, and aliquot it in V Type 96-well plate in the first column, 200µL per well; add 180µL of detection medium from the second to the eighth column, take 30µL from the first column and add to the second column, mix up and down 10 times with a row gun, discard the pipette tip , The remaining detection concentration points are operated in sequence, and a 7-fold gradient concentration dilution is performed. Add the test sample of gradient concentration to the cells in the amount of 20uL per well. At the same time, add only 20uL of detection medium in the 11th column, set 3 replicate wells for each concentration, and then put the 96-well plate into 5% CO₂, 37°C cell incubator, culture for 5 days.

### 4) Detection

Take out the MTS reagent after the test sample is exposed for 5 days. After thawing at room temperature and avoiding light, vortex and mix thoroughly. In a biological safety cabinet, add 20 µL Cell Titer One Solution Reagen MTS reagent for every 100 µL cell culture volume along the side wall of the well. Gently tap the surface of the plate to mix the MTS solution evenly, and place it in a cell incubator with 5% CO₂, and incubate at 37°C in the dark for 2 hours. After the reaction, the 96-well plate was taken out, the OD490nm absorbance value was detected in the microplate reader, and the data was recorded, sorted, and stored.

### 5) Results

**Table 3: The IC50 value of the in vitro proliferation inhibition of N87 tumor cells by antibody-drug conjugates and toxins.**

| Sample | Trastuzumab | ADC-61 (control) | ADC-12 | ADC-6 | ADC-10 | ADC-2 | d₃ | d₆ | d₁ |
|---|---|---|---|---|---|---|---|---|---|
| IC50 (nM) | >500 | 2.992 | 0.565 | 0.622 | 0.907 | 1.075 | 8.025 | 2.147 | 13.344 |

**Table 4: IC50 value of the inhibition of the in vitro proliferation of SK-BR-3 tumor cells by antibody-drug conjugates and toxins.**

| Sample | Trastuzumab | ADC-61 (control) | ADC-12 | ADC-6 | ADC-10 | ADC-2 | d₃ | d₆ | d₁ |
|---|---|---|---|---|---|---|---|---|---|
| IC50 (nM) | >500 | >500 | 135.295 | 435.861 | 115.987 | 116.329 | 182.766 | 239.311 | 103.798 |

### 6) Discussion

As shown in Table 3, the ligand-drug conjugate of the disclosure for HER2 target has obvious in vitro proliferation inhibitory activity on HER2 positive cells N87, which is significantly better than naked antibody (Trastuzumab), control group ADC-61 and toxin single drug.

As shown in Table 4, compared with the naked antibody (Trastuzumab) and the control ADC, the ADC and the single agent disclosed in the disclosure also have obvious in vitro proliferation inhibitory activity on HER2-positive cells SK-BR-3.

### Example 111: In vivo activity test

### 1) Experimental materials

Cells: from the cell bank of the Chinese Academy of Sciences;
Tumor cell culture medium: Gibco;
Balb/c-nu nude mice: female, 5-7 weeks (the age of mice at the time of tumor cell inoculation), weighing 18.0-24.0 g, 170 (110 plus 60 surplus mice). Purchased from Beijing Weitong Lihua Laboratory Animal Technology Co., Ltd.;
Test substance and reference substance:
   Test products: ADC-61 and ADC-6 were provided by Chengdu Dote Antibody Drug Co., Ltd.
   Histidine buffer, provided by Chengdu Dote Antibody Pharmaceutical Co., Ltd.
   0.9% Sodium Chloride Injection: Kelun Pharmaceutical Co., Ltd.

### 2) Cell culture

NCI-H1975 (human non-small cell lung cancer adenocarcinoma cells) were cultured in RPMI1640 medium. NCI-H1975 cells in the exponential growth phase were collected and resuspended in RPMI1640 medium to a suitable concentration for subcutaneous tumor inoculation in mice.

NCI-N87 (human gastric cancer cells) were cultured in RPMI1640 medium. NCI-N87 cells in the exponential growth phase were collected, and RPMI1640 medium was resuspended to a suitable concentration for subcutaneous tumor inoculation in mice.

### 3) Animal modeling and random grouping

85 female nude mice were inoculated subcutaneously on the right shoulder with 5×10⁷ NCI-H1975 cells. When the average tumor volume is about 170mm³ , they are randomly grouped according to the tumor size. Fifty-five tumor-bearing mice with appropriate tumor volume were selected and randomly divided into groups and the administration was started (tail vein injection, the administration volume was 0.1ml/10g). The grouping day is defined as day 0.

85 female nude mice were inoculated subcutaneously on the right shoulder with 5×10⁷ NCI-N87 cells. When the average tumor volume is 170mm³, they are randomly grouped according to the tumor size. Fifty-five tumor-bearing mice with appropriate tumor volume were selected and randomly divided into groups and the administration was started (tail vein injection, the administration volume was 0.1ml/10g). The grouping day is defined as day 0.

### 4) Preparation of test substance and reference substance

**Table 5. Preparation of test and reference solutions for anti-tumor effects in NCI-H1975 (human non-small cell lung cancer adenocarcinoma cells) and NCI-N87 (human gastric cancer cells) subcutaneously transplanted tumor models in nude mice.**

| **Test Solution/Reference** | **Dosage (mg/kg)** | **Concentration (mg/ml)** | **Preparation** | **State after Preparation** | **Preparation Frequency** | **Storage post Preparation** |
|---|---|---|---|---|---|---|
| Vehicle | - | - | 1200uL Histidine buffer | solution | Prepare on spot | 4°C |
| | | | | □suspension | | |
| ADC-6 | 3.75 | 0.375 | 102uL ADC stock solution, plus 2298uL Histidine buffer, invert upside down to mix | solution | Prepare on spot | Discard after use |
| | | | | □ suspension | | |
| ADC-6 | 11.25 | 1.125 | 306uL ADC stock solution, plus 2094uL Histidine buffer, invert upside down to mix | solution | Prepare on spot | Discard after use |
| | | | | □ suspension | | |
| ADC-61 | 3.75 | 0.375 | 92uL ADC stock solution, plus 2308uL Histidine buffer, invert upside down to mix | □ solution | Prepare on spot | Discard after use |
| | | | | □ suspension | | |
| ADC-61 (control) | 11.25 | 1.125 | 277uL ADC stock solution, plus 2123uL Histidine buffer, invert upside down to mix | solution | Prepare on spot | Discard after use |
| | | | | □ suspension | | |
| Trastuzumab | 11.25 | 1.125 | 277uL ADC stock solution, plus 2123uL Histidine buffer, invert upside down to mix | solution | Prepare on spot | Discard after use |
| | | | | □suspension | | |

| | | | | | | |
|---|---|---|---|---|---|---|
| Note: Mix well before use to ensure that the preparation is uniform. | | | | | | |

### 5) Experimental observation and data collection

In the course of this experiment, the animal experiment operation was in accordance with the requirements of the standard operating procedures for the in vivo screening of anti-tumor drugs. After tumor inoculation, routine monitoring includes tumor growth (the tumor is measured twice a week) and the effect of treatment on the normal behavior of the animal. The specific content includes the activity of the experimental animal, food and drinking status, weight gain or loss (weight is measured weekly 2 times), eyes, coat and other abnormal conditions. The clinical symptoms observed during the experiment were recorded in the original data. Tumor volume calculation formula: tumor volume (mm³)=1/2× (a×b²) (where a represents the long diameter and b represents the short diameter). Manually recorded data was used in the experiment, including the measurement of the length and short diameter of the tumor and the weighing of the animal's weight.

### 6) Efficacy evaluation criteria

The relative tumor proliferation rate, T/C%, is the percentage value of the treatment group and the control group relative to the tumor volume or tumor weight at a certain point in time. Calculated as follows:
T/C%=TRTV/CRTV×100% (TRTV: average RTV in the treatment group; CRTV: average RTV in the vehicle control group; RTV=Vt/V0, V0 is the tumor volume of the animal at the time of grouping, and Vt is the animal's tumor volume after treatment Tumor volume); or T/C%=TTW/CTW×100% (TTW: average tumor weight at the end of the experiment in the treatment group; CTW: average tumor weight at the end of the experiment in the vehicle control group).

The relative tumor inhibition rate, TGI(%), is calculated as follows: TGI%=(1-T/C)×100%. [T and C are the relative tumor volume (RTV) or tumor weight (TW) of the treatment group and the control group at a specific time point, respectively].

### 7) Results

**Table 6: The in vivo efficacy of administration of antibody-drug conjugates on NCI-H1975 xenograft tumors.**

| **NCI-H1975 Group\Average Tumor Volume(mm³)** | **D0** | **D3** | **D7** | **D10** | **D14** | **D17** | **D21** | **D24** | **D28** | **D31** |
|---|---|---|---|---|---|---|---|---|---|---|
| Vehicle | 171.98 | 306.67 | 682.07 | 955.08 | 1484.48 | 1926.35 | 2086.61 | 2283.27 | 1908.57 | 2057.03 |
| Trastuzumab | 172.19 | 296.69 | 627.87 | 887.25 | 1094.88 | 1458.59 | 1659.49 | 1893.14 | 1795.55 | 1900.54 |
| ADC-6 (3.75mg/kg) | 177.23 | 287.29 | 456.33 | 461.82 | 429.12 | 427.21 | 434.03 | 466.98 | 342.13 | 318.87 |
| ADC-6 (11.25 mg/kg) | 176.38 | 302.16 | 281.28 | 127.32 | 49.21 | 35.49 | 20.87 | 21.05 | 8.57 | 6.35 |
| ADC-61 (3.75 mg/kg) | 175.70 | 310.96 | 524.82 | 590.38 | 691.41 | 801.68 | 741.46 | 805.82 | 744.25 | 590.86 |
| ADC-61 (11.25 mg/kg) | 176.93 | 258.15 | 322.19 | 226.12 | 208.91 | 232.64 | 198.01 | 232.77 | 150.44 | 163.34 |

**Table 7: The in vivo efficacy of administration of antibody-drug conjugates on NCI-N87 xenografts.**

| **NCI-N87\ Group\Average Tumor Volume (mm³)** | **D0** | **D3** | **D7** | **D10** | **D14** | **D17** | **D21** | **D24** | **D28** | **D31** |
|---|---|---|---|---|---|---|---|---|---|---|
| Vehicle | 176.61 | 257.61 | 541.37 | 646.95 | 857.49 | 915.65 | 1118.54 | 1093.71 | 1396.93 | 1625.59 |
| Trastuzumab | 180.57 | 226.45 | 322.76 | 445.83 | 542.50 | 631.28 | 725.55 | 848.82 | 942.65 | 1004.93 |
| ADC-6 (3.75mg/kg) | 174.88 | 165.59 | 170.49 | 113.27 | 75.82 | 44.08 | 30.78 | 11.16 | 24.76 | 17.02 |
| ADC-61 (3.75 mg/kg) Control | 174.86 | 217.78 | 236.78 | 233.98 | 201.96 | 139.46 | 137.63 | 83.22 | 106.70 | 130.81 |

**Table 8: The effect of administration of antibody drug conjugate (11.25 mg/kg) on the body weight of NCI-H1975 transplanted tumor mice.**

| **NCI-H1975\Group\average weight (g)** | **D0** | **D3** | **D7** | **D10** | **D14** | **D17** | **D21** | **D24** | **D28** | **D31** |
|---|---|---|---|---|---|---|---|---|---|---|
| Vehicle | 19.83 | 20.52 | 20.13 | 19.79 | 20.42 | 19.83 | 19.57 | 20.45 | 18.21 | 19.79 |
| ADC-6 (11.25mg/kg) | 19.99 | 20.66 | 19.70 | 20.46 | 20.65 | 19.99 | 20.68 | 19.89 | 20.52 | 20.69 |
| ADC-61 (11.25 mg/kg) Control | 19.78 | 20.55 | 19.52 | 19.32 | 19.57^{∗} | 19.78^{∗} | 19.36^{∗} | 19.59^{∗} | 20.14* | 18.99* |

| | | | | | | | | | | |
|---|---|---|---|---|---|---|---|---|---|---|
| Note: *The death of two mice was observed in the identification group | | | | | | | | | | |

### 8) Discussion

As shown in Table 6, the ADC-6 disclosed herein in the low-dose control group (3.75mg/Kg) has significantly better in vivo efficacy on tumor-bearing mice NCI-H1975 than the control group ADC-61 and naked antibody; when the dose is increased to 11.25 mg/Kg, the therapeutic effect of ADC-6 disclosed herein is further improved and is significantly better than the control ADC-61.

As shown in Table 7, at the same dose (3.75mg/Kg), the ADC-6 disclosed herein has significantly better in vivo efficacy on tumor-bearing mice NCI-N87 than the control group ADC-61. Compared with the high-dose naked antibody (11.25mg) /Kg), the in vivo efficacy is more pronounced.

As shown in Table 8, the application discloses that the 11.25mg/Kg of ADC-6 in the high-dose control group has a significantly smaller effect on the body weight of NCI-H1975 tumor-bearing mice than ADC-61, even under the high-dose group. There was no death of mice as shown in the control group, which proves that the ADC drug disclosed herein has a significant advantage in terms of safety.

## Claims

1. A ligand-drug conjugate having a highly stable hydrophilic connecting unit as shown in Formula I, or its pharmaceutically acceptable salt thereof, 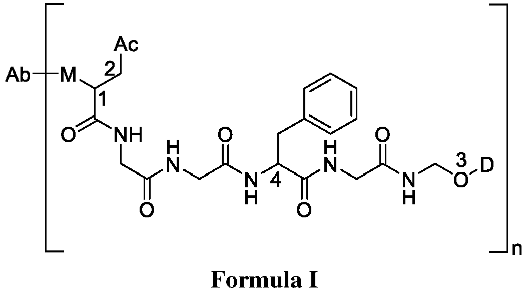 wherein:
Ab is a ligand unit, selected from antibodies, antibody fragments, targeting proteins, or Fc-fusion proteins;
M is a connecting unit connected with Ab;
Ac is a hydrophilic structural unit;
D is a camptothecin drug;
the position-1 carbon and position-4 chiral carbon each has the chirality of R or S configuration; and
n is selected from an integer of 1-20.

2. The ligand-drug conjugate or its pharmaceutically acceptable salt thereof according to claim 1, wherein the M has a succinimide structure represented by the Formula a, or an open-ringed succinimide structure represented by Formula b1 or b2, 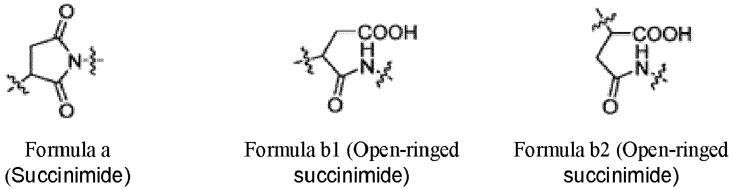 Wherein, in Formula a, Formula b1, or Formula b2, the wavy line on the left represents the connection to a connection site on Ab, and the wavy line on the right indicates the connection to the position-1 tertiary carbon in formula I.

3. The ligand-drug conjugate or its pharmaceutically acceptable salt thereof according to claim 1, wherein Ac has the structure shown in Formula c, 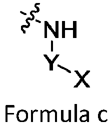
wherein X is selected from a group consisting of a hydrophilic carboxyl group, phosphoric acid group, polyphosphoric acid group, phosphorous acid group, sulfonic acid group, sulfinic acid group, and polyethylene glycol (PEG) group;
Y is a scaffold connecting the amino group and X; and
Ac is connected to the position-2 methylene carbon in formula I through the amino group.

4. The ligand-drug conjugate or its pharmaceutically acceptable salt thereof according to claim 1 or 3, wherein Ac is selected from Glycine, (D/L)-Alanine, (D/L)-Leucine, (D/L)-Isoleucine, (D/L)-Valine, (D/L)-Phenylalanine, (D/L)-Proline, (D/L)-Tryptophan, (D/L)-Serine, (D/L)-Tyrosine, (D/L)-Cysteine, (D/L)-Cystine, (D/L)-Arginine, (D/L)-Histidine, (D/L)-Methionine, (D/L)-Asparagine, (D/L)-Glutamine, (D/L)-Threonine, (D/L)-Aspartic acid, (D/L)-Glutamic acid, natural or unnatural amino acid derivatives or the following structures,

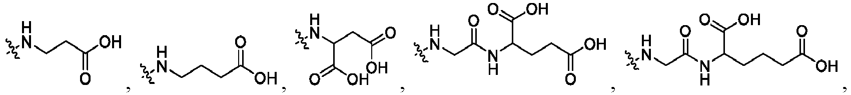

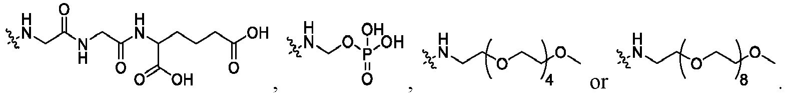

5. The ligand-drug conjugate or its pharmaceutically acceptable salt thereof according to claim 1, wherein the camptothecin drug has the structure shown in Formula d; 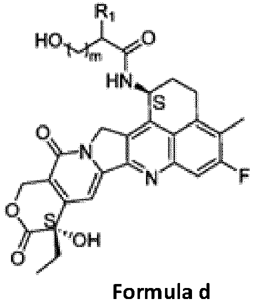
wherein R ₁ is selected from hydrogen atom, deuterium atom, halogen, alkyl, deuterated alkyl, haloalkyl, cycloalkyl, cycloalkylalkyl, alkoxyalkyl, heterocyclyl, aryl, substituted aryl, or heteroaryl; alternatively, R ₁ and its connected carbon atom form a C₃₋₆ cycloalkyl, cycloalkylalkyl or heterocyclic group;
the R1-connected chiral carbon atom has R or S configuration;
m is selected from 0 or 1; and
the hydroxyl group linked to the R1-connected chiral carbon in Formula d is configured to conjugate D to Ab as the position-3 oxygen atom in Formula I.

6. The ligand-drug conjugate or its pharmaceutically acceptable salt thereof according to claims 1 or 5, wherein the camptothecin drug is independently selected from the following compounds:

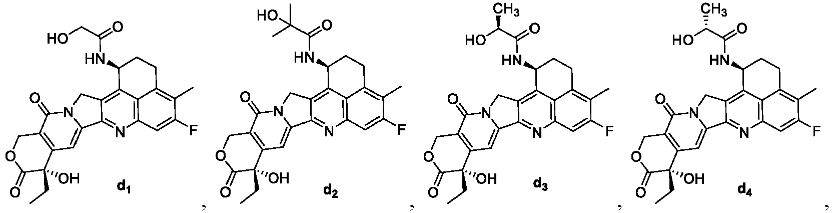

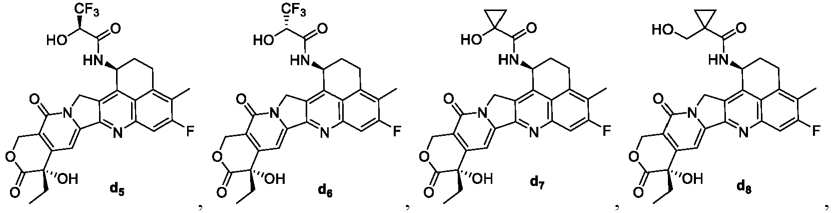

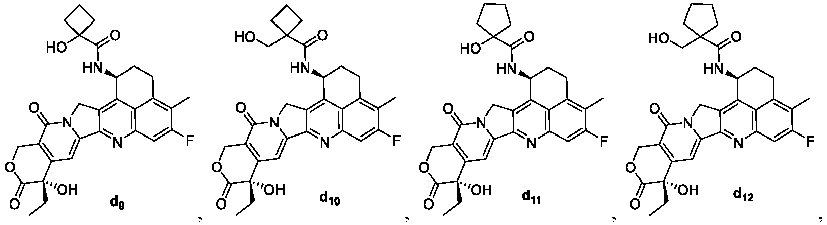

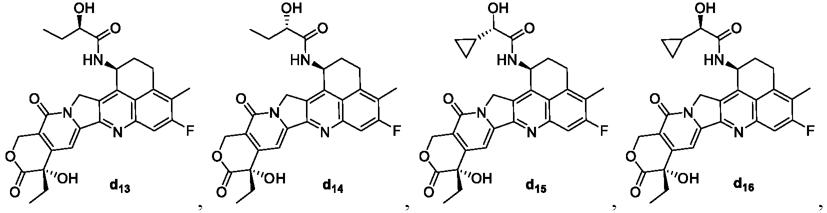

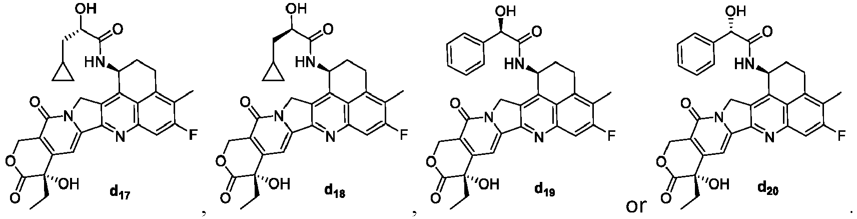

7. A linker-drug compound or a pharmaceutically acceptable salt thereof for coupling with the Ab ligand unit to form the ligand-drug conjugate of Formula I in claim 1, having the following structure shown in Formula II, 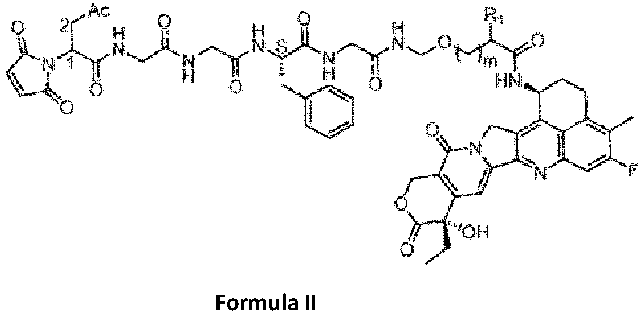
wherein R ₁ is selected from hydrogen atom, deuterium atom, halogen, alkyl, deuterated alkyl, haloalkyl, cycloalkyl, cycloalkylalkyl, alkoxyalkyl, heterocyclyl, aryl, substituted aryl, or heteroaryl; alternatively, the R ₁ and its connected carbon atom form a C₃₋₆ cycloalkyl, cycloalkylalkyl or heterocyclic group;
the position-1 chiral carbon has R or S configuration;
Ac is a hydrophilic structural unit; and
m is 0 or 1.

8. The linker-drug compound or its pharmaceutically acceptable salt thereof according to claim 7, wherein Ac is selected from glycine, phosphoric acid, (D/L)-glutamic acid, or polyethylene glycol Hydrophilic structure.

9. The linker-drug compound or its pharmaceutically acceptable salt thereof according to any one of claims 7 or 8, wherein the linker-drug compound is selected from the following structures, 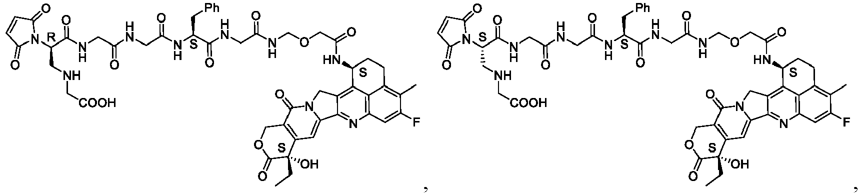 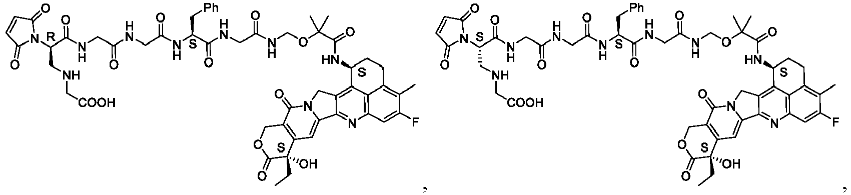 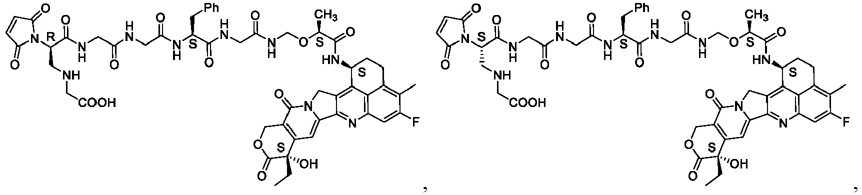 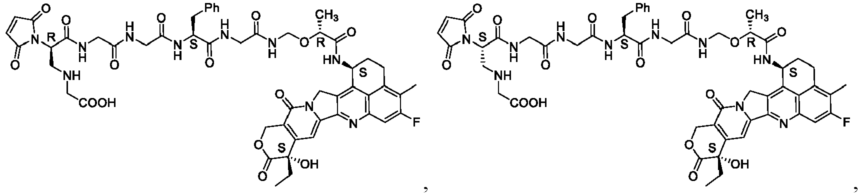 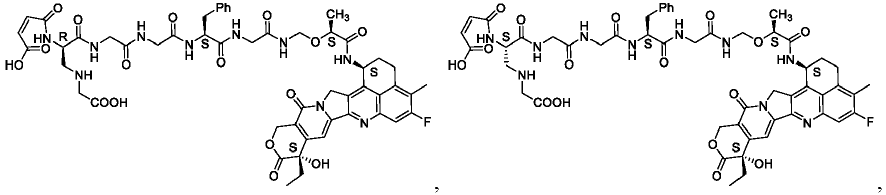 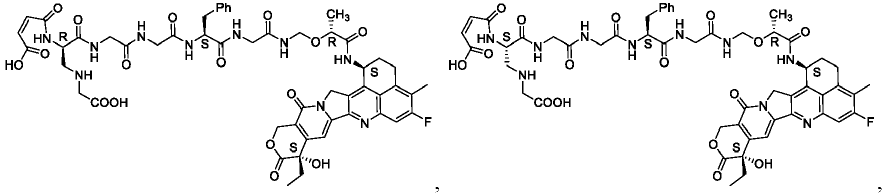 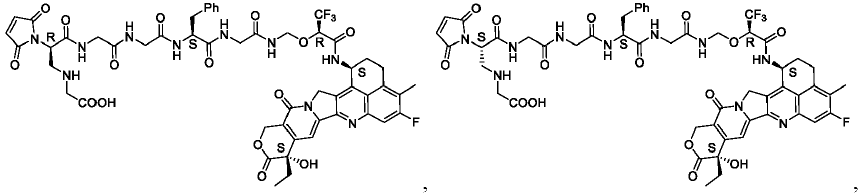 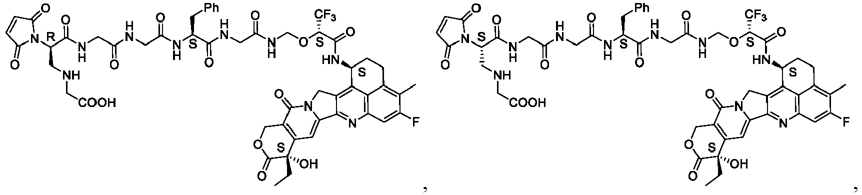 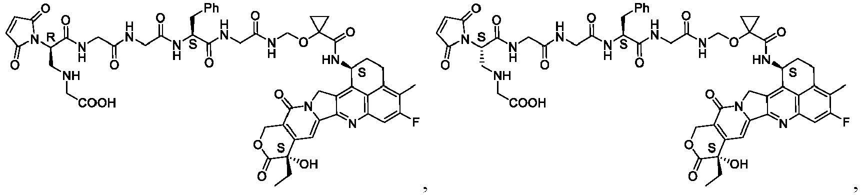 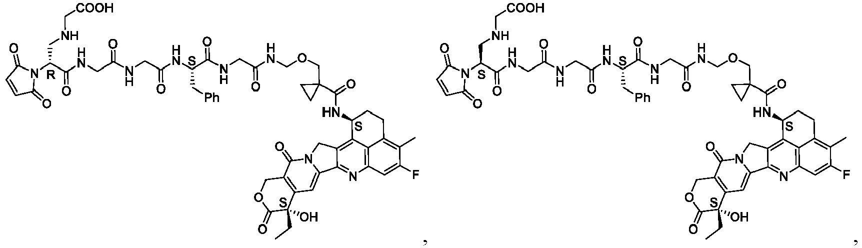 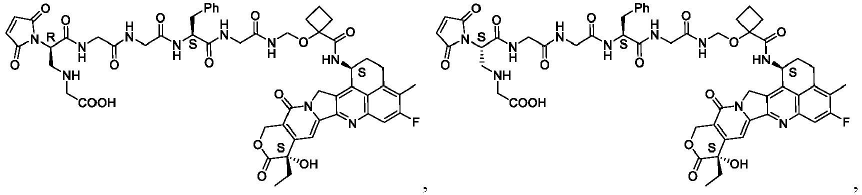 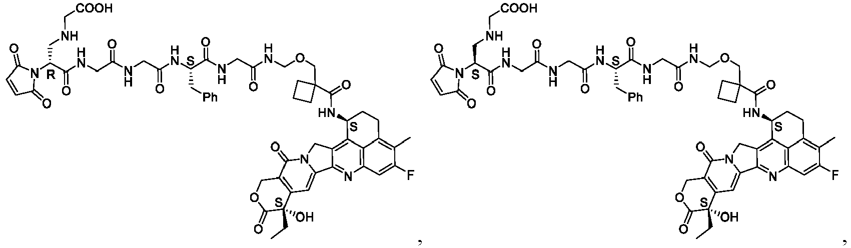 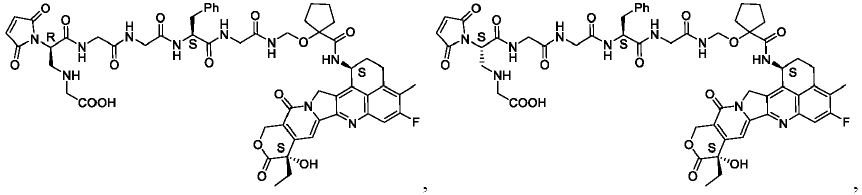 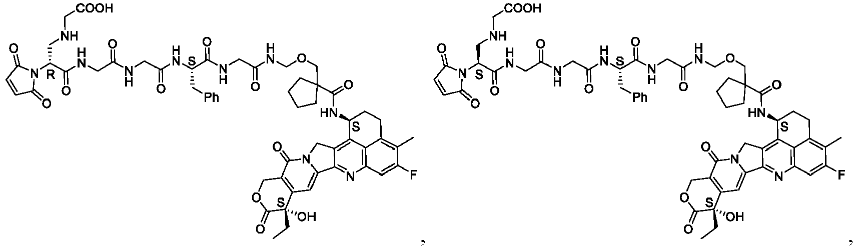 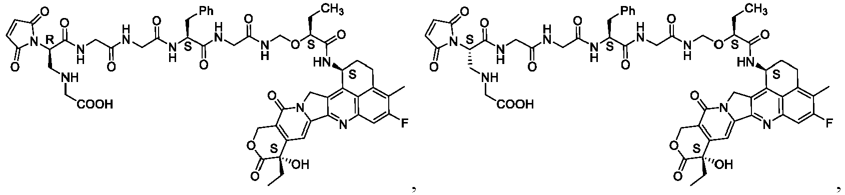 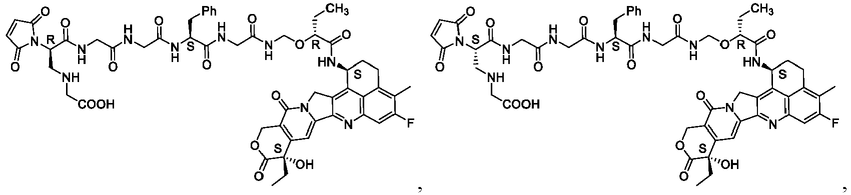 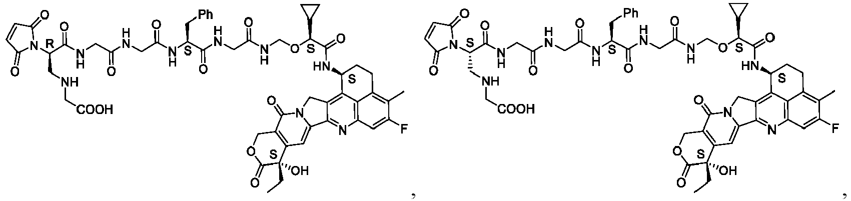 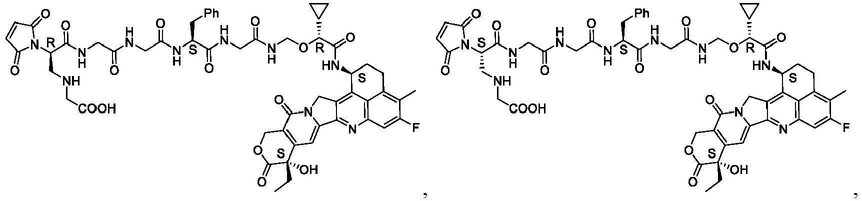 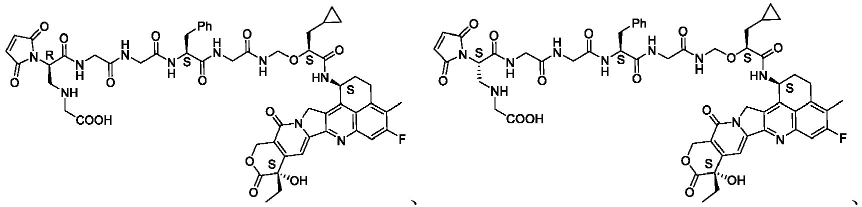 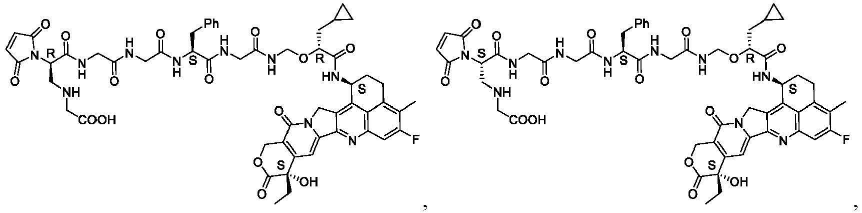 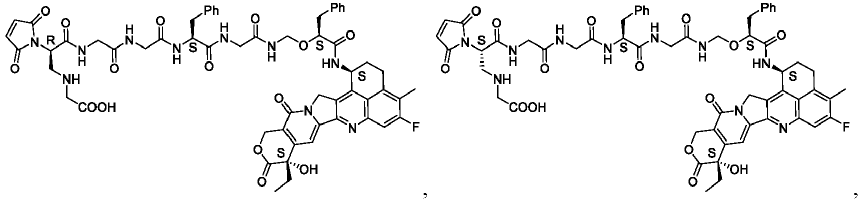 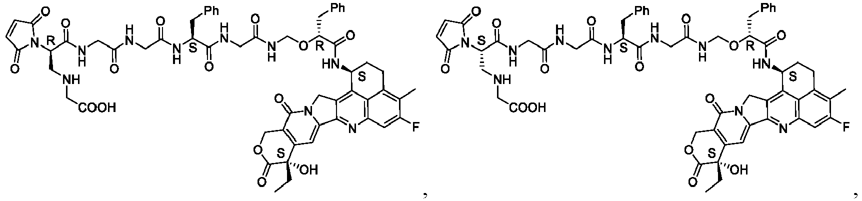 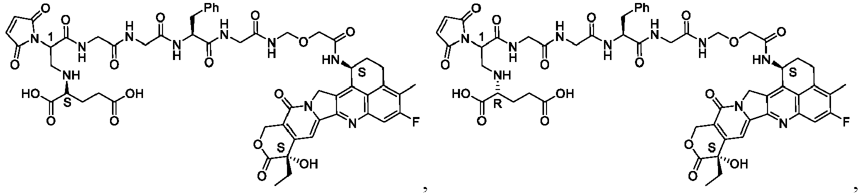 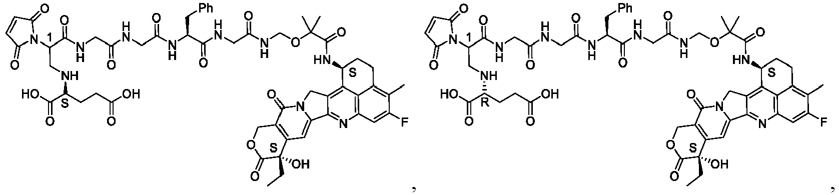 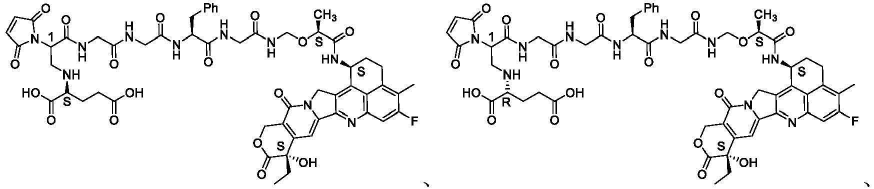 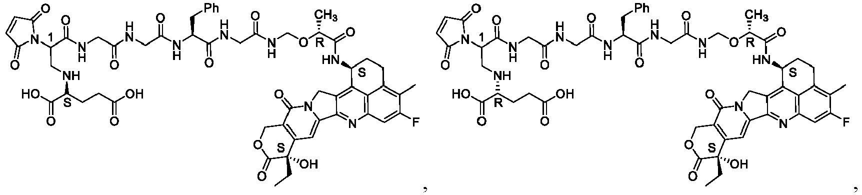 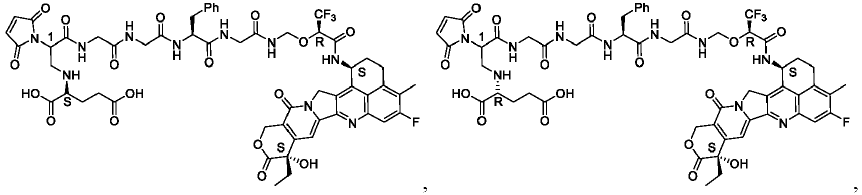 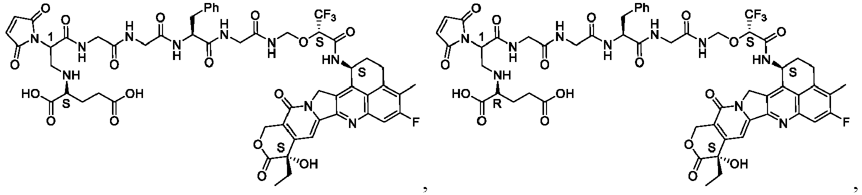 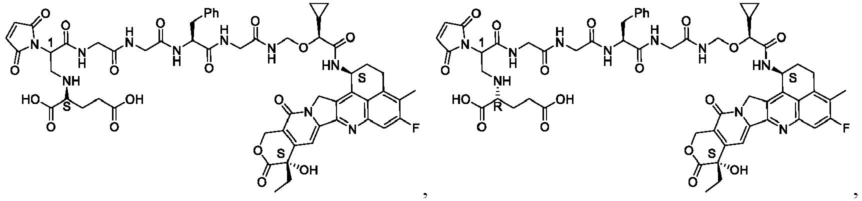 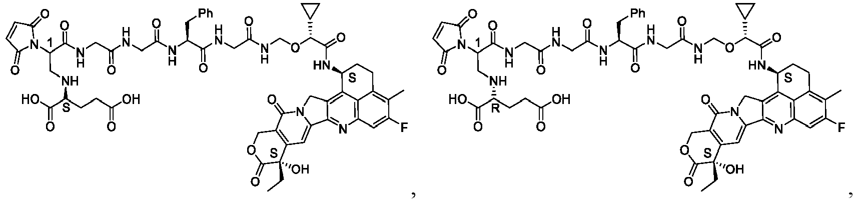 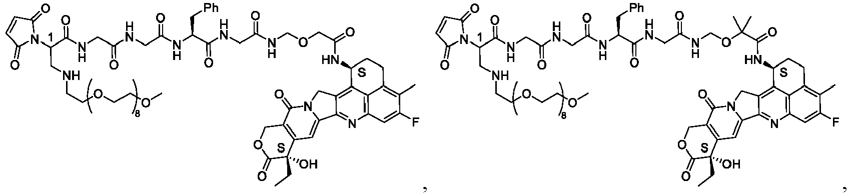 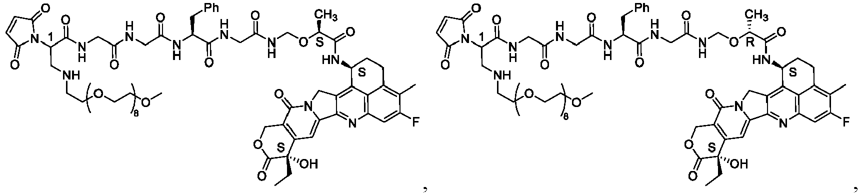 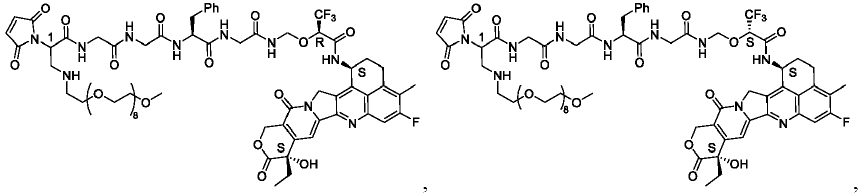 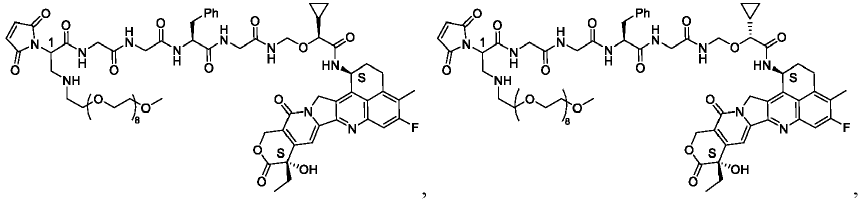 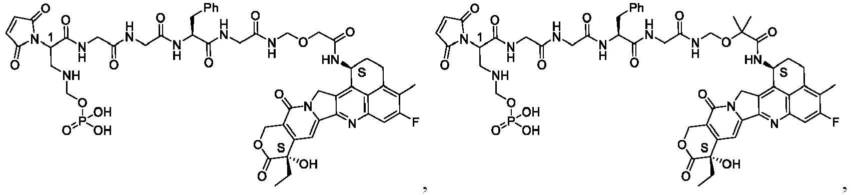 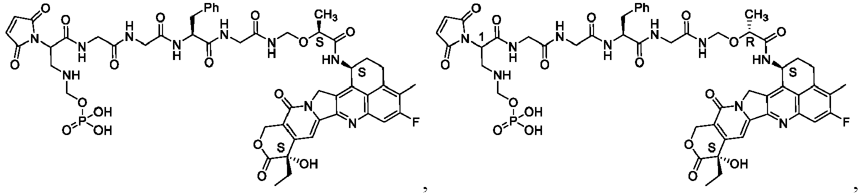 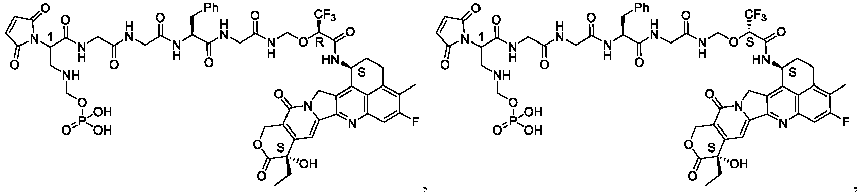 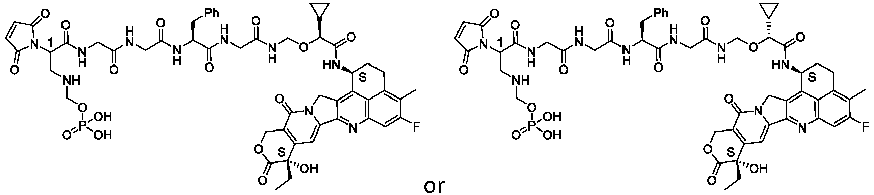 where the position-1 chiral carbon has the chirality of R or S configuration.

10. The ligand-drug conjugate or its pharmaceutically acceptable salt thereof according to any one of claims 1-9, wherein the ligand-drug conjugate or its pharmaceutically acceptable salt thereof has the structure shown in the following Formula III, Formula IV-1 or Formula IV-2, 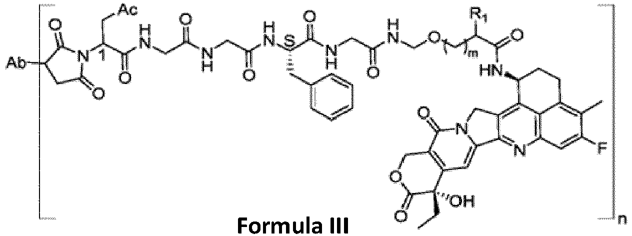 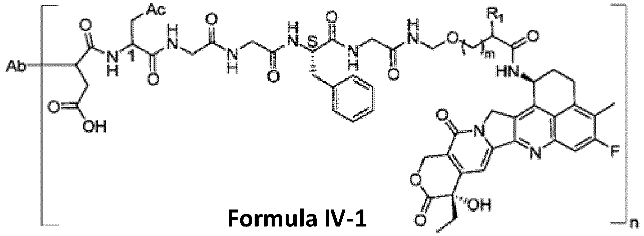 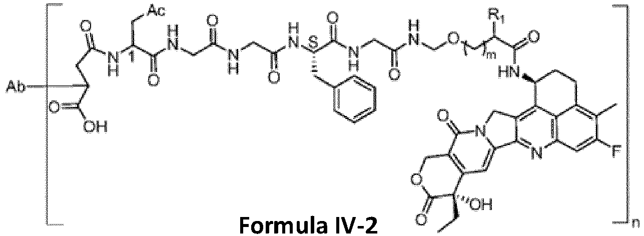 wherein
Ab is the ligand unit;
Ac is the hydrophilic structural unit;
the position-1 chiral carbon has a R or S configuration;
R₁, m and n are as described in Formula II.

11. The ligand-drug conjugate or its pharmaceutically acceptable salt thereof according to claim 10, wherein the ligand unit Ab is selected from an antibody, an antibody fragment, or a protein, wherein the antibody is selected from murine antibodies, rabbit antibodies, phage display antibodies, yeast display antibodies, chimeric antibodies, humanized antibodies, fully human antibodies, antibody fragments, bispecific antibodies, or multi-specific antibodies.

12. The antibody-drug conjugate comprising different drugs or its pharmaceutically acceptable salt thereof according to claim 10, wherein the antibody is a monoclonal antibody, and is selected from the group consisting of anti-EGFRvIII antibody, anti-PD-1 antibody, anti-PD-L1 antibody, anti-DLL-3 antibody, anti-PSMA antibody, anti-CD70 antibody, anti-MUC16 antibody, anti-ENPP3 antibody, anti-TDGF1 antibody, anti-ETBR antibody, anti-MSLN antibody, anti-TIM-1 antibody, Anti-LRRC15 antibody, anti-LIV-1 antibody, anti-CanAg/AFP antibody, anti-cladin 18.2 antibody, anti-Mesothelin antibody, anti-HER2 (ErbB2) antibody, anti-EGFR antibody, anti-c-MET antibody, anti-SLlTRK6 antibody, anti-KIT/CD117 Antibody, anti-STEAP1 antibody, anti-SLAMF7/CS1 antibody, anti-NaPi2B/SLC34A2 antibody, anti-GPNMB antibody, anti-HER3 (ErbB3) antibody, anti-MUC1/CD227 antibody, anti-AXL antibody, anti-CD166 antibody, anti-B7-H3 (CD276) Antibody, anti-PTK7/CCK4 antibody, anti-PRLR antibody, anti-EFNA4 antibody, anti-5T4 antibody, anti-NOTCH3 antibody, anti-Nectin 4 antibody, anti-TROP-2 antibody, anti-CD142 antibody, anti-CA6 antibody, anti-GPR20 antibody, anti-CD174 antibody , Anti-CD71 antibody, anti-EphA2 antibody, anti-LYPD3 antibody, anti-FGFR2 antibody, anti-FGFR3 antibody, anti-FRα antibody, anti-CEACAMs antibody, anti-GCC antibody, anti-Integrin Av antibody, anti-CAIX antibody, anti-P-cadherin antibody, anti-GD3 Antibody, anti-Cadherin 6 antibody, anti-LAMP1 antibody, anti-FLT3 antibody, anti-BCMA antibody, anti-CD79b antibody, anti-CD19 antibody, anti-CD33 antibody, anti-CD56 antibody, anti-CD74 antibody, anti-CD22 antibody, anti-CD30 antibody, anti-CD37 antibody, anti-CD47 antibody, anti-CD138 antibody, anti-CD352 antibody, anti-CD25 antibody, and anti-CD123 antibody.

13. The ligand-drug conjugate or its pharmaceutically acceptable salt thereof according to claim 10, wherein the antibody or antigen-binding fragment comprises Trastuzumab having
a light chain comprising: 
MDMRVPAQLLGLLLLWLRGARC
DIQMTQSPSSLSASVGDRVTITCRASQDVNTAVAWYQQKPGKAPKLLIYSASFLYSGVPSRFSGSRSG
TDFTLTISSLQPEDFATYYCQQHYTTPPTFGQGTKVEIK
RTVAAPSVFIFPPSDEQLKSGTASVVCLLNNFYPREAKVQWKVDNALQSGNSQESVTEQDSKDSTYS
LSSTLTLSKADYEKHKVYACEVTHQGLSSPVTKSFNRGEC* (SEQ ID NO: 1);
 and
a heavy chain comprising:

MDMRVPAQLLGLLLLWLRGARC
EVQLVESGGGLVQPGGSLRLSCAASGFNIKDTYIHWVRQAPGKGLEWVARIYPTNGYTRYADSVKGR
FTISADTSKNTAYLQMNSLRAEDTAVYYCSRWGGDGFYAMDYWGQGTLVTVSS
ASTKGPSVFPLAPSSKSTSGGTAALGCLVKDYFPEPVTVSWNSGALTSGVHTFPAVLQSSGLYSLSSVV
TVPSSSLGTQTYICNVNHKPSNTKVDKKVEPKSCDKTHTCPPCPAPELLGGPSVFLFPPKPKDTLMISRTPEVTC
VVVDVSHEDPEVKFNWYVDGVEVHNAKTKPREEQYNSTYRVVSVLTVLHQDWLNGKEYKCKVSNKALPAPI
EKTISKAKGQPREPQVYTLPPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPG (SEQ ID NO: 2).

14. The ligand-drug conjugate or its pharmaceutically acceptable salt thereof according to claim 10, wherein the ligand-drug conjugate or its pharmaceutically acceptable salt thereof is selected from the following succinimide structures or succinimide open-ring structures, 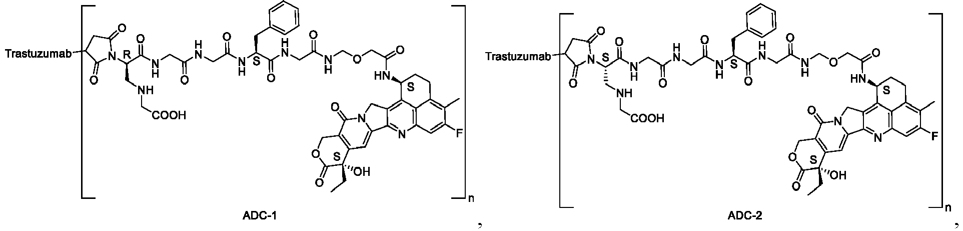 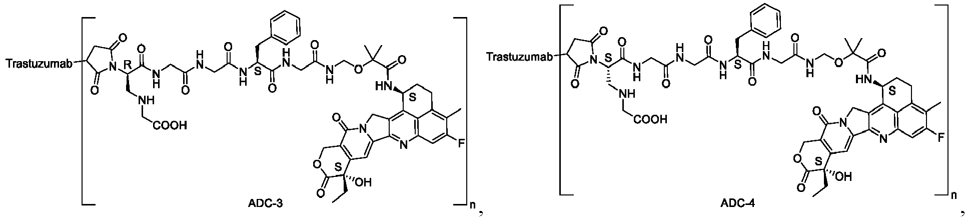 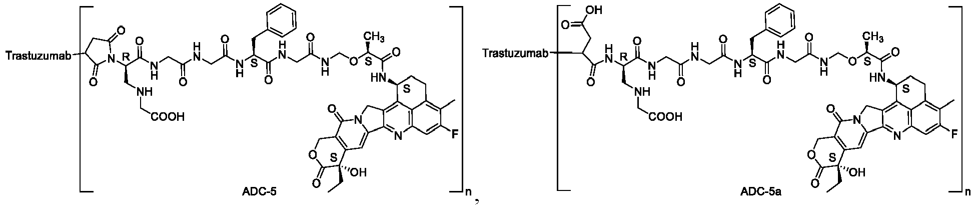 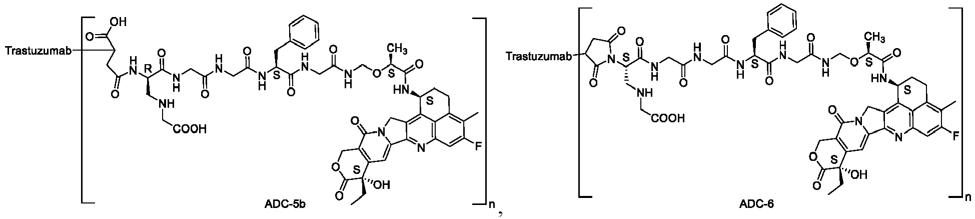 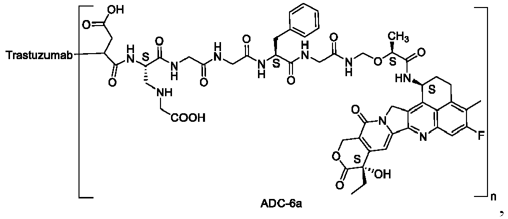 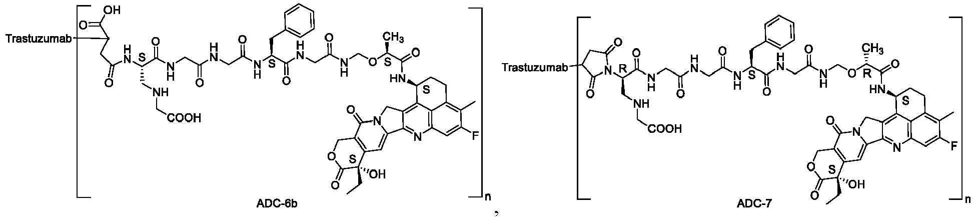 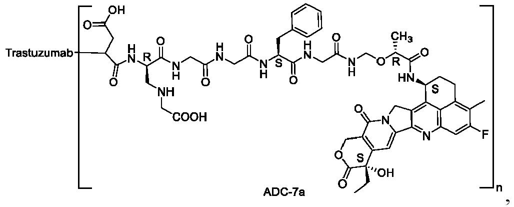 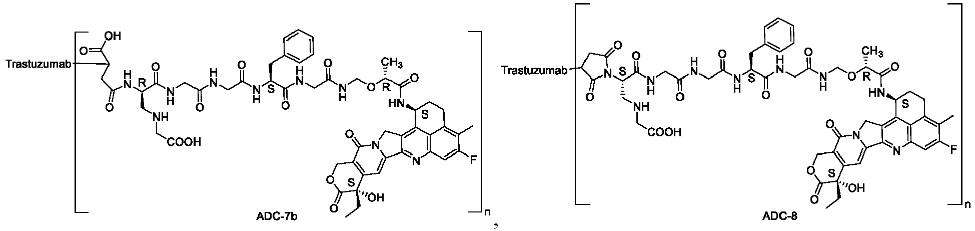 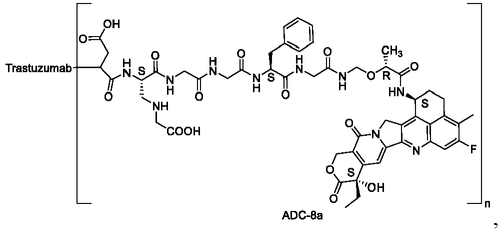 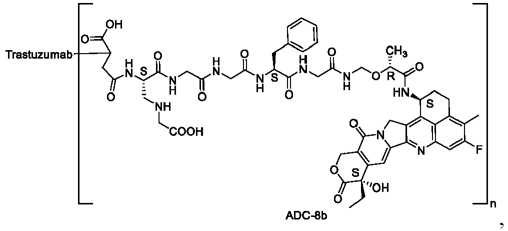 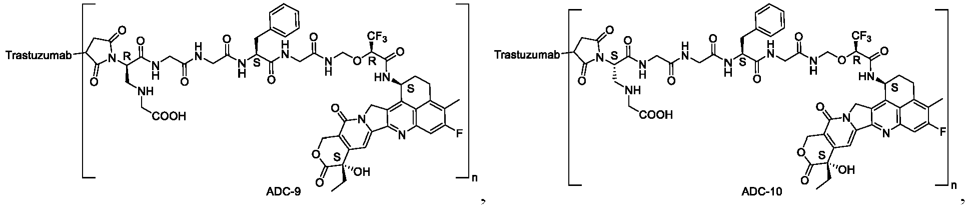 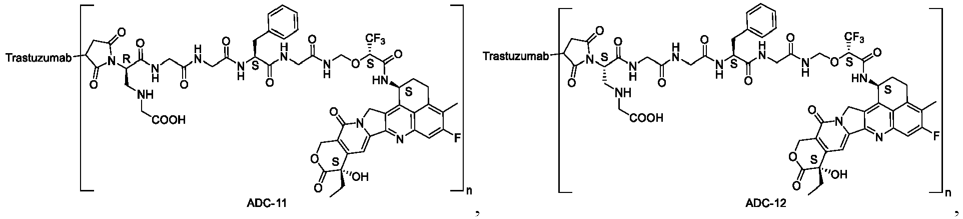 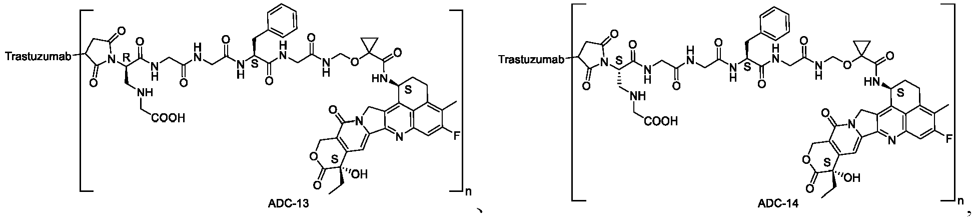 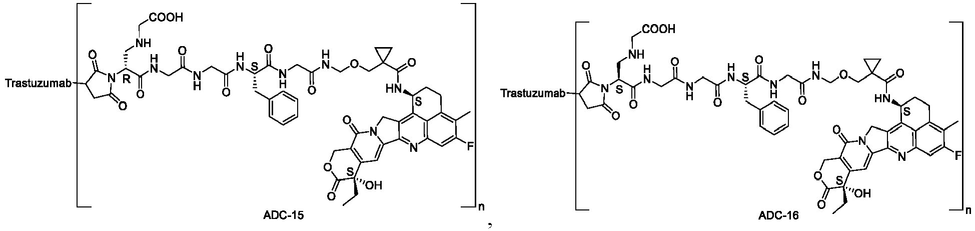 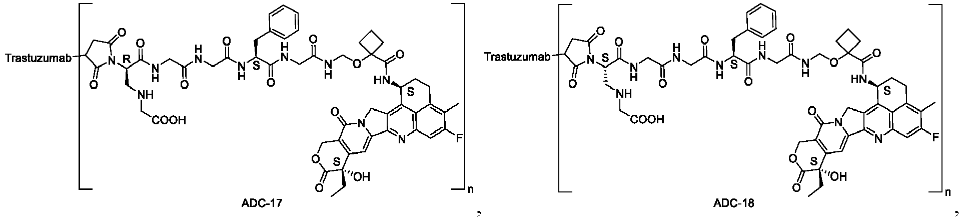 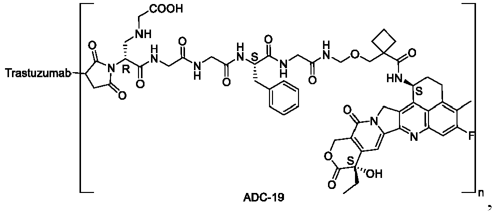 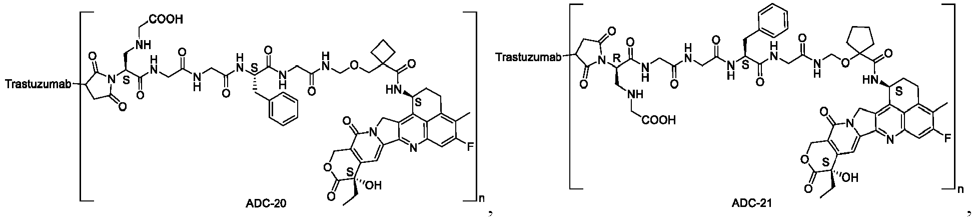 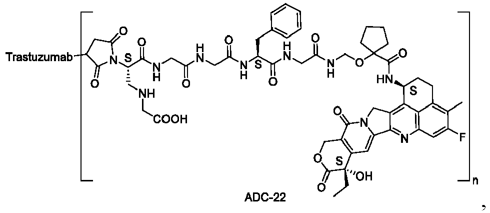 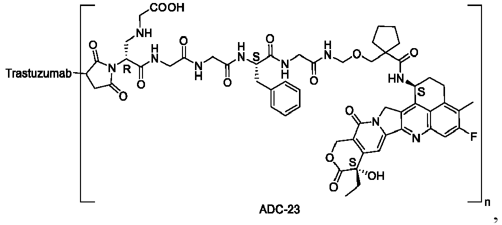 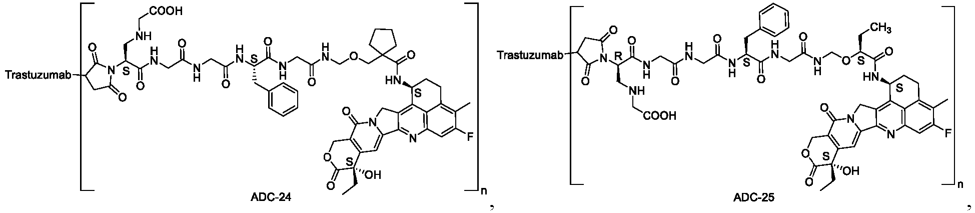 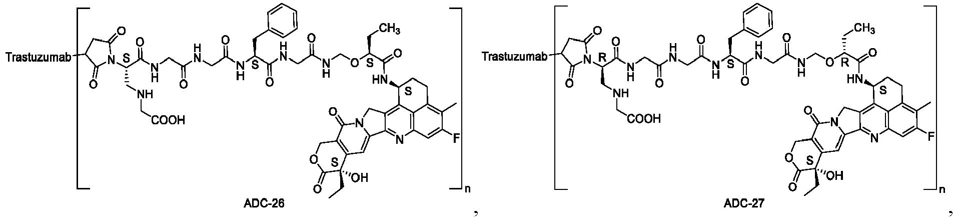 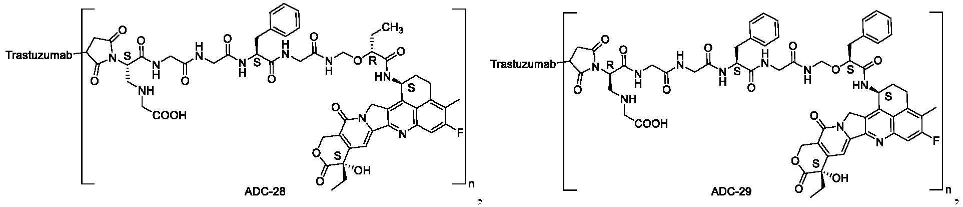 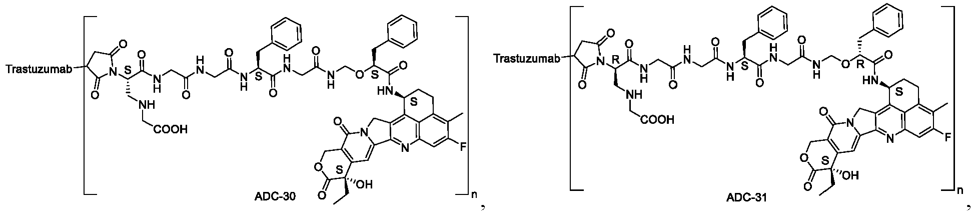 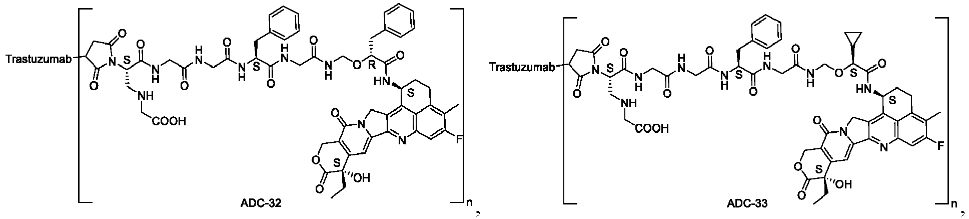 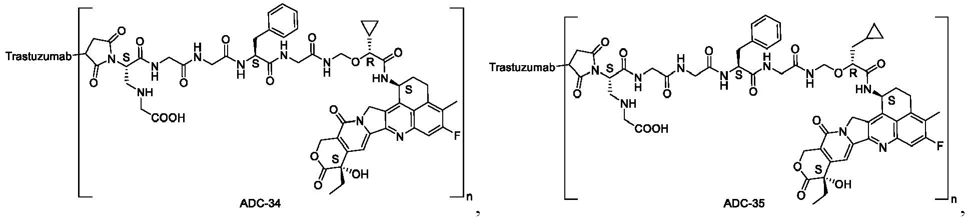 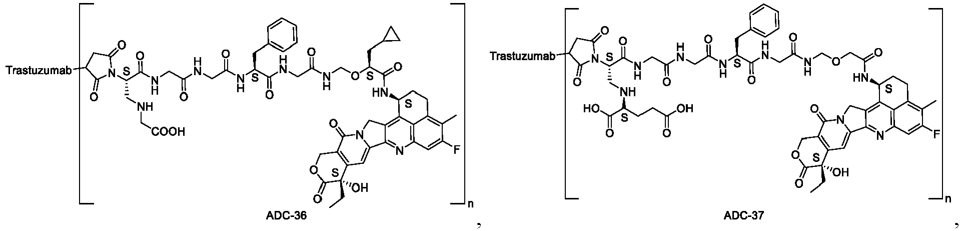 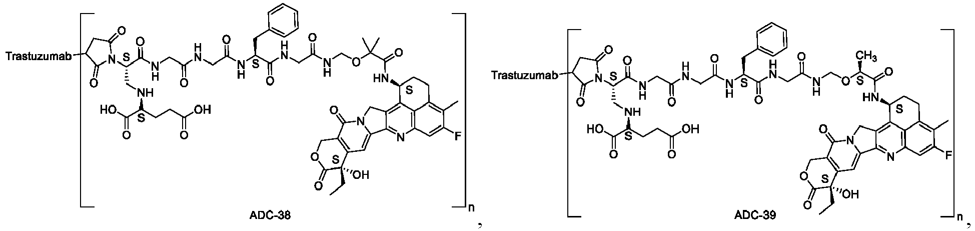 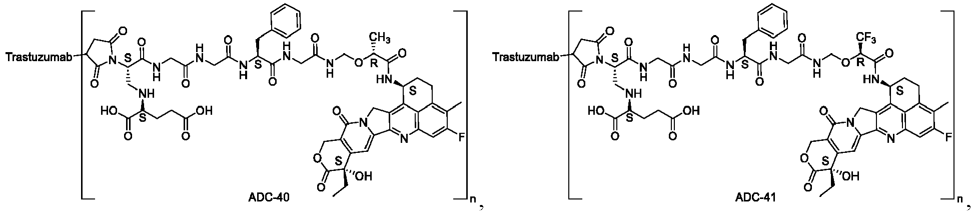 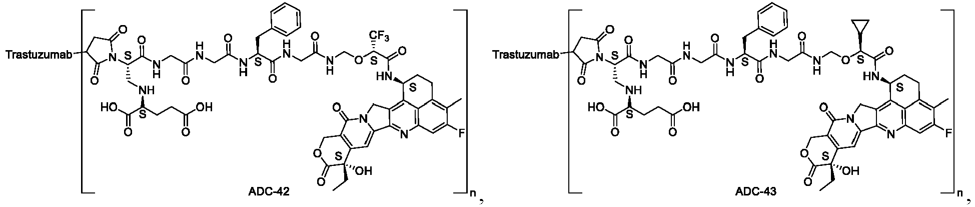 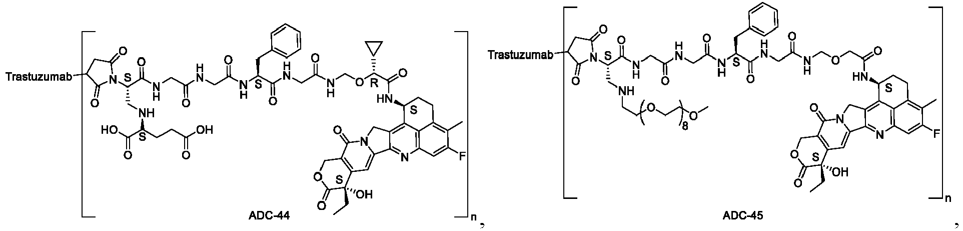 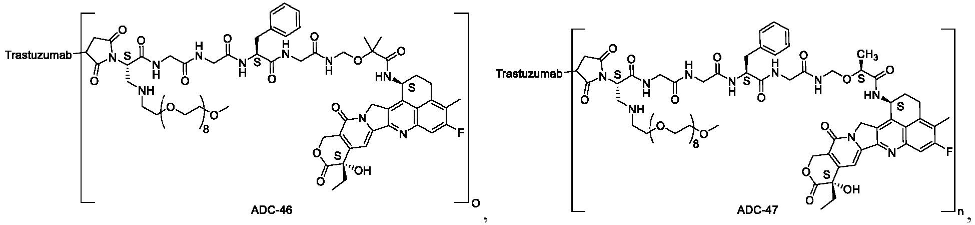 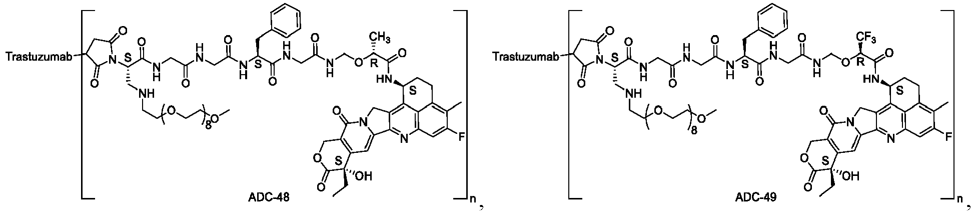 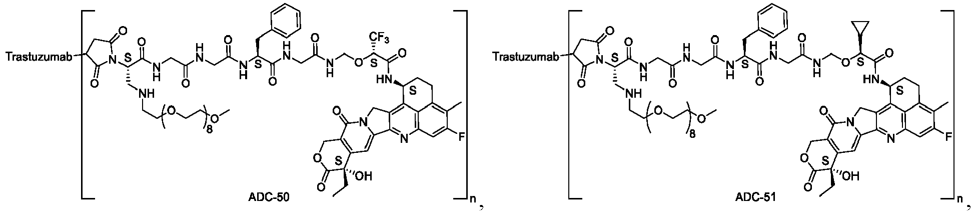 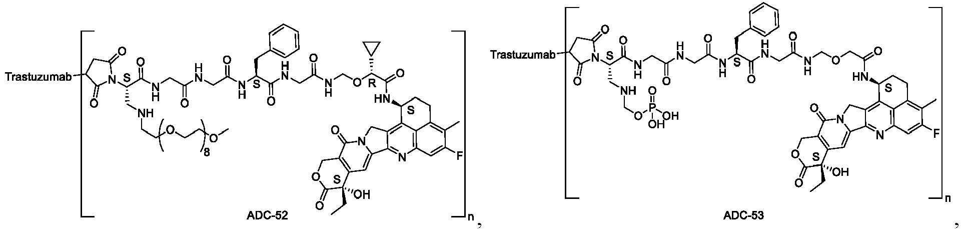 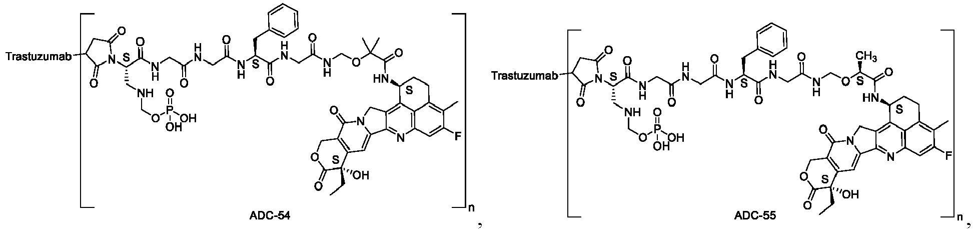 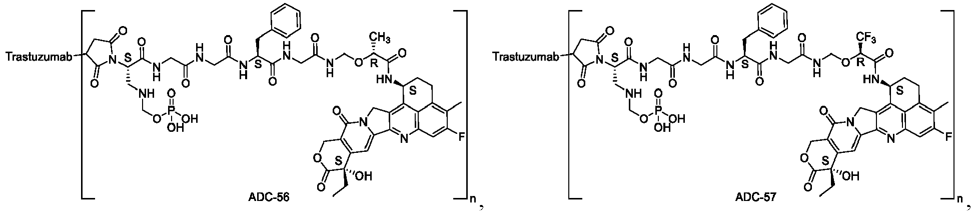 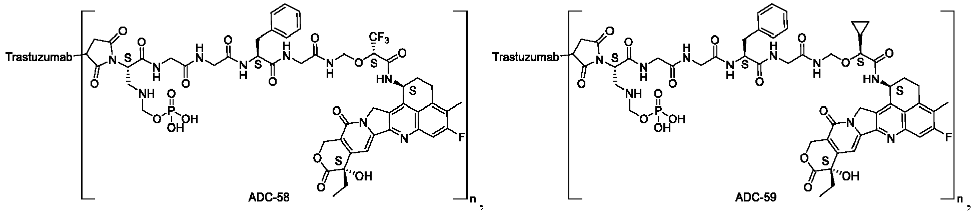 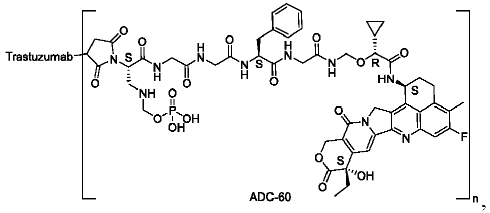 wherein n is selected from an integer of 1-10.

15. A method for preparing the linker-drug compound according to claims 7-9 or its pharmaceutically acceptable salt thereof, comprising the following steps: 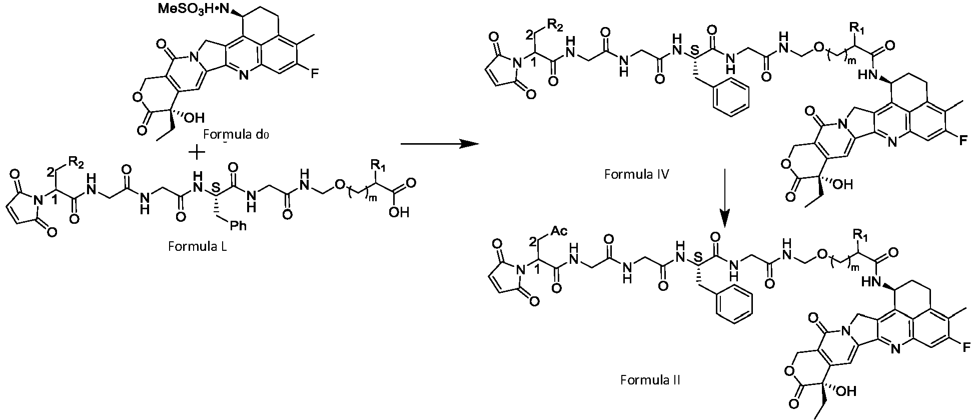
reacting a compound having Formula L with Exatecan having a Formula d₀ or its salt in the presence of a condensing agent under an alkaline condition to provide a compound having Formula IV, and
converting the compound having Formula IV to a compound having Formula II;
wherein,
the position-1 carbon and the R₁-connected carbon each has R or S absolute configuration;
R₂ is configured to form Ac; and
Ac, R₁, and m are as defined in Formula II.

16. The method for preparing the linker-drug compound or its pharmaceutically acceptable salt thereof according to claim 15, wherein converting the compound having Formula IV to a compound having Formula II is carried out with a deprotecting agent and a solvent, wherein the deprotecting agent is zinc bromide, and the solvent is nitromethane.

17. A method for preparing the ligand-drug conjugate or its pharmaceutically acceptable salt thereof according to claims 1-6, comprising the following steps: 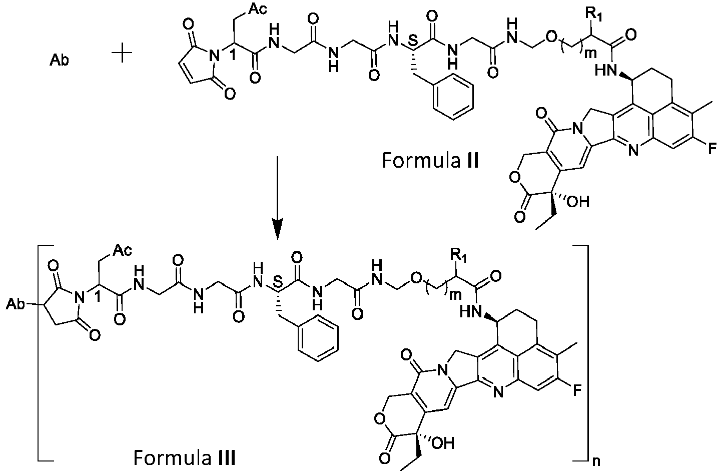
conjugating the ligand unit Ab with a compound having Formula II to provide the ligand-drug conjugate having Formula III;
wherein
Ab is selected from an antibody, antibody fragment, or protein;
Ac comprises a hydrophilic structural unit;
the position-1 carbon and the R ₁-connected carbon each has the chirality of R or S absolute configuration;
R₁, m and n are as described in Formula II.

18. A pharmaceutical composition comprising a therapeutically effective amount of the ligand-drug conjugate according to any one of claims 1-17 or its pharmaceutically acceptable salt or solvate thereof, and a pharmaceutically acceptable carrier, diluent, or excipient.

19. A pharmaceutical composition comprising the ligand-drug conjugate according to any one of claims 1-18 or its pharmaceutically acceptable salt thereof, for use in the preparation of a drug for the treatment of cancer, autoimmune diseases, or infectious diseases.

20. The use according to claim 19, wherein the cancer comprising breast cancer, ovarian cancer, cervical cancer, uterine cancer, prostate cancer, kidney cancer, urethral cancer, bladder cancer, liver cancer, gastric cancer, endometrial cancer, salivary gland cancer, esophageal cancer, lung cancer, colon cancer, rectal cancer, colorectal cancer, bone cancer, skin cancer, thyroid cancer, pancreatic cancer, melanoma, glioma, neuroblastoma, glioma multiforme, sarcoma, lymphoma and leukemia and other solid tumors or hematoma drugs.
